# EUROPEAN PATENT APPLICATION

(11) **EP 2 382 995 A2**
(43) Date of publication of application: **02.11.2011**
(21) Application number: 11162671.9
(22) Date of filing: 18.08.2006
(51) Int. Cl.: A61K 47/48, A61P 35/00

(54) **Ligand conjugates of Vinca alkaloids, analogs and derivatives**

(30) Priority: 19.08.2005 US 709936 P
(62) Divisional of application: 06801980.1
(71) Applicant: Endocyte, Inc., West Lafayette, IN 47906 (US)
(72) Inventor: Leamon, Christopher, Paul, West Lafayette, IN 47906 (US); Vlahov, Iontcho, Radoslavov, West Lafayette, IN 47906 (US)
(74) Representative: Elsy, David

(57) **Abstract**

Described herein are compounds, pharmaceutical compositions and methods for treating pathogenic cell populations in a patient. The compounds described herein include conjugates of cytotoxic drugs and vitamin receptor binding ligands. The conjugates also include a linker that is formed from one or more spacer linkers, heteroatom linkers, and releasable linkers.

## Description

### CROSS REFERENCE TO RELATED APPLICATION

This application claims the benefit under 35 U.S.C. § 119(e) of U.S. provisional patent application Serial No. 60/709,936, filed August 19, 2005, the entirety of the disclosure of which is incorporated herein by reference.

### TECHNICAL FIELD

The present invention relates to compositions and methods for use in targeted drug delivery. In particular, the invention relates to ligand conjugates of vinca alkaloids, and analogs and derivatives thereof, such as conjugates of vitamin receptor binding compounds and vinca alkaloids.

### BACKGROUND

The mammalian immune system provides a means for the recognition and elimination of tumor cells, other pathogenic cells, and invading foreign pathogens. While the immune system normally provides a strong line of defense, there are many instances where cancer cells, other pathogenic cells, or infectious agents evade a host immune response and proliferate or persist with concomitant host pathogenicity. Chemotherapeutic agents and radiation therapies have been developed to eliminate, for example, replicating neoplasms. However, many of the currently available chemotherapeutic agents and radiation therapy regimens have adverse side effects because they work not only to destroy pathogenic cells, but they also affect normal host cells, such as cells of the hematopoietic system. The adverse side effects of these anticancer drugs highlight the need for the development of new therapies selective for pathogenic cell populations and with reduced host toxicity.

Researchers have developed therapeutic protocols for destroying pathogenic cells by targeting cytotoxic compounds to such cells. Many of these protocols utilize toxins conjugated to antibodies that bind to antigens unique to or overexpressed by the pathogenic cells in an attempt to minimize delivery of the toxin to normal cells. Using this approach, certain immunotoxins have been developed consisting of antibodies directed to specific antigens on pathogenic cells, the antibodies being linked to toxins such as ricin, Pseudomonas exotoxin, Diptheria toxin, and tumor necrosis factor. These immunotoxins target pathogenic cells, such as tumor cells, bearing the specific antigens recognized by the antibody (Olsnes, S., Immunol. Today, 10, pp. 291-295, 1989; Melby, E.L., Cancer Res., 53(8), pp. 1755-1760, 1993; Better, M.D., PCT publication no. WO 91/07418, published May 30, 1991).

Another approach for targeting populations of pathogenic cells, such as cancer cells or foreign pathogens, in a host is to enhance the host immune response against the pathogenic cells to avoid the need for administration of compounds that may also exhibit independent host toxicity. One reported strategy for immunotherapy is to bind antibodies, for example, genetically engineered multimeric antibodies, to the surface of tumor cells to display the constant region of the antibodies on the cell surface and thereby induce tumor cell killing by various immune-system mediated processes (De Vita, V.T., Biologic Therapy of Cancer, 2d ed. Philadelphia, Lippincott, 1995; Soulillou, J.P., U.S. Patent No. 5,672,486). However, these approaches have been complicated by the difficulties in defining tumor-specific antigens.

### SUMMARY OF THE INVENTION

Conjugates of vinca alkaloids, and analogs and derivatives thereof are described herein. The conjugates include ligands, such as ligands of cell surface receptors covalently attached to vinca alkaloids, and analogs and derivatives thereof, optionally through a linker. The vinca alkaloids useful in the conjugates described herein include all members of the vinca indole-dihydroindole family of alkaloids, such as but not limited to vindesine, vinblastine, vincristine, catharanthine, vindoline, leurosine, vinorelbine, imidocarb, sibutramine, toltrazuril, vinblastinoic acid, and the like, and analogs and derivatives thereof.

In one embodiment, a receptor binding drug delivery conjugate is described. The drug delivery conjugate comprises a ligand, such as a ligand of a cell surface receptor, a vinca alkaloid, and optionally a bivalent linker, which may be generally represented by the formula

(B)-(L)-(D)

wherein (B) represents a receptor binding moiety, including but not limited to vitamins, and vitamin receptor binding analogs or derivatives thereof, such as vitamins and analogs and derivatives thereof that are capable of binding vitamin receptors; (D) represents a vinca alkaloid, or analog or derivative thereof; and (L) represents a bivalent linker. The bivalent linker (L) can comprise multiple linkers. For example, the bivalent linker (L) can comprise one or more spacer linkers (lₛ), and releasable linkers (lᵣ), each connected to the other and to the ligand and the vinca alkaloid by one or more heteroatom linkers (l_{H}). These various linkers may be selected and placed in any order to construct the bivalent linker (L). Illustratively, the bivalent linker (L) may be one of the following:
-(L)-
-(lᵣ)_{c}-
-(lₛ)ₐ-
-(lₛ)ₐ-(lᵣ)_{c}-
-(lᵣ)_{c}-(lₛ)ₐ-
-(l_{H})_{b}-(lᵣ)_{c}-
-(lᵣ)_{c}-(l_{H})_{b}-
-(l_{H})_{d}-(lᵣ)_{c}-(l_{H})ₑ-
-(lₛ)ₐ-(l_{H})_{b}-(lᵣ)_{c}-
-(lᵣ)_{c}-(l_{H})_{b}-(lₛ)ₐ-
-(l_{H})_{d}-(lₛ)ₐ-(lᵣ)_{c}-(l_{H})ₑ-
-(l_{H})_{d}-(lᵣ)_{c}-(lₛ)ₐ-(l_{H})ₑ-
-(l_{H})_{d}-(lₛ)ₐ-(l_{H})_{b}-(lᵣ)_{c}-(l_{H})ₑ-
-(l_{H})_{d}-(lᵣ)_{c}-(l_{H})_{b}-(lₛ)ₐ-(l_{H})ₑ-
-(lₛ)ₐ-(lᵣ)_{c}-(l_{H})_{b}-
-(lₛ)ₐ-l_{H})_{b}]_{d}-(lᵣ)_{c}-(l_{H})ₑ-
wherein a, b, c, d, and e are integers, such as integers in the range from 0 to about 4, and (lₛ), (l_{H}), and (lᵣ) are the spacer linkers, releasable linkers, heteroatom linkers, respectively. Additional illustrative examples of bivalent linkers are described in U.S. patent application publication no. US 2005/0002942 A1 and PCT international publication no. WO 2006/012527, the entirety of the disclosures of which are incorporated herein by reference. In one variation, more than one receptor binding ligand is included in the drug delivery conjugates described herein. It is to be understood that each of these receptor binding ligands may be the same or different.

In one illustrative embodiment of the drug delivery conjugates described herein, the bivalent linker includes at least one releasable linker (lᵣ). In another illustrative embodiment of the drug delivery conjugates described herein, the bivalent linker includes at least two releasable linkers (lᵣ)₂. In another illustrative aspect, the bivalent linker (L) includes at least one releasable linkers (lᵣ) that is not a disulfide releasable linker. In another illustrative aspect, the bivalent linker (L) has at least two releasable linkers (lᵣ)₂ where one releasable linker is not a disulfide releasable linker. It is appreciated that when more than one releasable linker is included in the bivalent linker, those releasable linkers may be adjacent. It is further appreciated that when two releasable linkers are adjacent in the bivalent linker, the two releasable linkers may cooperate to cause release of the vinca alkaloid, or analog or derivative thereof.

In another embodiment, the bivalent linker includes at least one spacer linker that is a peptide formed from amino acids. In one aspect, the peptide includes naturally occurring amino acids, and stereoisomers thereof. In another aspect, the peptide is formed only from naturally occurring amino acids, and stereoisomers thereof.

The ligands described herein generally include ligands of cell surface receptors. Illustrative ligands useful in the conjugates described herein include, but are not limited to, vitamins, and other moieties that bind to a vitamin receptor, transporter, or other surface-presented protein that specifically binds vitamins, or analogs or derivatives thereof, peptide ligands identified from library screens, tumor cell-specific peptides, tumor cell-specific aptamers, tumor cell-specific carbohydrates, tumor cell-specific monoclonal or polyclonal antibodies, Fab or scFv (i.e., a single chain variable region) fragments of antibodies such as, for example, an Fab fragment of an antibody directed to EphA2 or other proteins specifically expressed or uniquely accessible on metastatic cancer cells, small organic molecules derived from combinatorial libraries, growth factors, such as EGF, FGF, insulin, and insulin-like growth factors, and homologous polypeptides, somatostatin and its analogs, transferrin, lipoprotein complexes, bile salts, selectins, steroid hormones, Arg-Gly-Asp containing peptides, retinoids, various Galectins, δ-oploid receptor ligands, cholecystokinin A receptor ligands, ligands specific for angiotensin AT1 or AT2 receptors, peroxisome proliferator-activated receptor λ ligands, β-lactam antibiotics such as penicillin, small organic molecules including antimicrobial drugs, and other molecules that bind specifically to a receptor preferentially expressed on the surface of tumor cells or on an infectious organism, antimicrobial and other drugs designed to fit into the binding pocket of a particular receptor based on the crystal structure of the receptor or other cell surface protein, ligands of tumor antigens or other molecules preferentially expressed on the surface of tumor cells, or fragments of any of these molecules. Tumor-specific antigens that could function as a binding site for ligand-vinca conjugates include extracellular epitopes of members of the Ephrin family of proteins, such as EphA2. EphA2 expression is restricted to cell-cell junctions in normal cells, but EphA2 is distributed over the entire cell surface in metastatic tumor cells. Thus, EphA2 on metastatic cells would be accessible for binding to, for example, an Fab fragment of an antibody conjugated to a vinca alkaloid, whereas the protein would not be accessible for binding to the Fab fragment on normal cells, resulting in a ligand-vinca conjugate specific for metastatic cancer cells.

In another embodiment, a pharmaceutical composition is described. The pharmaceutical composition comprises a ligand-vinca conjugate described herein in combination with a pharmaceutically acceptable carrier, excipient, and/or diluent therefor.

In another embodiment, a method for eliminating a population of pathogenic cells in a host animal harboring the population of pathogenic cells is described. In one illustrative aspect, the members of the pathogenic cell population have an accessible binding site for a receptor binding moiety, or the analog or derivative thereof, and that binding site is uniquely expressed, overexpressed, or preferentially expressed by the pathogenic cells. The method includes the step of administering to the host a drug delivery conjugate described herein, or a pharmaceutical composition thereof, as described herein.

### BRIEF DESCRIPTION OF THE DRAWINGS

FIG. 1A shows the relative binding affinity of for Example 6 (■, 0.35) versus folic acid (●, 1.0) at folic acid receptors for 1 hour at 37°C.
FIG. 1B shows the activity of Example 6 on ³H-thymidine incorporation with (■) and without (●) excess folic acid; IC₅₀ of Example 6 = 14 nM.
FIG. 2 shows the activity of Example 6 (■) at 1.5 µmol/kg given TIW (7 doses) against M109 tumors in Balb/c mice versus untreated controls (●).
FIG. 3 shows the activity of Example 6 (b) at 10 µmol/kg given TIW for 3 weeks (the vertical dashed line indicated the last treatment day) on FR-positive M109 tumors versus untreated controls (a).
FIG. 4A shows the activity of Example 6 (A) at 3 µmol/kg TIW for 3 weeks on FR-positive M109 tumors versus untreated controls (■),
FIG. 4B shows the absence of activity of Example 6 (b) at 3 µmol/kg TIW for 3 weeks on FR-negative 4T-1 tumor cells versus untreated controls (a).
FIG. 5A shows the activity of Example 6 (●) at 10 µmol/kg TIW for 3 weeks on FR-positive KB tumors in nu/nu mice versus untreated controls (■).
FIG. 5B shows the absence of an effect by Example 6 (●) at 10 µmol/kg TIW for 3 weeks on the weight of nu/nu mice versus untreated controls (■).
FIG. 6A shows the activity of Example 6 at 1 µmol/kg (b), 5 µmol/kg (c), and 10 µmol/kg (d) TIW for 3 weeks (the vertical dashed line indicated the last treatment day) on FR-positive KB tumors in nu/nu mice versus untreated controls (a); average tumor volume at to = 50-100 mm³).
FIG. 6B shows the absence of an effect by Example 6 at 1 µmol/kg (b), 5 µmol/kg (c), and 10 µmol/kg (d) TIW for 3 weeks on the weight of nu/nu mice versus untreated controls (a).
FIG. 7A shows the activity of Example 6 (b) at 10 µmol/kg TIW for 3 weeks (the vertical dashed line indicated the last treatment day) on large FR-positive KB tumors in nu/nu mice versus untreated controls (a); average tumor volume at to = 100-150 mm³.
FIG. 7B shows the absence of an effect by Example 6 (a) at 10 µmol/kg TIW for 3 weeks (the vertical dashed line indicated the last treatment day) on the weight of nu/nu mice versus unconjugated desacetylvinblastine monohydrazide (b).
FIG. 8 shows the relative binding affinity of Example 7 (b, 0.2) versus folic acid (●) at folic acid receptors.
FIG. 9A shows the activity of Example 7 on ³H-thymidine incorporation into FR-positive KB cells with (a) and without (b) excess folic acid; IC₅₀ of Example 7=9 nM.
FIG. 9B shows the effect of incubation time on the activity of Example 7 at 100 nM on ³H-thymidine incorporation into FR-positive KB cells with (a) and without (b) excess folic acid, versus the pulse time for treatment.
FIG. 10A shows the effect of incubation time on the activity of Example 7 at 10 nM on ³H-thymidine incorporation into 2002 KB cells harvested at 48 hours with (a) and without (b) excess folic acid, versus the pulse time for treatment.
FIG. 10B shows the effect of incubation time on the activity of Example 7 at 100 nM on ³H-thymidine incorporation into 2002 KB cells harvested at 48 hours with (a) and without (b) excess folic acid, versus the pulse time for treatment.
FIG. 11 shows the activity of Example 7 (▼) at 5 µmol/kg TIW for 3 weeks on FR-positive KB tumors versus untreated controls (■); average tumor volume at to = 50-100 mm³.
FIG. 12 shows the activity of Example 6 and 14B, (b) and (c), respectively, each at 5 µmol/kg TIW for 3 weeks (the vertical dashed line indicated the last treatment day), on FR-positive KB tumors in nu/nu mice versus untreated controls (a); average tumor volume at t₀ = 50-80 mm³; Example 7 shows 5/5 complete responses.
FIG. 13 shows the activity of Example 7 (A) at 1.5 µmol/kg TIW against M109 tumors in Balb/c mice versus untreated controls (■).
FIG. 14A shows the activity of Examples 6 and 7, (b) and (c), respectively, each at 10 µmol/kg for 3 weeks (the vertical dashed line indicated the last treatment day) against M109 tumors in Balb/c mice versus untreated controls (a); average tumor volume at t₀ = 50-80 mm³.
FIG. 14B shows the absence of an effect by Examples 6 and 7, (b) and (c), respectively, (each at 10 µmol/kg for 3 weeks (the vertical dashed line indicated the last treatment day) on the weight of Balb/c mice.
FIG. 15 shows the activity of Example 7 at 2 µmol/kg TIW for 2 weeks on FR-positive KB tumors with (b) and without (c) 40 µmol/kg EC20 (rhenium complex) versus untreated controls (a); Example 7 alone showed 4/5 complete responses; Example 7 + EC20 showed 0/5 complete responses.
FIG. 16A shows the activity of Examples 6 and 7, (b) and (c), respectively, each at 5 µmol/kg TIW for 3 weeks on FR-positive KB tumors in nu/nu mice versus untreated controls (a).
FIG. 16B shows the absence of an effect by Examples 6 and 7, (b) and (c), respectively, (each at 5 µmol/kg for 3 weeks on the weight of nu/nu mice.
FIG. 17 shows the activity of Examples 14 (a) and 15 (b) alone (lefthand bars; each at 100 nM for 1 h with a 72 h chase, n = 2) versus Examples 14 (a) and 15 (b) under the same conditions with excess folic acid (right-hand bars) on ³H-thymidine incorporation into FR-positive KB cells.
FIG. 18 shows the activity of Example 16 on ³H-thymidine incorporation in KB cells; IC₅₀ is about 250 nM.
FIG. 19A shows the activity of Example 5 on ³H-thymidine incorporation in KB cells.
FIG. 19B shows the activity of Example 17 on ³H-thymidine incorporation in KB cells.
FIG. 20A shows the relative binding affinity of Example 19 (b, 0.046), Example 18 (c, 0.13), and Example 7 (d) versus folic acid (a, 1.0) at folic acid receptors.
FIG. 20B shows the activity of Example 7 on ³H-thymidine incorporation in KB cells with (b) and without (a) excess folic acid; IC₅₀ of Example 7 is about 16 nM; and of Example 19 on ³H-thyrnidine incorporation in 2002 KB cells with (d) and without (c) excess folic acid; IC₅₀ of Example 19 is about 100 nM .
FIG. 20C shows the activity of Example 18 on ³H-thymidine incorporation in 2002 KB cells with (b) and without (a) excess folic acid; IC₅₀ of Example 18 is about 6 nM.
FIG. 21A shows the relative binding affinity of Example 10 (■, 0.24) versus folic acid (●, 1.0) at folic acid receptors.
FIG. 21B shows the activity of Example 10 on ³H-thymidine incorporation in KB cells with (o) and without (●) excess folic acid; IC₅₀ of Example 10 is about 58 nM.
FIG. 22 shows the activity of Example 20 on ³H-thymidine incorporation in KB cells with (o) and without (●) excess folic acid; IC₅₀ of Example 20 is about 58 nM.
FIG. 23A shows the relative binding affinity of Example 21 (■, 0.16) versus folic acid (●, 1.0) at folic acid receptors.
FIG. 23B shows the activity of Example 21 on ³H-thymidine incorporation in KB cells with (o) and without (●) excess folic acid.
FIG. 24A shows the relative binding affinity of Example 22 (o, 0.26) versus folic acid (●, 1.0) at folic acid receptors.
FIG. 24B shows the activity of Example 22 on ³H-thymidine incorporation in KB cells with (o) and without (●) excess folic acid.
FIG. 25A shows the activity of Example 7 (●), Example 21 (▲), and Example 22 (T), each at 3 µmol/kg TIW for 3 weeks on FR-positive M109 tumors in Balb/c mice versus untreated controls (■).
FIG. 25B shows the absence of an effect by Example 7 (●), Example 21 (▲), and Example 22 (▼), each at 3 µmol/kg TIW for 3 weeks on the weight of Balb/c mice versus untreated controls (■).
FIG. 26A shows the activity of Example 11 (●) and Example 12 (▼), each at 2 µmol/kg TIW for 3 weeks on FR-positive KB tumors in nu/nu mice versus untreated controls (■).
FIG. 26B shows the absence of an effect by Example 11 (●) and Example 12 (▼), each at 2 µmol/kg TIW for 3 weeks on the weight of nu/nu mice versus untreated controls (■).

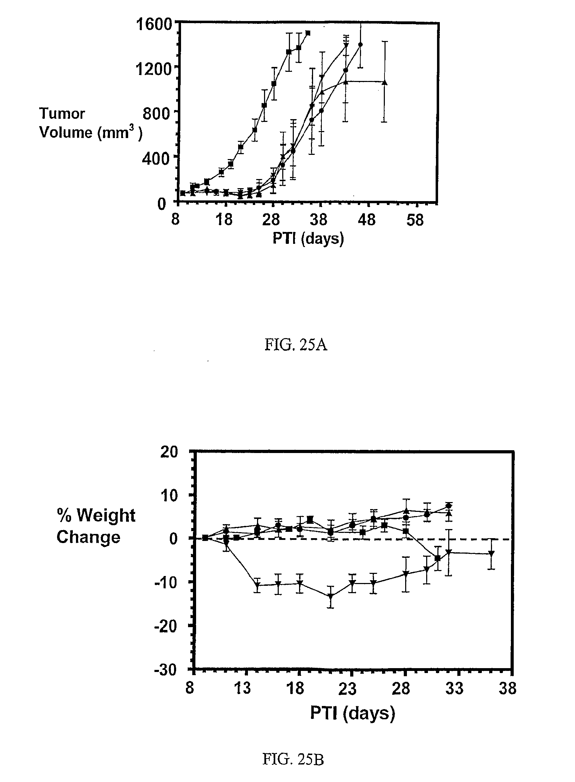

FIGS. 25A and 25B show the activity of Examples 21 and 22 in comparison to 7 (each at 3 µmol/kg) against M109 tumors in Balb/c mice and on the weight of Balb/c mice (Balb/c mice were used for the M109 tumor volume assay)

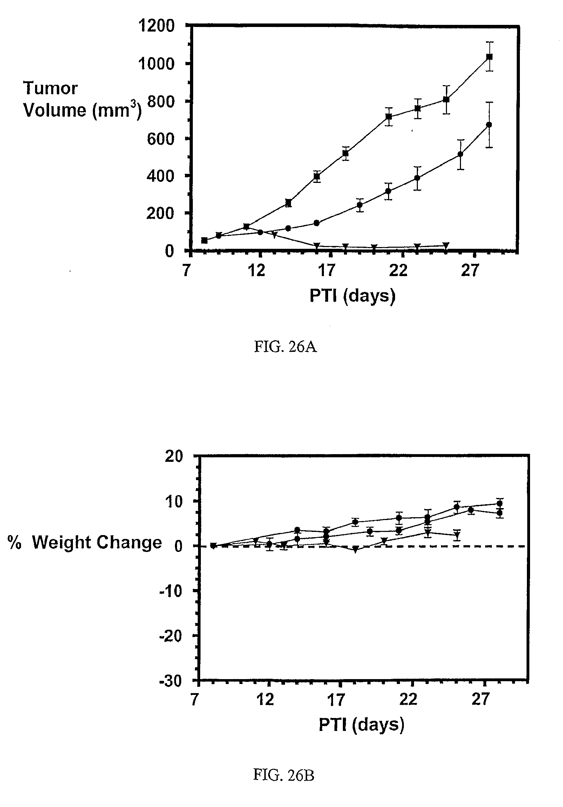

FIGS. 26A and 26B show the activities of Examples 11 and 12 at 2 µmol/kg TIW for 3 weeks on FR-positive KB tumors and on the weight of nu/nu mice (nu/nu mice were used for the KB tumor volume assay)

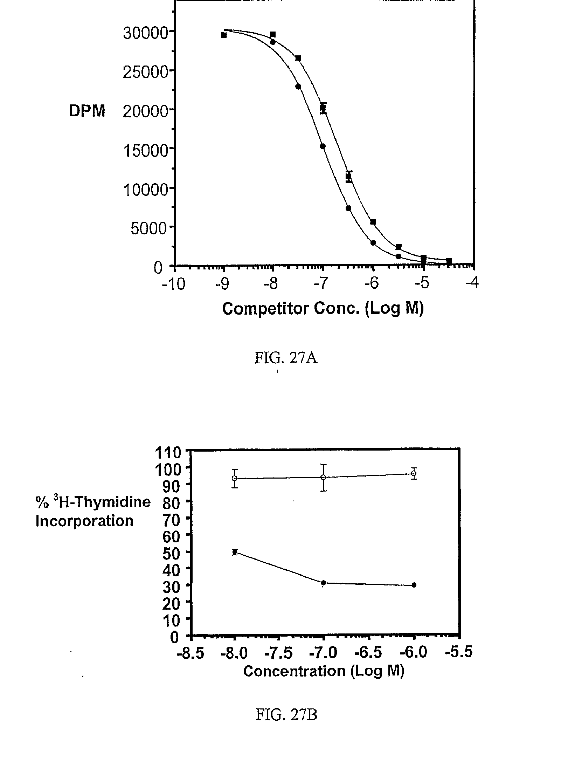

FIG. 27A shows the relative binding affinity of Example 23 (■, 0.51) versus folic acid (●, 1.0) at folic acid receptors.
FIG. 27B shows the activity of Example 23 on ³H-thymidine incorporation in KB cells with (o) and without (●) excess folic acid; IC₅₀ of Example 23 is about 15 nM.

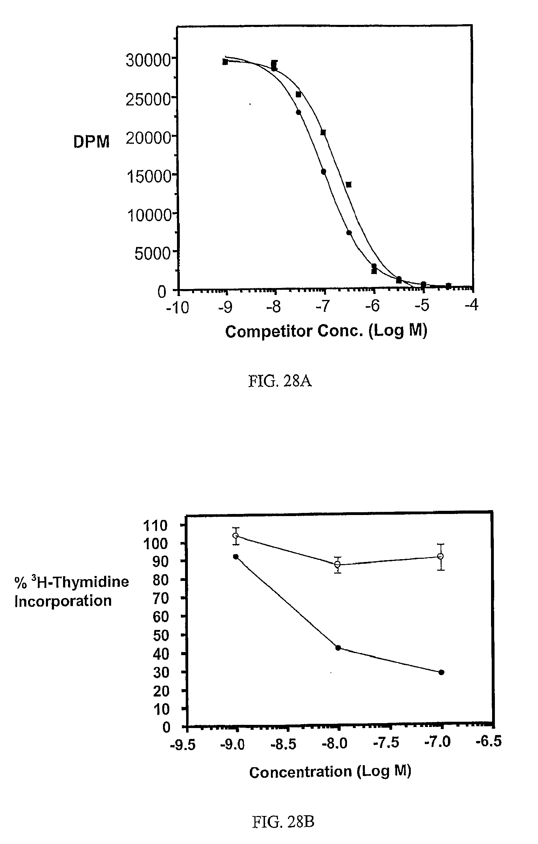

FIG. 28A shows the relative binding affinity of Example 242B (■, 0.45) versus folic acid (●, 1.0) at folic acid receptors.
FIG. 28B shows the activity of Example 24 on ³H-thymidine incorporation in KB cells with (o) and without (●) excess folic acid; IC₅₀ of Example 24 is about 9 nM.
FIGS. 27A and 27B show the relative binding affinity for folate versus Example 23, and the effects of Example 23 on ³H-thymidine incorporation, the IC₅₀ of the conjugate (15 nM), and that folate competes with the conjugate for binding to the folate receptor demonstrating the specificity of binding of the conjugate. The assays were conducted according to Method Examples 4 and 3, respectively
FIGS. 28A and 28B show the relative binding affinity for folate versus Example 24, and the effects of Example 24 on ³H-thymidine incorporation, the IC₅₀ of the conjugate (9 nM), and that folate competes with the conjugate for binding to the folate receptor demonstrating the specificity of binding of the conjugate.

### DETAILED DESCRIPTION

Ligand conjugates of drugs, and analogs and derivatives thereof, are described herein. The conjugates include cell receptor binding ligands, including ligands of cell surface receptors, that are covalently attached to two or more drugs that may be targeted to cells, including pathogenic cells. The conjugates described herein may also include a polyvalent linker for attaching the ligands to the drugs.

In one embodiment, a receptor binding drug delivery conjugate is described. The drug delivery conjugate comprises a ligand of a cell surface receptor, a vinca alkaloid, and optionally a bivalent linker, which may be generally represented by the formula

(B)-(L)-(D)

wherein (B) represents a receptor binding moiety, including but not limited to vitamins, and vitamin receptor binding analogs or derivatives thereof, such as vitamins and analogs and derivatives thereof that are capable of binding vitamin receptors; (D) represents a vinca alkaloid, or analog or derivative thereof; and (L) represents a bivalent linker. The bivalent linker (L) can comprise multiple linkers. For example, the bivalent linker (L) can comprise one or more spacer linkers (lₛ), and releasable linkers (lᵣ), each connected to the other and to the ligand and the vinca alkaloid by one or more heteroatom linkers (l_{H}). These various linkers may be selected and placed in any order to construct the bivalent linker (L). Illustratively, the bivalent linker (L) may be one of the following:
-(L)-
-(lᵣ)c-
-(lₛ)ₐ-
-(lₛ)ₐ-(lᵣ)_{c}-
-(lᵣ)_{c}-(lₛ)ₐ-
-(l_{H})_{b}-(lᵣ)_{c}-
-(lᵣ)_{c}-(l_{H})_{b}-
-(l_{H})_{d}-(lᵣ)_{c}-(l_{H})ₑ-
-(lₛ)ₐ-(l_{H})_{b}-(lᵣ)_{c}-
-(lᵣ)_{c}-(l_{H})_{b}-(lₛ)ₐ-
-(l_{H})_{d}-(lₛ)ₐ-(lᵣ)_{c}-(l_{H})ₑ-
-(l_{H})_{d}-(lᵣ)_{c}-(lₛ)ₐ-(l_{H})ₑ-
-(l_{H})_{d}-(lₛ)ₐ-(l_{H})_{b}-(lᵣ)_{c}-(l_{H})ₑ-
-(l_{H})_{d}-(lᵣ)_{c}-(l_{H})_{b}-(lₛ)ₐ-(l_{H})ₑ-
-(lₛ)ₐ-(lᵣ)_{c}-(l_{H})_{b}-
-[(lₛ)ₐ-(l_{H})_{b}]_{d}-(lᵣ)_{c}-(l_{H})ₑ-
wherein a, b, c, d, and e are integers, such as integers in the range from 0 to about 4, and (lₛ), (l_{H}), and (lᵣ) are the spacer linkers, releasable linkers, heteroatom linkers, respectively. Additional illustrative examples of bivalent linkers are described in U.S. patent application publication no. US 2005/0002942-A1 and PCT international publication no. WO 2006/012527, the entirety of the disclosures of which are incorporated herein by reference.

Receptor binding drug delivery conjugates comprising a receptor binding moiety (B), a bivalent linker (L), and a vinca alkaloid drug, or analog or derivative thereof, (D) are described wherein the receptor binding moiety (B) and the vinca alkaloid drug (D) are each bound to the bivalent linker (L), through an heteroatom linker (l_{H}). The bivalent linker (L) comprises one or more spacer linkers, heteroatom linkers, and releasable linkers, and combinations thereof, in any order.

For example, in one illustrative embodiment of the manner in which linkers are covalently assembled to form the bivalent linker, or part of the bivalent linker, heteroatom linkers, spacer linkers, and releasable linkers are connected to form a bivalent group of the formula: 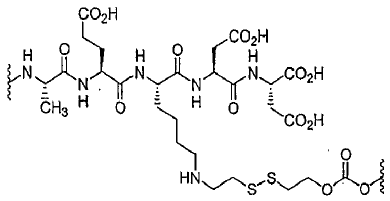 where the formula may also be represented as -(lₛ)₅-(lₛ)'-(lᵣ)-(l_{H})-. In that formula, (lₛ)₅ is the pentapeptide Ala-Glu-Lys-Asp-Asp-OH, (lₛ)' is CH₂CH₂, (lᵣ) isS-S-(CH₂)₂-O-C(O), and (l_{H}) is O. The releasable linker (lᵣ) is connected to the Lys of (lₛ)₅ at the ω-amino group.

In one illustrative embodiment of the drug delivery conjugates described herein, the bivalent linker includes at least one releasable linker (lᵣ). In another illustrative embodiment of the drug delivery conjugates described herein, the bivalent linker includes at least two releasable linkers (lᵣ)₂. In another illustrative aspect, the bivalent linker (L) includes at least one releasable linkers (lᵣ) that is not a disulfide releasable linker. In another illustrative aspect, the bivalent linker (L) has at least two releasable linkers (lᵣ)₂ where one releasable linker is not a disulfide releasable linker. It is appreciated that when more than one releasable linker is included in the bivalent linker, those releasable linkers may be adjacent. It is further appreciated that when two releasable linkers are adjacent in the bivalent linker, the two releasable linkers may cooperate to cause release of the vinca alkaloid, or analog or derivative thereof.

The term "releasable linker" as used herein, and also known as cleavable linker, refers to a linker that includes at least one bond that can be broken under physiological conditions (e.g., a pH-labile, acid-labile, oxidatively-labile, or enzyme-labile bond). It should be appreciated that such physiological conditions resulting in bond breaking include standard chemical hydrolysis reactions that occur, for example, at physiological pH, or as a result of compartmentalization into a cellular organelle such as an endosome having a lower pH than cytosolic pH.

It is understood that a cleavable bond can connect two adjacent atoms within the releasable linker and/or connect other linkers or (B) and/or (D), as described herein, at either or both ends of the releasable linker. In the case where a cleavable bond connects two adjacent atoms within the releasable linker, following breakage of the bond, the releasable linker is broken into two or more fragments. Alternatively, in the case where a cleavable bond is between the releasable linker and another moiety, such as an heteroatom linker, a spacer linker, another releasable linker, the drug, or analog or derivative thereof, or the vitamin, or analog or derivative thereof, following breakage of the bond, the releasable linker is separated from the other moiety.

The lability of the cleavable bond can be adjusted by, for example, substitutional changes at or near the cleavable bond, such as including alpha branching adjacent to a cleavable disulfide bond, increasing the hydrophobicity of substituents on silicon in a moiety having a silicon-oxygen bond that may be hydrolyzed, homologating alkoxy groups that form part of a ketal or acetal that may be hydrolyzed, and the like.

Illustrative mechanisms for cleavage of the bivalant linkers described herein include the following 1,4 and 1,6 fragmentation mechanisms 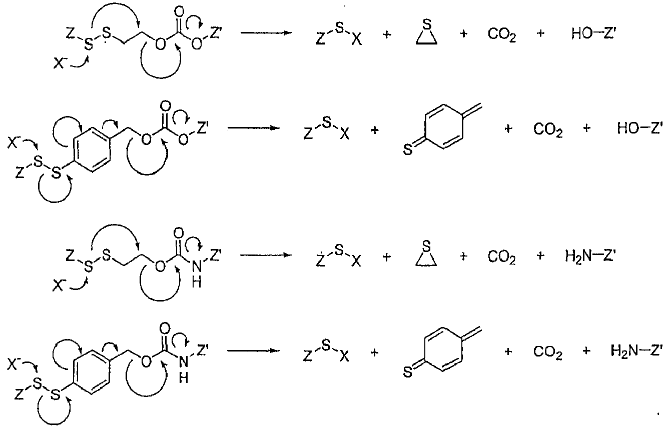 where X is an exogenous or endogenous nucleophile, glutathione, or bioreducing agent, and the like, and either of Z or Z' is the vitamin, or analog or derivative thereof, or the drug, or analog or derivative thereof, or a vitamin or drug moiety in conjunction with other portions of the bivalent linker. It is to be understood that although the above fragmentation mechanisms are depicted as concerted mechanisms, any number of discrete steps may take place to effect the ultimate fragmentation of the bivalent linker to the final products shown. For example, it is appreciated that the bond cleavage may also occur by acid-catalyzed elimination of the carbamate moiety, which may be anchimerically assisted by the stabilization provided by either the aryl group of the beta sulfur or disulfide illustrated in the above examples. In those variations of this embodiment, the releasable linker is the carbamate moiety. Alternatively, the fragmentation may be initiated by a nucleophilic attack on the disulfide group, causing cleavage to form a thiolate. The thiolate may intermolecularly displace a carbonic acid or carbamic acid moiety and form the corresponding thiacyclopropane. In the case of the benzyl-containing bivalent linkers, following an illustrative breaking of the disulfide bond, the resulting phenyl thiolate may further fragment to release a carbonic acid or carbamic acid moiety by forming a resonance stabilized intermediate. In any of these cases, the releasable nature of the illustrative bivalent linkers described herein may be realized by whatever mechanism may be relevant to the chemical, metabolic, physiological, or biological conditions present.

Other illustrative mechanisms for bond cleavage of the releasable linker include oxonium-assisted cleavage as follows: 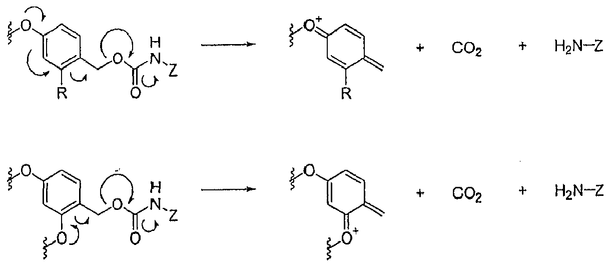 where Z is the vitamin, or analog or derivative thereof, or the drug, or analog or derivative thereof, or each is a vitamin or drug moiety in conjunction with other portions of the bivalent linker, such as a drug or vitamin moiety including one or more spacer linkers, heteroatom linkers, and/or other releasable linkers. In this embodiment, acid-catalyzed elimination of the carbamate leads to the release of CO₂ and the nitrogen-containing moiety attached to Z, and the formation of a benzyl cation, which may be trapped by water, or any other Lewis base.

Another illustrative mechanism involves an arrangement of the releasable, spacer, and heteroatom linkers in such a way that subsequent to the cleavage of a bond in the bivalent linker, released functional groups chemically assist the breakage or cleavage of additional bonds, also termed anchimeric assisted cleavage or breakage. An illustrative embodiment of such a bivalent linker or portion thereof includes compounds having the formula: 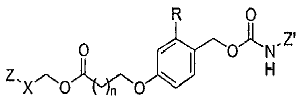 where X is an heteroatom, such as nitrogen, oxygen, or sulfur, n is an integer selected from 0, 1, 2, and 3, R is hydrogen, or a substituent, including a substituent capable of stabilizing a positive charge inductively or by resonance on the aryl ring, such as alkoxy, and the like, and either of Z or Z' is the vitamin, or analog or derivative thereof, or the drug, or analog or derivative thereof, or a vitamin or drug moiety in conjunction with other portions of the bivalent linker. It is appreciated that other substituents may be present on the aryl ring, the benzyl carbon, the carbamate nitrogen, the alkanoic acid, or the methylene bridge, including but not limited to hydroxy, alkyl, alkoxy, alkylthio, halo, and the like. Assisted cleavage may include mechanisms involving benzylium intermediates, benzyne intermediates, lactone cyclization, oxonium intermediates, beta-elimination, and the like. It is further appreciated that, in addition to fragmentation subsequent to cleavage of the releasable linker, the initial cleavage of the releasable linker may be facilitated by an anchimerically assisted mechanism.

In this embodiment, the hydroxyalkanoic acid, which may cyclize, facilitates cleavage of the methylene bridge, by for example an oxonium ion, and facilitates bond cleavage or subsequent fragmentation after bond cleavage of the releasable linker. Alternatively, acid catalyzed oxonium ion-assisted cleavage of the methylene bridge may begin a cascade of fragmentation of this illustrative bivalent linker, or fragment thereof. Alternatively, acid-catalyzed hydrolysis of the carbamate may facilitate the beta elimination of the hydroxyalkanoic acid, which may cyclize, and facilitate cleavage of methylene bridge, by for example an oxonium ion. It is appreciated that other chemical mechanisms of bond breakage or cleavage under the metabolic, physiological, or cellular conditions described herein may initiate such a cascade of fragmentation. It is appreciated that other chemical mechanisms of bond breakage or cleavage under the metabolic, physiological, or cellular conditions described herein may initiate such a cascade of fragmentation.

In one embodiment, the bivalent linkers described herein are compounds of the following formulae 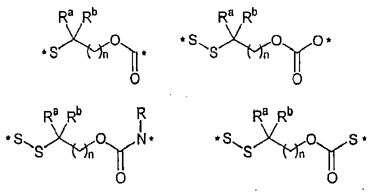 where n is an integer selected from 1 to about 4; R^{a} and R^{b} are each independently selected from the group consisting of hydrogen and alkyl, including lower alkyl such as C₁-C₄ alkyl that are optionally branched; or R^{a} and R^{b} are taken together with the attached carbon atom to form a carbocyclic ring; R is an optionally substituted alkyl group, an optionally substituted acyl group, or a suitably selected nitrogen protecting group; and (*) indicates points of attachment for the drug, vitamin, imaging agent, diagnostic agent, other bivalent linkers, or other parts of the conjugate.

In another embodiment, the bivalent linkers described herein include compounds of the following formulae 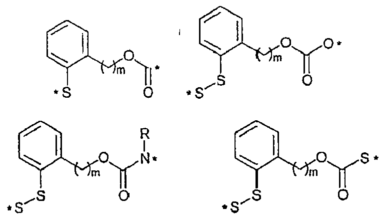 where m is an integer selected from 1 to about 4; R is an optionally substituted alkyl group, an optionally substituted acyl group, or a suitably selected nitrogen protecting group; and (*) indicates points of attachment for the drug, vitamin, imaging agent, diagnostic agent, other bivalent linkers, or other parts of the conjugate.

In another embodiment, the bivalent linkers described herein include compounds of the following formulae 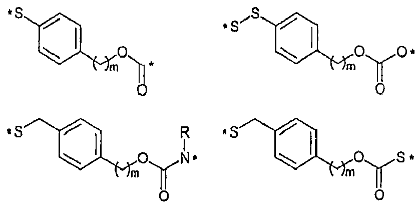 where m is an integer selected from 1 to about 4; R is an optionally substituted alkyl group, an optionally substituted acyl group, or a suitably selected nitrogen protecting group; and (*) indicates points of attachment for the drug, vitamin, imaging agent, diagnostic agent, other bivalent linkers, or other parts of the conjugate.

In another embodiment, the releasable, spacer, and heteroatom linkers may be arranged in such a way that subsequent to the cleavage of a bond in the bivalent linker, released functional groups chemically assist the breakage or cleavage of additional bonds, also termed anchimeric assisted cleavage or breakage. An illustrative embodiment of such a bivalent linker or portion thereof includes compounds having the formula: 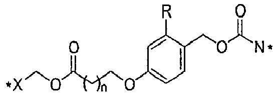 where X is an heteroatom, such as nitrogen, oxygen, or sulfur, n is an integer selected from 0, 1, 2, and 3, R is hydrogen, or a substituent, including a substituent capable of stabilizing a positive charge inductively or by resonance on the aryl ring, such as alkoxy, and the like, and the symbol (*) indicates points of attachment for additional spacer, heteroatom, or releasable linkers forming the bivalent linker, or alternatively for attachment of the drug, or analog or derivative thereof, or the vitamin, or analog or derivative thereof. It is appreciated that other substituents may be present on the aryl ring, the benzyl carbon, the alkanoic acid, or the methylene bridge, including but not limited to hydroxy, alkyl, alkoxy, alkylthio, halo, and the like. Assisted cleavage may include mechanisms involving benzylium intermediates, benzyne intermediates, lactone cyclization, oxonium intermediates, beta-elimination, and the like. It is further appreciated that, in addition to fragmentation subsequent to cleavage of the releasable linker, the initial cleavage of the releasable linker may be facilitated by an anchimerically assisted mechanism.

In another embodiment, the bivalent linker includes heteroatom linkers, spacer linkers, and releasable linkers connected to form a bivalent 3-thiosuccinimid-1-ylalkyloxymethyloxy group, illustrated by the following formula 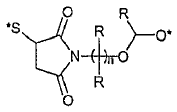 where n is an integer from 1 to 6, the alkyl group is optionally substituted, and the methyl is optionally substituted with an additional alkyl or optionally substituted aryl group, each of which is represented by an independently selected group R. The (*) symbols indicate points of attachment of the bivalent linker fragment to other parts of the conjugates described herein. In another embodiment, the bivalent linker includes heteroatom linkers, spacer linkers, and releasable linkers connected to form a bivalent 3-thiosuccinimid-1-ylalkylcarbonyl group, illustrated by the following formula 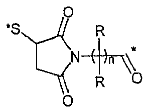 where n is an integer from 1 to 6, and the alkyl group is optionally substituted. The (*) symbols indicate points of attachment of the bivalent linker fragment to other parts of the conjugates described herein. In another embodiment, the bivalent linker includes heteroatom linkers, spacer linkers, and releasable linkers connected to form a bivalent 3-thioalkylsulfonylalkyl(disubstituted silyl)oxy group, where the disubstituted silyl is substituted with alkyl and/or optionally substituted aryl groups.

In another embodiment, the bivalent linker includes heteroatom linkers, spacer linkers, and releasable linkers connected to form a bivalent dithioalkylcarbonylhydrazide group, or a bivalent 3-thio or 3-dithiosuccinimid-1-ylalkylcarbonylhydrazide, illustrated by the following formulae 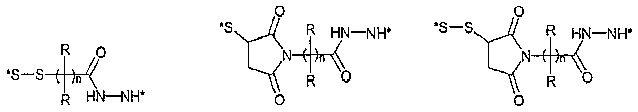 where n is an integer from 1 to 6, the alkyl group is optionally substituted, and the hydrazide forms an hydrazone with (B), (D), or another part of the bivalent linker (L). The (*) symbols indicate points of attachment of the bivalent linker fragment to other parts of the conjugates described herein.

In another embodiment, the bivalent linker includes heteroatom linkers, spacer linkers, and releasable linkers connected to form a bivalent 3-thiosuccinimid-1-ylalkyloxyalkyloxyalkylidene group, illustrated by the following formula 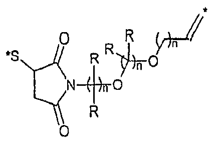 where each n is an independently selected integer from 1 to 6, each alkyl group independently selected and is optionally substituted, such as with alkyl or optionally substituted aryl, and where the alkylidene forms an hydrazone with (B), (D), or another part of the bivalent linker (L). The (*) symbols indicate points of attachment of the bivalent linker fragment to other parts of the conjugates described herein.

In another embodiment, the bivalent linker includes heteroatom linkers, spacer linkers, and releasable linkers connected to form a bivalent 3-thio or 3-dithioarylalkyloxycarbonyl group, 3-thio or 3-dithioarylalkylaminocarbonyl group, a bivalent 3-thio or 3-dithioalkyloxycarbonyl, or a bivalent 3-thio or 3-dithioalkylaminocarbonyl, where the alkyl carbonyl forms a carbonate, a carbamate, or urea with (B), (D), or another part of the bivalent linker (L). Illustratively, the alkyl group is ethyl.

In another embodiment, the bivalent linker includes heteroatom linkers, spacer linkers, and releasable linkers connected to form a bivalent 3-dithioalkylamino group, where the amino forms a vinylogous amide with (B), (D), or another part of the bivalent linker (L). Illustratively, the alkyl group is ethyl.

In another embodiment, the bivalent linker includes heteroatom linkers, spacer linkers, and releasable linkers connected to form a bivalent 1-alkoxycycloalkylenoxy group, a bivalent alkyleneaminocarbonyl(dicarboxylarylene)carboxylate group, a bivalent 3-dithioalkyloxycarbonyl group, a bivalent 3-dithioalkyloxycarbonylhydrazide group, a bivalent.

In another embodiment, the bivalent linker includes at least one spacer linker that is a peptide formed from amino acids. In one aspect, the peptide includes naturally occurring amino acids, and stereoisomers thereof. In another aspect, the peptide is formed only from naturally occurring amino acids, and stereoisomers thereof.

Additional illustrative examples of spacer and releasable linkers are shown in Table 1 and 2, where the (*) indicates the point of attachment to another linker, to the vinca alkaloid, or analog or derivative thereof, or to the receptor binding moiety.

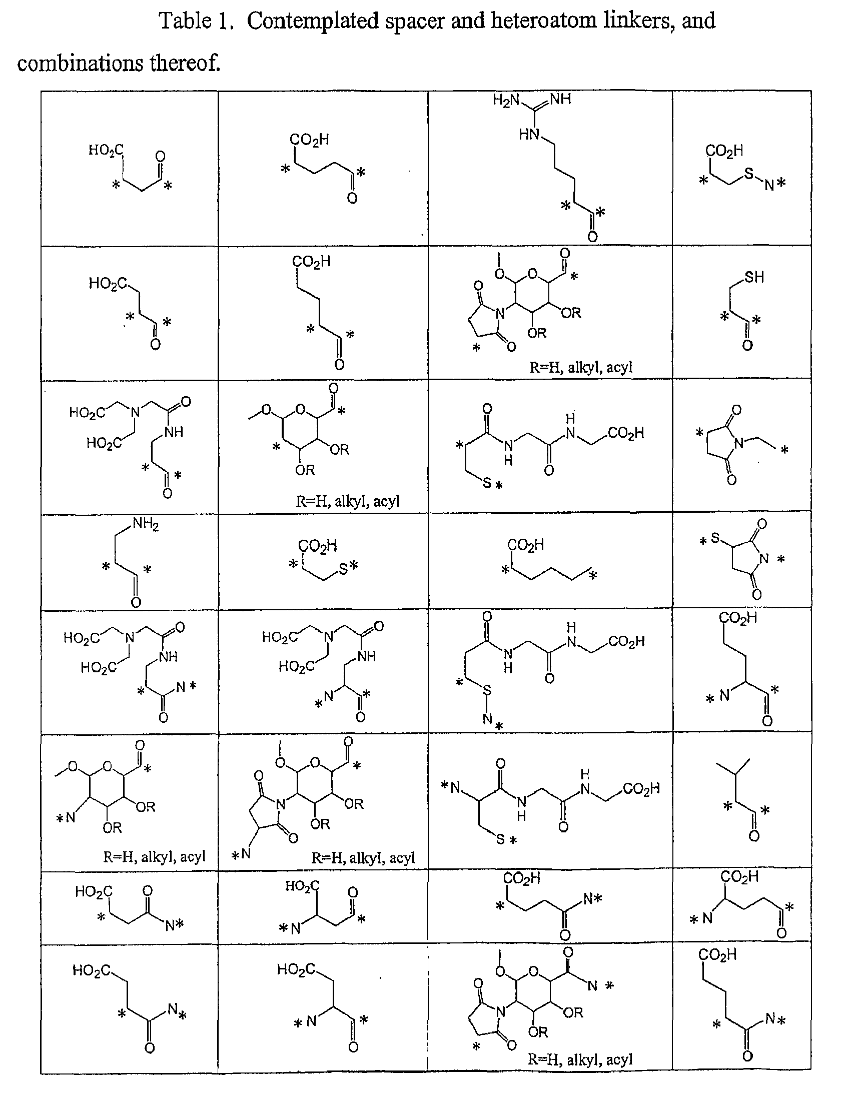

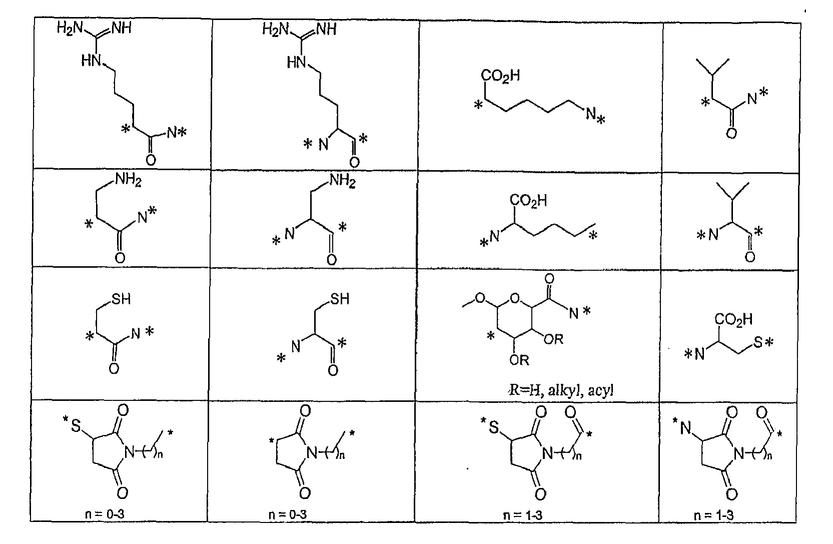

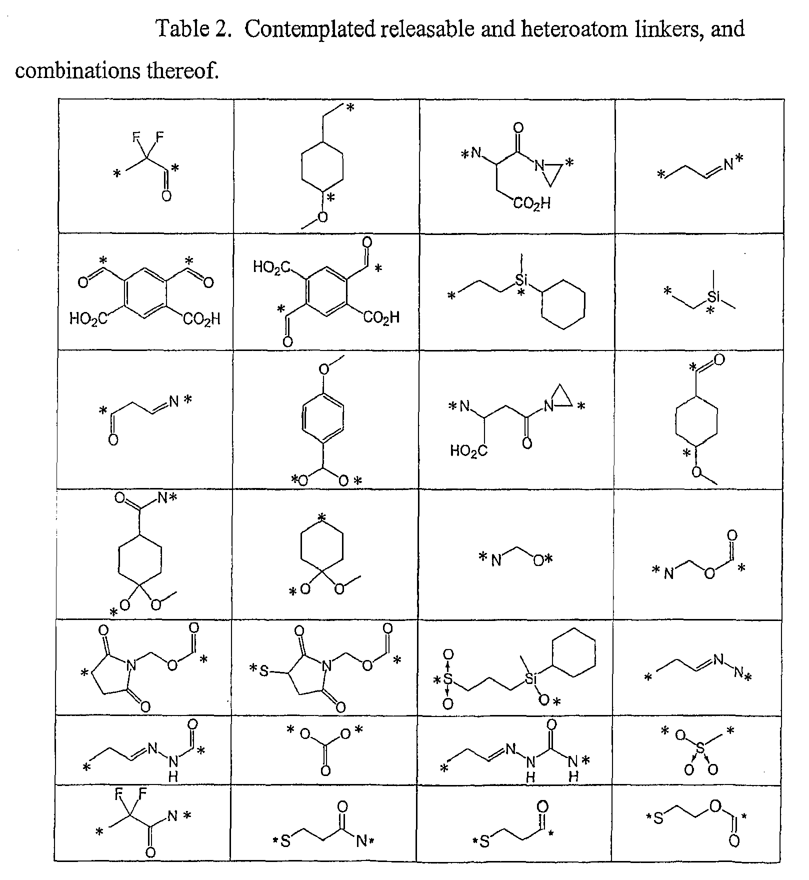

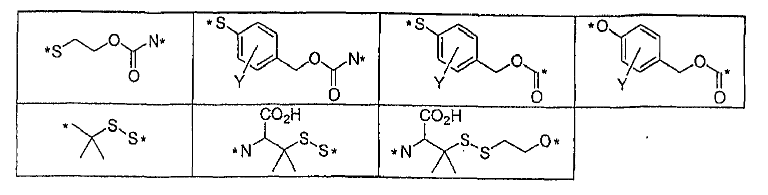

As referred to herein, the vinca drugs useable in the conjugates described herein include all members of the vinca indole-dihydroindole family of alkaloids, such as vindesine, vinblastine, vincristine, catharanthine, vindoline, leurosine, vinorelbine, imidocarb, sibutramine, toltrazuril, vinblastinoic acid, and the like, and analogs and derivatives thereof. Illustratively, such analogs and derivatives include the 3-carboxazides described in U.S. Patent No. 4,203,898; the N²-alkyl and other derivatives of 4-desacetylvinblastine-3-carboxhydrazide described in U.S. Patent No. 4,166,810; leurosine hydrazide described in Neuss et al. Tetrahedron Lett. 783 (1968); the hydrazide derivatives described in Barnett et al. J. Med. Chem. 21:88 (1978); the C-4 ester derivatives described in U.S. Patents Nos. 3,392,173 and 3,387,001; the dicarboxylic acid derivatives resulting from oxidation described in Langone et al. Anal. Biochem. 95:214 (1979); and the vinca hydrazides described in EP 0 247 792 A2. Each of the foregoing patents and publications is incorporated herein by reference for all that it discloses regarding synthetic routes, and reaction conditions for preparing vinca compounds.

In one illustrative embodiment, the vinca drugs are compounds of the formula 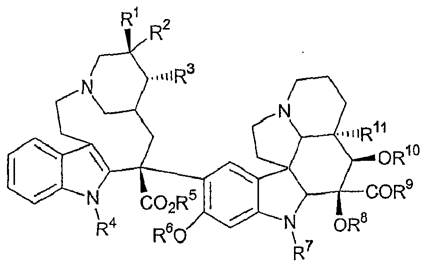 wherein:
one of R¹ and R² is H, and the other is ethyl, and R³ is H, or R¹ is ethyl R², and R³ are taken together to form -O-;
R⁴, R⁷, and R⁸ are each independently selected from H, alkyl, and acyl
R⁵ and R⁶ are each independently selected alkyl;
R⁹ is a group -NHNHR, where R is H, alkyl, or acyl;
R¹⁰ is H or acyl; and
R¹¹ is ethyl.

In one aspect, the vinca drugs are compounds of the above formula wherein R⁴ and R⁸ are each H; and R⁵, R⁶, R⁹, and R¹⁰ are each methyl.

The ligands of cell surface receptors useful in the conjugates described herein include, but are not limited to, vitamins, and other moieties that bind to a vitamin receptor, transporter, or other surface-presented protein that specifically binds vitamins, or analog or derivative thereof, peptide ligands identified from library screens, tumor cell-specific peptides, tumor cell-specific aptamers, tumor cell-specific carbohydrates, tumor cell-specific monoclonal or polyclonal antibodies, Fab or scFv (i.e., a single chain variable region) fragments of antibodies such as, for example, an Fab fragment of an antibody directed to EphA2 or other proteins specifically expressed or uniquely accessible on metastatic cancer cells, small organic molecules derived from combinatorial libraries, growth factors, such as EGF, FGF, insulin, and insulin-like growth factors, and homologous polypeptides, somatostatin and its analogs, transferrin, lipoprotein complexes, bile salts, selectins, steroid hormones, Arg-Gly-Asp containing peptides, retinoids, various Galectins, δ-opioid receptor ligands, cholecystokinin A receptor ligands, ligands specific for angiotensin AT1 or AT2 receptors, peroxisome proliferator-activated receptor λ ligands, β-lactam antibiotics such as penicillin, small organic molecules including antimicrobial drugs, and other molecules that bind specifically to a receptor preferentially expressed on the surface of tumor cells or on an infectious organism, antimicrobial and other drugs designed to fit into the binding pocket of a particular receptor based on the crystal structure of the receptor or other cell surface protein, ligands of tumor antigens or other molecules preferentially expressed on the surface of tumor cells,or fragments of any of these molecules. An example of a tumor-specific antigen that could function as a binding site for ligand-vinca conjugates include extracellular epitopes of a member of the Ephrin family of proteins, such as EphA2. EphA2 expression is restricted to cell-cell junctions in normal cells, but EphA2 is distributed over the entire cell surface in metastatic tumor cells. Thus, EphA2 on metastatic cells would be accessible for binding to, for example, an Fab fragment of an antibody conjugated to a vinca compound, whereas the protein would not be accessible for binding to the Fab fragment on normal cells, resulting in a ligand-vinca conjugate specific for metastatic cancer cells.

The vitamins that can be used in accordance with the methods and compounds described herein include carnitine, inositol, lipoic acid, pyridoxal, ascorbic acid, niacin, pantothenic acid, folic acid, riboflavin, thiamine, biotin, vitamin B_{12,} vitamins A, D, E and K, other related vitamin molecules, analogs and derivatives thereof, and combinations thereof. These vitamins, and their receptor-binding analogs and derivatives, constitute illustrative targeting entities that can be coupled with the vinca compounds by the bivalent linkers (L) described herein to make drug delivery conjugates.

In one illustrative aspect, the vitamin can be folic acid, a folic acid analog, or another folate receptor-binding molecule. Exemplary of analogs of folate that can be used include folinic acid, pteroylpolyglutamic acid, pteroic acid and other amino acid derivatives thereof, and folate receptor-binding pteridines such as tetrahydropterins, dihydrofolates, tetrahydrofolates, and their deaza and dideaza analogs. The terms "deaza" and "dideaza" analogs refers to the art recognized analogs having a carbon atom substituted for one or two nitrogen atoms in the naturally occurring folic acid structure. For example, the deaza analogs include the 1-deaza, 3-deaza, 5-deaza, 8-deaza, and 10-deaza analogs. The dideaza analogs include, for example, 1,5 dideaza, 5,10-dideaza, 8,10-dideaza, and 5,8-dideaza analogs. The foregoing folic acid analogs are conventionally termed "folates," reflecting their capacity to bind to folate receptors. Other folate receptor-binding analogs include aminopterin, amethopterin (methotrexate), N¹⁰-methylfolate, 2-deamino-hydroxyfolate, deaza analogs such as 1-deazamethopterin or 3-deazamethopterin, and 3',5'-dichloro-4-amino-4-deoxy-N¹⁰-methylpteroylglutamic acid (dichloromethotrexate). Other suitable ligands capable of binding to folate receptors to initiate receptor mediated endocytotic transport of the drug delivery conjugate include antibodies to the folate receptor. Accordingly, in one illustrative aspect, a vinca compound in complex with an antibody to a folate receptor can be used to trigger transmembrane transport of the complex.

Illustrative embodiments of vitamin analogs and/or derivatives also include analogs and derivatives of biotin such as biocytin, biotin sulfoxide, oxybiotin and other biotin receptor-binding compounds, and the like. It is appreciated that analogs and derivatives of the other vitamins described herein are also contemplated herein.

The drug delivery conjugates described herein can be prepared by conventional synthetic methods. The synthetic methods are chosen depending upon the selection of the heteroatom linkers, and the functional groups present on the spacer linkers and the releasable linkers. In general, the relevant bond forming reactions are described in Richard C. Larock, "Comprehensive Organic Transformations, a guide to functional group preparations," VCH Publishers, Inc. New York (1989), and in Theodora E. Greene & Peter G.M. Wuts, "Protective Groups ion Organic Synthesis," 2d edition, John Wiley & Sons, Inc. New York (1991), the disclosures of which in their entirety are incorporated herein by reference. Additional synthetic routes and reaction conditions are described in U.S. patent application publication no. US 2005/0002942 A1.

Illustratively, the drug delivery conjugates described herein may be prepared using both linear and convergent synthetic routes. Illustrative intermediates useable in such routes include intermediates comprising a bivalent linker that includes a coupling group at each end suitable for covalent attachment to the receptor binding moiety, or analog or derivative thereof, and the vinca alkaloid, or analog or derivative thereof. Other illustrative intermediates useable in such routes include intermediates comprising a receptor binding moiety, or analog or derivative thereof, attached to a bivalent linker, which includes a coupling group. Other illustrative intermediates useable in such routes include intermediates comprising a vinca alkaloid, or analog or derivative thereof, attached to a bivalent linker, which includes a coupling group. In either case, the coupling group may be a nucleophile, an electrophile, or a precursor thereof.

In one illustrative embodiment synthetic intermediates, the coupling group is a Michael acceptor, and the bivalent linker includes a releasable linker having the formula -C(O)NHN=, -NHC(O)NHN=, or -CH₂C(O)NHN=. In one illustrative aspect, the coupling group and the bivalent linker are taken together to form a compound having the formula: 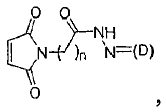 or a protected derivative thereof, where (D) is the vinca alkaloid, or an analog or a derivative thereof, capable of forming a hydrazone as illustrated herein; and n is an integer such as 1, 2, 3, or 4. In another illustrative aspect of the receptor binding drug delivery conjugate intermediate described herein, a second linker is covalently attached to the above formula through an alkylthiol nucleophile included on the second linker. In another illustrative aspect, the receptor binding moiety, or analog or derivative thereof, is covalently attached to the above formula through an alkylthiol nucleophile included on that moiety.

In another illustrative embodiment, the coupling group is a heteroatom such as nitrogen, oxygen, or sulfur, and the bivalent linker includes one or more heteroatom linkers and one or more spacer linkers covalently connecting the receptor binding moiety to the coupling group. In one illustrative aspect, the intermediate described herein includes a compound having the formula: 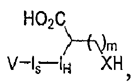 or a protected derivative thereof, where X is oxygen, nitrogen, or sulfur, and m is an integer such as 1, 2, or 3, and where (B), lₛ, and l_{H} are as defined herein. In one illustrative aspect, l_{H} is -NH-, and m is 1. In another illustrative aspect, l_{H} is -NH-, m is 1, and X is -S-.

In another illustrative embodiment, the intermediate described herein includes a compound having the formula: 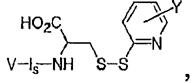 or a protected derivative thereof, where Y is H or a substituent, illustratively an electron withdrawing substituent, including but not limited to nitro, cyano, halo, alkylsulfonyl, a carboxylic acid derivative, and the like, and where (B) and lₛ are as defined herein.

In another illustrative embodiment of the intermediate described herein, the coupling group is a Michael acceptor, and the bivalent linker includes one or more heteroatom linkers and one or more spacer linkers covalently connecting the receptor binding moiety to the coupling group. In one illustrative aspect, the coupling group and the bivalent linker are taken together to form a compound having the formula: 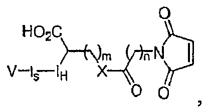 or a protected derivative thereof, where X is oxygen, nitrogen, or sulfur, and m and n are independently selected integers, such as 1, 2, or 3, and where (B), lₛ, and l_{H} are as defined herein. In another illustrative aspect, the vinca alkaloid, or analog or derivative thereof, is covalently attached to the above formula through an alkylthiol nucleophile included on the vinca alkaloid.

In another illustrative aspect, the intermediate includes compounds having the formulae: 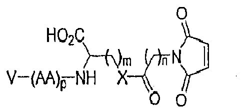 or 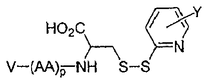 or protected derivatives thereof, where AA is one or more amino acids, illustratively selected from the naturally occurring amino acids, or stereoisomers thereof, X is nitrogen, oxygen, or sulfur, Y is hydrogen or a substituent, illustratively an electron withdrawing substituent, including but not limited to nitro, cyano, halo, alkylsulfonyl, a carboxylic acid derivative, and the like, n and m are independently selected integers, such as 1, 2, or 3, and p is an integer such as 1, 2, 3, 4, or 5. AA can also be any other amino acid, such as any amino acid having the general formula:

-N(R)-(CR'R")_{q}-C(O)-

where R is hydrogen, alkyl, acyl, or a suitable nitrogen protecting group, R' and R" are hydrogen or a substiuent, each of which is independently selected in each occurrence, and q is an integer such as 1, 2, 3, 4, or 5. illustratively, R' and/or R" independently correspond to, but are not limited to, hydrogen or the side chains present on naturally occurring amino acids, such as methyl, benzyl, hydroxymethyl, thiomethyl, carboxyl, carboxylmethyl, guanidinopropyl, and the like, and derivatives and protected derivatives thereof. The above described formula includes all stereoisomeric variations. For example, the amino acid may be selected from asparagine, aspartic acid, cysteine, glutamic acid, lysine, glutamine, arginine, serine, ornitine, threonine, and the like. In another illustrative aspect of the vitamin receptor binding drug delivery conjugate intermediate described herein, the drug, or an analog or a derivative thereof, includes an alkylthiol nucleophile.

Each of the above intermediates may be prepared using conventional synthetic routes. Additional synthetic routes and reaction conditions are described in U.S. patent application Serial No. 10/765,336 and PCT international patent application Serial No. US/2005/026068.

The foregoing illustrative embodiments are intended to be illustrative of the invention described herein, and should not be interpreted or construed as limiting in any way the invention as described herein. For example, compounds generally represented by the following illustrative vitamin-drug conjugate are to be included in the invention as described herein 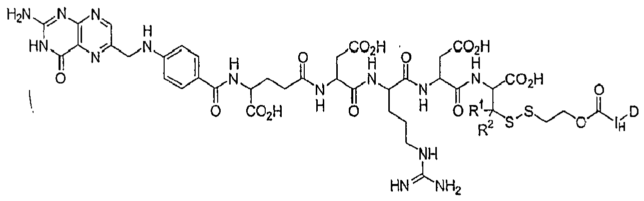 where R¹ and R² are each independently hydrogen or alkyl, such as methyl; and l_{H} is a heteroatom, such as oxygen, sulfur, optionally substituted nitrogen, or optionally protected nitrogen, and the like.

In another embodiment, the compounds described herein include a bivalent linker formed from a releasable linker that includes a ketal group. In one aspect, the ketal group is an optionally substituted ketal of 2-, or 4-oxybenzaldehyde, such as a 4-oxybenzaldehyde of the formula: 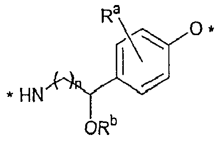 where n is selected from 1, 2, 3, and 4; Ra is an alkyl or optionally substituted aryalkyl, Ra is hydrogen or an optional substitution; and the (*) atoms are each attached to the receptor binding moiety, the bivalent linker, or the vinca alkaloid, or an analog or derivative thereof.

In another embodiment, the compounds described herein include a bivalent linker formed from a releasable linker that includes a carbonate. In one aspect, the carbonate is a bis alkyl carbonate. In another aspect, the carbonate is a bisalkylcarbonate including a dithio group and an amino group or hydrazino group. In another aspect, the carbonate is a structure of the formula: 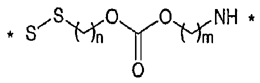 where n and m integers each indendently selected from 1, 2, 3, and 4; and the (*) atoms are each attached to the receptor binding moiety, the bivalent linker, or the vinca alkaloid, or an analog or derivative thereof.

In another embodiment, the compounds described herein include a bivalent linker formed from a releasable linker that includes a bivalent dithioalkylamino group or a bivalent dithiobenzyloxycarbonyl group. In one aspect, the bivalent dithioalkylamino group is structure of the formula: 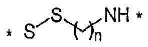 where n is selected from 1, 2, 3, and 4; and the (*) atoms are each attached to the receptor binding moiety, the bivalent linker, or the vinca alkaloid, or an analog or derivative thereof. In another aspect, the bivalent dithiobenzyloxycarbonyl group is structure of the formula: 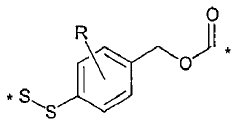 where R is hydrogen or an optional substitution,; and the (*) atoms are each attached to the receptor binding moiety, the bivalent linker, or the vinca alkaloid, or an analog or derivative thereof. In another aspect, the bivalent dithiobenzyloxycarbonyl group is structure of the formula: 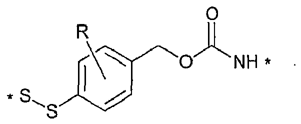 where R is hydrogen, alkyl, alkoxy, cyano, or nitro; and the (*) atoms are each attached to the receptor binding moiety, the bivalent linker, or the vinca alkaloid, or an analog or derivative thereof. In another aspect, the bivalent dithiobenzyloxycarbonyl group is structure of the formula: 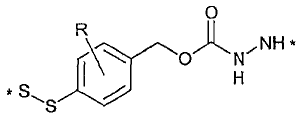 where R is hydrogen, alkyl, alkoxy, cyano, or nitro; and the (*) atoms are each attached to the receptor binding moiety, the bivalent linker, or the vinca alkaloid, or an analog or derivative thereof.

In another embodiment, the compounds described herein includes a vinca alkaloid, or an analog or derivative thereof that includes a carboxamide that is attached to the bivalent linker through the nitrogen to form a conjugate. In another embodiment, the compounds described herein includes a vinca alkaloid, or an analog or derivative thereof that includes a carboxhydrazide that is attached to the bivalent linker through one the nitrogen atoms to form a conjugate. In one aspect, the that attachment is made through the terminal nitrogen. In another embodiment, the compounds described herein includes a vinca alkaloid, or an analog or derivative thereof that includes a carboxylate that is attached to the bivalent linker through the oxygen to form a conjugate.

In another illustrative embodiment, the receptor binding moiety (B) is not folate when the linker (L)-(D) is the following structure: 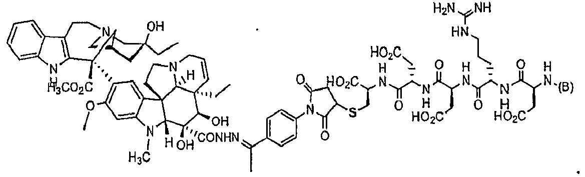 In another illustrative embodiment, the receptor binding moiety (B) is not folate when the linker (L)-(D) is the following structure:

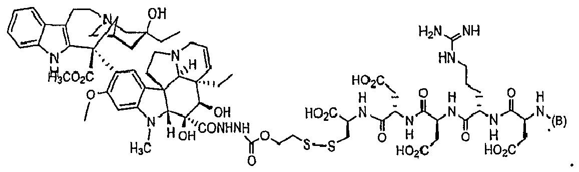

In another embodiment, a pharmaceutical composition is described. The pharmaceutical composition comprises a drug delivery conjugate described herein in combination with a pharmaceutically acceptable carrier, excipient, and/or diluent therefor.

In another embodiment, a method for eliminating a population of pathogenic cells in a host animal harboring the population of pathogenic cells is described. In one illustrative aspect, the members of the pathogenic cell population have an accessible binding site for a receptor binding moiety, or the analog or derivative thereof, and that binding site is uniquely expressed, overexpressed, or preferentially expressed by the pathogenic cells. The method includes the step of administering to the host a drug delivery conjugate described herein, or a pharmaceutical composition thereof, as described herein.

The drug delivery conjugates described herein can be used for both human clinical medicine and veterinary applications. Thus, the host animal harboring the population of pathogenic cells and treated with the drug delivery conjugates can be human or, in the case of veterinary applications, can be a laboratory, agricultural, domestic, or wild animal. The drug delivery conjugates described herein can be administered to host animals including, but not limited to, humans, laboratory animals such rodents (e.g., mice, rats, hamsters, etc.), rabbits, monkeys, chimpanzees, domestic animals such as dogs, cats, and rabbits, agricultural animals such as cows, horses, pigs, sheep, goats, and wild animals in captivity such as bears, pandas, lions, tigers, leopards, elephants, zebras, giraffes, gorillas, dolphins, and whales.

The drug delivery conjugates described herein can be used to treat a variety of pathologies and pathogenic cells in host animals. As used herein, "pathogenic cells" means cancer cells, infectious agents such as bacteria and viruses, bacteria- or virus-infected cells, activated macrophages capable of causing a disease state, and any other type of pathogenic cells that uniquely express, preferentially express, or overexpress ligand receptors, such as vitamin receptors or receptors that bind analogs or derivatives of vitamins. Pathogenic cells can also include any cells causing a disease state for which treatment with the drug delivery conjugates results in reduction of the symptoms of the disease. The pathogenic cells can also be host cells that are pathogenic under some circumstances, such as cells of the immune system that are responsible for graft versus host disease, but not pathogenic under other circumstances.

Thus, the population of pathogenic cells can be a cancer cell population that is tumorigenic, including benign tumors and malignant tumors, or it can be non-tumorigenic. The cancer cell population can arise spontaneously or by such processes as mutations present in the germline of the host animal or somatic mutations, or it can be chemically, virally, or radiation-induced. The invention can be utilized to treat such cancers as carcinomas, sarcomas, lymphomas, Hodgekin's disease, melanomas, mesotheliomas, Burkitt's lymphoma, nasopharyngeal carcinomas, leukemias, and myelomas. The cancer cell population can include, but is not limited to, oral, thyroid, endocrine, skin, gastric, esophageal, laryngeal, pancreatic, colon, bladder, bone, ovarian, cervical, uterine, breast, testicular, prostate, rectal, kidney, liver, and lung cancers.

In embodiments where the pathogenic cell population is a cancer cell population, the effect of drug delivery conjugate administration is a therapeutic response measured by reduction or elimination of tumor mass or of inhibition of tumor cell proliferation. In the case of a tumor, the elimination can be an elimination of cells of the primary tumor or of cells that have metastasized or are in the process of dissociating from the primary tumor. A prophylactic treatment with the drug delivery conjugate to prevent return of a tumor after its removal by any therapeutic approach including surgical removal of the tumor, radiation therapy, chemotherapy, or biological therapy is also contemplated. The prophylactic treatment can be an initial treatment with the drug delivery conjugate, such as treatment in a multiple dose daily regimen, and/or can be an additional treatment or series of treatments after an interval of days or months following the initial treatment(s). Accordingly, elimination of any of the pathogenic cell populations described above includes reduction in the number of pathogenic cells, inhibition of proliferation of pathogenic cells, a prophylactic treatment that prevents return of pathogenic cells, or a treatment of pathogenic cells that results in reduction of the symptoms of disease.

In cases where cancer cells are being eliminated, the method described herein can be used in combination with surgical removal of a tumor, radiation therapy, chemotherapy, or biological therapies such as other immunotherapies including, but not limited to, monoclonal antibody therapy, treatment with immunomodulatory agents, adoptive transfer of immune effector cells, treatment with hematopoietic growth factors, cytokines and vaccination.

The method described herein is also applicable to populations of pathogenic cells that cause a variety of infectious diseases. For example, the present invention is applicable to such populations of pathogenic cells as bacteria, fungi, including yeasts, viruses, virus-infected cells, mycoplasma, and parasites. Infectious organisms that can be treated with the drug delivery conjugates described herein are any art-recognized infectious organisms that cause pathogenesis in an animal, including such organisms as bacteria that are gram-negative or gram-positive cocci or bacilli. For example, Proteus species, Klebsiella species, Providencia species, Yersinia species, Erwinia species, Enterobacter species, Salmonella species, Serratia species, Aerobacter species, Escherichia species, Pseudomonas species, Shigella species, Vibrio species, Aeromonas species, Campylobacter species, Streptococcus species, Staphylococcus species, Lactobacillus species, Micrococcus species, Moraxella species, Bacillus species, Clostridium species, Corynebacterium species, Eberthella species, Micrococcus species, Mycobacterium species, Neisseria species, Haemophilus species, Bacteroides species, Listeria species, Erysipelothrix species, Acinetobacter species, Brucella species, Pasteurella species, Vibrio species, Flavobacterium species, Fusobacterium species, Streptobacillus species, Calymmatobacterium species, Legionella species, Treponema species, Borrelia species, Leptospira species, Actinomyces species, Nocardia species, Rickettsia species, and any other bacterial species that causes disease in a host animal can be treated with the drug delivery conjugates described herein.

Of particular interest are bacteria that are resistant to antibiotics such as antibiotic-resistant Streptococcus species and Staphlococcus species, or bacteria that are susceptible to antibiotics, but cause recurrent infections treated with antibiotics so that resistant organisms eventually develop. Bacteria that are susceptible to antibiotics, but cause recurrent infections treated with antibiotics so that resistant organisms eventually develop, can be treated with the drug delivery conjugates described herein in the absence of antibiotics, or in combination with lower doses of antibiotics than would normally be administered to a host animal, to avoid the development of these antibiotic-resistant bacterial strains.

Diseases caused by viruses, such as DNA and RNA viruses, can also be treated with the drug delivery conjugates described herein. Such viruses include, but are not limited to, DNA viruses such as papilloma viruses, parvoviruses, adenoviruses, herpesviruses and vaccinia viruses, and RNA viruses, such as arenaviruses, coronaviruses, rhinoviruses, respiratory syncytial viruses, influenza viruses, picornaviruses, paramyxoviruses, reoviruses, retroviruses, lentiviruses, and rhabdoviruses.

The drug delivery conjugates described herein can also be used to treat diseases caused by any fungi, including yeasts, mycoplasma species, parasites, or other infectious organisms that cause disease in animals. Examples of fungi that can be treated with the method and drug delivery conjugates described herein include fungi that grow as molds or are yeastlike, including, for example, fungi that cause diseases such as ringworm, histoplasmosis, blastomycosis, aspergillosis, cryptococcosis, sporotrichosis, coccidioidomycosis, paracoccidio-idomycosis, mucormycosis, chromoblastomycosis, dermatophytosis, protothecosis, fusariosis, pityriasis, mycetoma, paracoccidioidomycosis, phaeohyphomycosis, pseudallescheriasis, sporotrichosis, trichosporosis, pneumocystis infection, and candidiasis.

The drug delivery conjugates described herein can also be used to treat parasitic infections including, but not limited to, infections caused by tapeworms, such as Taenia, Hymenolepsis, Diphyllobothrium, and Echinococcus species, flukes, such as Fasciolopsis, Heterophyes, Metagonimus, Clonorchis, Fasciola, Paragonimus, and Schitosoma species, roundworms, such as Enterobius, Trichuris, Ascaris, Ancylostoma, Necator, Strongyloides, Trichinella, Wuchereria, Brugia, Loa Onchocerca, and Dracunculus species, ameba, such as Naegleria and Acanthamoeba species, and protozoans, such as Plasmodium, Trypanosoma, Leishmania, Toxoplasma, Entamoeba, Giardia, Isospora, Cryptosporidium, and Enterocytozoon species.

The pathogenic cells to which the drug delivery conjugates are directed can also be cells harboring endogenous pathogens, such as virus-, mycoplasma-, parasite-, or bacteria-infected cells, if these cells preferentially express ligand receptors, such as receptors for vitamins, or analogs or derivatives thereof.

In one embodiment, the drug delivery conjugates can be internalized into the targeted pathogenic cells upon binding of the ligand to a receptor, transporter, or other surface-presented protein that specifically binds the ligand and which is preferentially expressed on the pathogenic cells. Such internalization can occur, for example, through receptor-mediated endocytosis. If the drug delivery conjugate contains a releasable linker, the ligand and the vinca compound can dissociate intracellularly and the vinca can act on its intracellular target.

In another illustrative embodiment, the ligand of the drug delivery conjugate can bind to the pathogenic cell placing the vinca compound in close association with the surface of the pathogenic cell. The vinca compound can then be released by cleavage of the releasable linker. For example, the vinca compound can be released by a protein disulfide isomerase if the releasable linker is a disulfide group. The vinca compound can then be taken up by the pathogenic cell to which the receptor binding drug delivery conjugate is bound, or the vinca compound can be taken up by another pathogenic cell in close proximity thereto. Alternatively, the vinca compound could be released by a protein disulfide isomerase inside the cell where the releasable linker is a disulfide group. The vinca compound may also be released by a hydrolytic mechanism, such as acid-catalyzed hydrolysis, as described above for certain beta elimination mechanisms, or by an anchimerically assisted cleavage through an oxonium ion or lactonium ion producing mechanism. The selection of the releasable linker or linkers will dictate the mechanism by which the vinca compound is released from the conjugate. It is appreciated that such a selection can be pre-defined by the conditions under which the drug delivery conjugate will be used.

In another illustrative embodiment, where the linker does not comprise a releasable linker, the ligand moiety of the drug delivery conjugate can bind to the pathogenic cell placing the vinca compound on the surface of the pathogenic cell to target the pathogenic cell for attack by other molecules capable of binding to the vinca compound. Alternatively, in this embodiment, the drug delivery conjugates can be internalized into the targeted cells upon binding, and the ligand moiety and the vinca compound can remain associated intracellularly with the vinca compound exhibiting its effects without dissociation from the ligand moiety.

In still another embodiment, or in combination with the above-described embodiments, where the drug delivery conjugate binds a vitamin receptor or another ligand receptor, the conjugate can bind to soluble vitamin receptors present in the serum or to serum proteins, such as albumin, resulting in prolonged circulation of the conjugates relative to the unconjugated vinca compound, and in increased activity of the conjugates towards the pathogenic cell population relative to the unconjugated vinca compound.

The binding site for the ligand, such as a vitamin, can include receptors for the ligand capable of specifically binding to the ligand wherein the receptor or other protein is uniquely expressed, overexpressed, or preferentially expressed by a population of pathogenic cells. A surface-presented protein uniquely expressed, overexpressed, or preferentially expressed by the pathogenic cells is typically a receptor that is either not present or present at lower concentrations on non-pathogenic cells providing a means for selective elimination of the pathogenic cells. The drug delivery conjugates may be capable of high affinity binding to receptors on cancer cells or other types of pathogenic cells. The high affinity binding can be inherent to the ligand or the binding affinity can be enhanced by the use of a chemically modified ligand.

The drug delivery conjugates described herein can be administered in a combination therapy with any other known drug whether or not the additional drug is targeted. Illustrative additional drugs include, but are not limited to, peptides, oligopeptides, retro-inverso oligopeptides, proteins, protein analogs in which at least one non-peptide linkage replaces a peptide linkage, apoproteins, glycoproteins, enzymes, coenzymes, enzyme inhibitors, amino acids and their derivatives, receptors and other membrane proteins, antigens and antibodies thereto, haptens and antibodies thereto, hormones, lipids, phospholipids, liposomes, toxins, antibiotics, analgesics, bronchodilators, beta-blockers, antimicrobial agents, antihypertensive agents, cardiovascular agents including antiarrhythmics, cardiac glycosides, antianginals, vasodilators, central nervous system agents including stimulants, psychotropics, antimanics, and depressants, antiviral agents, antihistamines, cancer drugs including chemotherapeutic agents, tranquilizers, anti-depressants, H-2 antagonists, anticonvulsants, antinauseants, prostaglandins and prostaglandin analogs, muscle relaxants, anti-inflammatory substances, stimulants, decongestants, antiemetics, diuretics, antispasmodics, antiasthmatics, anti-Parkinson agents, expectorants, cough suppressants, mucolytics, and mineral and nutritional additives.

In another illustrative aspect, the additional drug can be selected from a compound capable of stimulating an endogenous immune response. Suitable compounds include, but are not limited to, cytokines or immune cell growth factors such as interleukins 1-18, stem cell factor, basic FGF, EGF, G-CSF, GM-CSF, FLK-2 ligand, HILDA, MIP-1α, TGF-α, TGF-β, M-CSF, IFN-α, IFN-β, IFN-γ, soluble CD23, LIF, and combinations thereof.

Therapeutically effective combinations of these immunostimulatory factors can be used. In one embodiment, for example, therapeutically effective amounts of IL-2, for example, in amounts ranging from about 0.1 MIU/m²/dose/day to about 15 MIU/m²/dose/day in a multiple dose daily regimen, and IFN-α, for example, in amounts ranging from about 0.1 MIU/m²/dose/day to about 7.5 MIU/m²/dose/day in a multiple dose daily regimen, can be used along with the drug delivery conjugates to eliminate, reduce, or neutralize pathogenic cells in a host animal harboring the pathogenic cells (MIU = million international units; m² = approximate body surface area of an average human). In another embodiment IL-12 and IFN-α can be used in the above-described therapeutically effective amounts for interleukins and interferons, and in yet another embodiment IL-15 and IFN-α can be used in the above described therapeutically effective amounts for interleukins and interferons. In an alternate embodiment IL-2, IFN-α or IFN-γ, and GM-CSF can be used in combination in the above described therapeutically effective amounts. Any other effective combination of cytokines including combinations of other interleukins and interferons and colony stimulating factors can also be used.

Further, the additional drug can be any drug known in the art which is cytotoxic or cytostatic, enhances tumor permeability, inhibits tumor cell proliferation, promotes apoptosis, decreases anti-apoptotic activity in target cells, is used to treat diseases caused by infectious agents, enhances an endogenous immune response directed to the pathogenic cells, or is useful for treating a disease state caused by any type of pathogenic cell. Exemplary suitable additional drugs include adrenocorticoids and corticosteroids, alkylating agents, antiandrogens, antiestrogens, androgens, aclamycin and aclamycin derivatives, estrogens, antimetabolites such as cytosine arabinoside, purine analogs, pyrimidine analogs, and methotrexate, busulfan, carboplatin, chlorambucil, cisplatin and other platinum compounds, tamoxiphen, taxol, paclitaxel, paclitaxel derivatives, Taxotere^{®}, cyclophosphamide, daunomycin, rhizoxin, T2 toxin, plant alkaloids, prednisone, hydroxyurea, teniposide, mitomycins, discodermolides, non-vinca microtubule inhibitors, epothilones, tubulysin, cyclopropyl benz[e]indolone, seco-cyclopropyl benz[e]indolone, *O-Ac-seco-*cyclopropyl benz[e]indolone, bleomycin and any other antibiotic, nitrogen mustards, nitrosureas, colchicine, colchicine derivatives, allocolchicine, thiocolchicine, trityl cysteine, Halicondrin B, dolastatins such as dolastatin 10, amanitins such as α-amanitin, camptothecin, irinotecan, and other camptothecin derivatives thereof, geldanamycin and geldanamycin derivatives, estramustine, nocodazole, MAP4, colcemid, vindesine, vinblastine, vincristine, catharanthine, vindoline, leurosine, vinorelbine, imidocarb, sibutramine, toltrazuril, vinblastinoic acid, maytansines and analogs and derivatives thereof, gemcitabine, inflammatory and proinflammatory agents, peptide and peptidomimetic signal transduction inhibitors, and any other art-recognized drug or toxin. Other drugs that can be used in combination therapies include penicillins, cephalosporins, vancomycin, erythromycin, clindamycin, rifampin, chloramphenicol, aminoglycoside antibiotics, gentamicin, amphotericin B, acyclovir, trifluridine, ganciclovir, zidovudine, amantadine, ribavirin, and any other art-recognized antimicrobial compound. Analogs or derivatives of any of the above-described additional drugs can also be used in combination therapies.

In another illustrative embodiment, pharmaceutical compositions are provided. The pharmaceutical compositions comprise an amount of a drug delivery conjugate effective to eliminate a population of pathogenic cells in a host animal when administered in one or more doses. The drug delivery conjugate is preferably administered to the host animal parenterally, e.g., intradermally, subcutaneously, intramuscularly, intraperitoneally, intravenously, or intrathecally. Alternatively, the drug delivery conjugate can be administered to the host animal by other medically useful processes, such as orally, and any effective dose and suitable therapeutic dosage form, including prolonged release dosage forms, can be used. Exemplary excipients useful for oral dosage forms include, but are not limited to, com starch, gelatin, lactose, magnesium stearate, sodium bicarbonate, cellulose derivatives, and sodium starch glycolate.

Examples of parenteral dosage forms include aqueous solutions of the active agent, in an isotonic saline, 5% glucose or other well-known pharmaceutically acceptable liquid carriers such as liquid alcohols, glycols, esters, and amides. The parenteral dosage form in accordance with this invention can be in the form of a reconstitutable lyophilizate comprising the dose of the drug delivery conjugate. In one aspect of the present embodiment, any of a number of prolonged release dosage forms known in the art can be administered such as, for example, the biodegradable carbohydrate matrices described in U.S. Patents Nos. 4,713,249; 5,266,333; and 5,417,982, the disclosures of which are incorporated herein by reference, or, alternatively, a slow pump (e.g., an osmotic pump) can be used.

The additional drug in the combination therapy can be administered to the host animal prior to, after, or at the same time as the drug delivery conjugates and the additional drug can be administered as part of the same composition containing the drug delivery conjugate or as part of a different composition than the drug delivery conjugate. Any such combination therapy at an effective dose of the additional drug can be used.

In another illustrative aspect, more than one type of drug delivery conjugate can be used. For example, the host animal can be treated in a co-dosing protocol with conjugates with different ligands such as, for example, folate-vinca and vitamin B₁₂-vinca conjugates in combination, and the like. In another illustrative embodiment, the host animal can be treated with conjugates comprising more than one ligand such as, for example, multiple folates or multiple vitamin B₁₂ molecules in one conjugate, or combinations of ligands in the same conjugate such as a vinca compound conjugated to both folate and vitamin B₁₂ ligands. Furthermore, drug delivery conjugates with different types of vinca compounds in separate drug delivery conjugates can be used.

The unitary daily dosage of the drug delivery conjugate can vary significantly depending on the host condition, the disease state being treated, the molecular weight of the conjugate, its route of administration and tissue distribution, and the possibility of co-usage of other therapeutic treatments such as radiation therapy or additional drugs in combination therapies. The effective amount to be administered to a host animal is based on body surface area, weight, and physician assessment of patient condition. Effective doses can range, for example, from about 1 ng/kg to about 1 mg/kg, from about 1 µg/kg to about 500 µg/kg, and from about 1 µg/kg to about 100 µg/kg.

Any effective regimen for administering the drug delivery conjugates can be used. For example, the drug delivery conjugates can be administered as single doses, or can be divided and administered as a multiple-dose daily regimen. Further, a staggered regimen, for example, one to three days per week can be used as an alternative to daily treatment, and for the purpose of defining this invention such intermittent or staggered daily regimen is considered to be equivalent to every day treatment and is contemplated. In one illustrative embodiment the host animal is treated with multiple injections of the drug delivery conjugate to eliminate the population of pathogenic cells. In one embodiment, the host is injected multiple times (preferably about 2 up to about 50 times) with the drug delivery conjugate, for example, at 12-72 hour intervals or at 48-72 hour intervals. Additional injections of the drug delivery conjugate can be administered to the host animal at an interval of days or months after the initial injections(s) and the additional injections can prevent recurrence of the disease state caused by the pathogenic cells.

In one illustrative aspect, vitamins, or analogs or derivatives thereof, that can be used in the drug delivery conjugates include those that bind to receptors expressed specifically on activated macrophages, such as the folate receptor which binds folate, or an analog or derivative thereof. The folate-linked conjugates, for example, can be used to kill or suppress the activity of activated macrophages that cause disease states in the host. Such macrophage targeting conjugates, when administered to a host animal suffering from an activated macrophage-mediated disease state, work to concentrate and associate the conjugated vinca compounds in the population of activated macrophages to kill the activated macrophages or suppress macrophage function. Elimination, reduction, or deactivation of the activated macrophage population works to stop or reduce the activated macrophage-mediated pathogenesis characteristic of the disease state being treated. Exemplary of diseases known to be mediated by activated macrophages include rheumatoid arthritis, ulcerative colitis, Crohn's disease, psoriasis, osteomyelitis, multiple sclerosis, atherosclerosis, pulmonary fibrosis, sarcoidosis, systemic sclerosis, organ transplant rejection (GVHD) and chronic inflammations. Administration of the drug delivery conjugate is typically continued until symptoms of the disease state are reduced or eliminated.

The drug delivery conjugates administered to kill activated macrophages or suppress the function of activated macrophages can be administered parenterally to the host animal, for example, intradermally, subcutaneously, intramuscularly, intraperitoneally, or intravenously in combination with a pharmaceutically acceptable carrier. Alternatively, the drug delivery conjugates can be administered to the host animal by other medically useful procedures and effective doses can be administered in standard or prolonged release dosage forms. The therapeutic method can be used alone or in combination with other therapeutic methods recognized for treatment of disease states mediated by activated macrophages.

The invention also provides:
1. A receptor binding drug delivery conjugate comprising:
   (a) a receptor binding moiety;
   (b) a bivalent linker; and
   (c) a vinca alkaloid, or an analog or derivative thereof;
   wherein the receptor binding moiety is covalently linked to the bivalent linker;
   the vinca alkaloid, or the analog or the derivative thereof, is covalently linked to the bivalent linker; and
   the bivalent linker comprises one or more components selected from the group consisting of spacer linkers, releasable linkers, and heteroatom linkers, and combinations thereof.
2. A vitamin receptor binding drug delivery conjugate having the formula:

   (B)-(L)-(D)

   wherein (L) is selected from the group consisting of (lₛ)ₐ, (l_{H})_{b}, and (lᵣ)_{c}, and combinations thereof; where (lᵣ) is a releasable linker, (lₛ) is a spacer linker, and (l_{H}) is an heteroatom linker;
   (B) is a vitamin receptor binding moiety, and (D) is a drug, or an analog or a derivative thereof; and
   a, b, and c are each independently 0, 1, 2, 3, or 4.
3. A vitamin receptor binding drug delivery conjugate having the formula:

   (B)-(L)-(D)

   wherein (L) is selected from the group consisting of (lₛ)ₐ and (l_{H})_{b}, and combinations thereof; where (lₛ) is a spacer linker and (l_{H}) is an heteroatom linker; and a and b are each independently 0, 1, 2, 3, or 4; and
   (B) is a vitamin receptor binding moiety, and (D) is a vinca alkaloid, or an analog or a derivative thereof.
4. The drug delivery conjugate of 1, 2, or 3 wherein the bivalent linker includes a releasable linker comprising a ketal.
5. The drug delivery conjugate of 4 wherein at least one spacer linker is a peptide.
6. The drug delivery conjugate of 1, 2, or 3 wherein the bivalent linker includes a releasable linker of the formula: 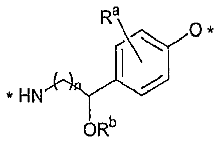 where n is selected from 1, 2, 3, and 4; Ra is an alkyl or optionally substituted aryalkyl, Ra is hydrogen or an optional substitution; and the (*) atoms are each attached to the receptor binding moiety, the bivalent linker, or the vinca alkaloid, or an analog or derivative thereof.
7. The drug delivery conjugate of 1, 2, or 3 wherein the bivalent linker includes a releasable linker comprising a carbonate.
8. The drug delivery conjugate of 4 wherein at least one spacer linker is a peptide.
9. The drug delivery conjugate of 1, 2, or 3 wherein the bivalent linker includes a releasable linker of the formula: 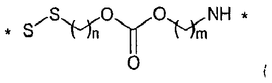 where n and m integers each indendently selected from 1, 2, 3, and 4; and the (*) atoms are each attached to the receptor binding moiety, the bivalent linker, or the vinca alkaloid, or an analog or derivative thereof.
10. The drug delivery conjugate of 1, 2, or 3 wherein the bivalent linker includes a releasable linker comprising a bivalent dithioalkylamino group or a bivalent dithiobenzyloxycarbonyl group.
11. The drug delivery conjugate of 4 wherein at least one spacer linker is a peptide.
12. The drug delivery conjugate of 1, 2, or 3 wherein the bivalent linker includes a releasable linker of the formula: 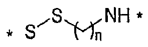 where n is selected from 1, 2, 3, and 4; and the (*) atoms are each attached to the receptor binding moiety, the bivalent linker, or the vinca alkaloid, or an analog or derivative thereof.
13. The drug delivery conjugate of 1, 2, or 3 wherein the bivalent linker includes a releasable linker of the formula: 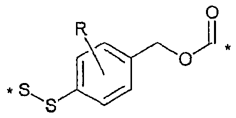 where R is hydrogen or an optional substitution,; and the (*) atoms are each attached to the receptor binding moiety, the bivalent linker, or the vinca alkaloid, or an analog or derivative thereof.
14. The drug delivery conjugate of 1, 2, or 3 wherein the bivalent linker includes a releasable linker of the formula: 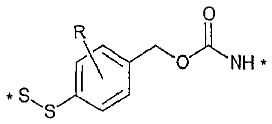 where R is hydrogen, alkyl, alkoxy, cyano, or nitro; and the (*) atoms are each attached to the receptor binding moiety, the bivalent linker, or the vinca alkaloid, or an analog or derivative thereof.
15. The drug delivery conjugate of 1, 2, or 3 wherein the bivalent linker includes a releasable linker of the formula: 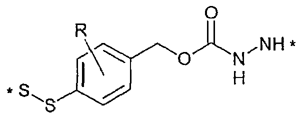 where R is hydrogen, alkyl, alkoxy, cyano, or nitro; and the (*) atoms are each attached to the receptor binding moiety, the bivalent linker, or the vinca alkaloid, or an analog or derivative thereof.
16. The drug delivery conjugate of 1, 2, or 3 wherein the vinca alkaloid, or an analog or derivative thereof includes a carboxamide attached to the bivalent linker through the nitrogen.
17. The drug delivery conjugate of 4 wherein at least one spacer linker is a peptide.
18. The drug delivery conjugate of 1, 2, or 3 wherein the vinca alkaloid, or an analog or derivative thereof includes an carboxylate attached to the bivalent linker through the oxygen.
19. The drug delivery conjugate of 4 wherein at least one spacer linker is a peptide.
20. The drug delivery conjugate of 1, 2, or 3 wherein the bivalent linker includes at least one releasable linker that is not a disulfide.
21. The drug delivery conjugate of 1, 2, or 3 wherein the heteroatom linker is a nitrogen, oxygen, or sulfur atom, or is selected from the group of formulae consisting of -NHR¹NHR²-, -SO-, -S(O)₂-, and -NR³O-, wherein R¹, R², and R³ are each independently selected from the group consisting of hydrogen, alkyl, aryl, arylalkyl, substituted aryl, substituted arylalkyl, heteroaryl, substituted heteroaryl, and alkoxyalkyl.
22. The drug delivery conjugate of 1, 2, or 3 wherein the spacer linker is selected from the group consisting of carbonyl, thionocarbonyl, alkylene, cycloalkylene, alkylenecycloalkyl, alkylenecarbonyl, cycloalkylenecarbonyl, carbonylalkylcarbonyl, 1-alkylenesuccinimid-3-yl, 1-(carbonylalkyl)succinimid-3-yl, alkylenesulfoxyl, sulfonylalkyl, alkylenesulfoxylalkyl, alkylenesulfonylalkyl, carbonyltetrahydro-2H-pyranyl, carbonyltetrahydrofuranyl, 1-(carbonyltetrahydro-2H-pyranyl)succinimid-3-yl, and 1-(carbonyltetrahydrofuranyl)succinimid-3-yl, wherein each of said spacer linkers is optionally substituted with one or more substituents X¹;
   wherein each substituent X¹ is independently selected from the group consisting of alkyl, alkoxy, alkoxyalkyl, hydroxy, hydroxyalkyl, amino, aminoalkyl, alkylaminoalkyl, dialkylaminoalkyl, halo, haloalkyl, sulfhydrylalkyl, alkylthioalkyl, aryl, substituted aryl, arylalkyl, substituted arylalkyl, heteroaryl, substituted heteroaryl, carboxy, carboxyalkyl, alkyl carboxylate, alkyl alkanoate, guanidinoalkyl, R⁴-carbonyl, R⁵-carbonylalkyl, R⁶-acylamino, and R⁷-acylaminoalkyl, wherein R⁴ and R⁵ are each independently selected from the group consisting of an amino acid, an amino acid derivative, and a peptide, and wherein R⁶ and R⁷ are each independently selected from the group consisting of an amino acid, an amino acid derivative, and a peptide.
23. The drug delivery conjugate of 22 wherein the heteroatom linker is nitrogen, and wherein the substituent X¹ and the heteroatom linker are taken together with the spacer linker to which they are bound to form an heterocycle.
24. The drug delivery conjugate of 23 wherein the heterocycle is selected from the group consisting ofpyrrolidines, piperidines, oxazolidines, isoxazolidines, thiazolidines, isothiazolidines, pyrrolidinones, piperidinones, oxazolidinones, isoxazolidinones, thiazolidinones, isothiazolidinones, and succinimides.
25. The drug delivery conjugate of 1, 2, or 3 wherein the releasable linker is selected from the group consisting of methylene, 1-alkoxyalkylene, 1-alkoxycycloalkylene, 1-alkoxyalkylenecarbonyl, 1-alkoxycycloalkylenecarbonyl, carbonylarylcarbonyl, carbonyl(carboxyaryl)carbonyl, carbonyl(biscarboxyaryl)carbonyl, haloalkylenecarbonyl, alkylene(dialkylsilyl), alkylene(alkylarylsilyl), alkylene(diarylsilyl), (dialkylsilyl)aryl, (alkylarylsilyl)aryl, (diarylsilyl)aryl, oxycarbonyloxy, oxycarbonyloxyalkyl, sulfonylalkyl, iminoalkylidenyl, carbonylalkylideniminyl, iminocycloalkylidenyl, carbonylcycloalkylideniminyl, alkylenesulfonyl, alkylenethio, alkylenearylthio, and carbonylalkylthio, wherein each of said releasable linkers is optionally substituted with one or more substituents X²;
   wherein each substituent X² is independently selected from the group consisting of alkyl, alkoxy, alkoxyalkyl, hydroxy, hydroxyalkyl, amino, aminoalkyl, alkylaminoalkyl, dialkylaminoalkyl, halo, haloalkyl, sulfhydrylalkyl, alkylthioalkyl, aryl, substituted aryl, arylalkyl, substituted arylalkyl, heteroaryl, substituted heteroaryl, carboxy, carboxyalkyl, alkyl carboxylate, alkyl alkanoate, guanidinoalkyl, R⁴-carbonyl, R⁵-carbonylalkyl, R⁶-acylamino, and R⁷-acylaminoalkyl, wherein R⁴ and R⁵ are each independently selected from the group consisting of an amino acid, an amino acid derivative, and a peptide, and wherein R⁶ and R⁷ are each independently selected from the group consisting of an amino acid, an amino acid derivative, and a peptide.
26. The drug delivery conjugate of 25 wherein the heteroatom linker is nitrogen, and wherein the substituent X² and the heteroatom linker are taken together with the releasable linker to which they are bound to form an heterocycle.
27. The drug delivery conjugate of 26 wherein the heterocycle is selected from the group consisting of pyrrolidines, piperidines, oxazolidines, isoxazolidines, thiazolidines, isothiazolidines, pyrrolidinones, piperidinones, oxazolidinones, isoxazolidinones, thiazolidinones, isothiazolidinones, and succinimides.
28. The drug delivery conjugate of 1, 2, or 3 wherein the heteroatom linker is nitrogen, and wherein the releasable linker and the heteroatom linker are taken together to form a divalent radical comprising alkyleneaziridin-1-yl, alkylenecarbonylaziridin-1-yl, carbonylalkylaziridin-1-yl, alkylenesulfoxylaziridin-1-yl, sulfoxylalkylaziridin-1-yl, sulfonylalkylaziridin-1-yl, or alkylenesulfonylaziridin-1-yl, wherein each of said releasable linkers is optionally substituted with one or more substituents X²;
   wherein each substituent X² is independently selected from the group consisting of alkyl, alkoxy, alkoxyalkyl, hydroxy, hydroxyalkyl, amino, aminoalkyl, alkylaminoalkyl, dialkylaminoalkyl, halo, haloalkyl, sulfhydrylalkyl, alkylthioalkyl, aryl, substituted aryl, arylalkyl, substituted arylalkyl, heteroaryl, substituted heteroaryl, carboxy, carboxyalkyl, alkyl carboxylate, alkyl alkanoate, guanidinoalkyl, R⁴-carbonyl, R⁵-carbonylalkyl, R⁶-acylamino, and R⁷-acylaminoalkyl, wherein R⁴ and R⁵ are each independently selected from the group consisting of an amino acid, an amino acid derivative, and a peptide, and wherein R⁶ and R⁷ are each independently selected from the group consisting of an amino acid, an amino acid derivative, and a peptide.
29. The drug delivery conjugate of 28 wherein the heteroatom linker is nitrogen, and the releasable linker and the heteroatom linker are taken together to form a divalent radical comprising alkyleneaziridin-1-yl, carbonylalkylaziridin-1-yl, sulfoxylalkylaziridin-1-yl, or sulfonylalkylaziridin-1-yl,.
30. The drug delivery conjugate of 1,2, or 3 wherein the spacer linker is selected from the group consisting of carbonyl, thionocarbonyl, alkylenecarbonyl, cycloalkylenecarbonyl, carbonylalkylcarbonyl, and 1-(carbonylalkyl)succinimid-3-yl, wherein each of said spacer linkers is optionally substituted with one or more substituents X¹;
   wherein each substituent X¹ is independently selected from the group consisting of alkyl, alkoxy, alkoxyalkyl, hydroxy, hydroxyalkyl, amino, aminoalkyl, alkylaminoalkyl, dialkylaminoalkyl, halo, haloalkyl, sulfhydrylalkyl, alkylthioalkyl, aryl, substituted aryl, arylalkyl, substituted arylalkyl, heteroaryl, substituted heteroaryl, carboxy, carboxyalkyl, alkyl carboxylate, alkyl alkanoate, guanidinoalkyl, R⁴-carbonyl, R⁵-carbonylalkyl, R⁶-acylamino, and R⁷-acylaminoalkyl, wherein R⁴ and R⁵ are each independently selected from the group consisting of an amino acid, an amino acid derivative, and a peptide, and wherein R⁶ and R⁷ are each independently selected from the group consisting of an amino acid, an amino acid derivative, and a peptide;
   and wherein the spacer linker is bonded to the releasable linker to form an aziridine amide.
31. The drug delivery conjugate of 1, 2, or 3 wherein the vinca alkaloid is vinblastine, desacetylvinblastine, vindesine, or thiovindesine.
32. The drug delivery conjugate of 1, 2, or 3 wherein the vinca alkaloid is a derivative thereof that includes a double-bonded nitrogen atom, wherein the releasable linker is selected from the group consisting of alkylenecarbonylamino and 1-(alkylenecarbonylamino)succinimid-3-yl, and wherein the releasable linker is bonded to the drug nitrogen to form an hydrazone.
33. The drug delivery conjugate of 1,2, or 3 wherein the vinca alkaloid is a derivative thereof that includes a sulfur atom, the releasable linker is selected from the group consisting of alkylenethio and carbonylalkylthio, and wherein the releasable linker is bonded to the drug sulfur to form a disulfide.
34. The drug delivery conjugate of 1,2, or 3 wherein the heteroatom linker is oxygen, and the releasable linker is selected from the group consisting of alkylene(dialkylsilyl), alkylene(alkylarylsilyl), alkylene(diarylsilyl), (dialkylsilyl)aryl, (alkylarylsilyl)aryl, and (diarylsilyl)aryl, wherein each of said releasable linkers is optionally substituted with one or more substituents X², and the releasble linker is bonded to the oxygen to form a silanol.
35. The drug delivery conjugate of 1, 2, or 3 wherein the heteroatom linker is oxygen, the spacer linker is 1-alkylenesuccinimid-3-yl, optionally substituted with one or more substituents X¹, and the releasable linker is selected from the group consisting of methylene, 1-alkoxyalkylene, 1-alkoxycycloalkylene, 1-alkoxyalkylenecarbonyl, 1-alkoxycycloalkylenecarbonyl, wherein each of said releasable linkers is optionally substituted with one or more substituents X²;
   wherein each substituent X² is independently selected from the group consisting of alkyl, alkoxy, alkoxyalkyl, hydroxy, hydroxyalkyl, amino, aminoalkyl, alkylaminoalkyl, dialkylaminoalkyl, halo, haloalkyl, sulfhydrylalkyl, alkylthioalkyl, aryl, substituted aryl, arylalkyl, substituted arylalkyl, heteroaryl, substituted heteroaryl, carboxy, carboxyalkyl, alkyl carboxylate, alkyl alkanoate, guanidinoalkyl, R⁴-carbonyl, R⁵-carbonylalkyl, R⁶-acylamino, and R⁷-acylaminoalkyl, wherein R⁴ and R⁵ are each independently selected from the group consisting of an amino acid, an amino acid derivative, and a peptide, and wherein R⁶ and R⁷ are each independently selected from the group consisting of an amino acid, an amino acid derivative, and a peptide;
   and wherein the spacer linker and the releasable linker are each bonded to the heteroatom linker to form a succinimid-1-ylalkyl acetal or ketal.
36. The drug delivery conjugate of 1, 2, or 3 wherein the bivalent linker comprises an heteroatom linker, a spacer linker, and a releasable linker taken together to form 3-thiosuccinimid-1-ylalkyloxymethyloxy, where the methyl is optionally substituted with alkyl or substituted aryl.
37. The drug delivery conjugate of 1, 2, or 3 wherein the bivalent linker comprises an heteroatom linker, a spacer linker, and a releasable linker taken together to form 3-thiosuccinimid-1-ylalkylcarbonyl, where the carbonyl forms an acylaziridine with the drug, or analog or derivative thereof.
38. The drug delivery conjugate of 1, 2, or 3 wherein the bivalent linker comprises an heteroatom linker, a spacer linker, and a releasable linker taken together to form 1-alkoxycycloalkylenoxy.
39. The drug delivery conjugate of 1, 2, or 3 wherein the bivalent linker comprises a spacer linker, an heteroatom linker, and a releasable linker taken together to form alkyleneaminocarbonyl(dicarboxylarylene)carboxylate.
40. The drug delivery conjugate of 1, 2, or 3 wherein the bivalent linker comprises an heteroatom linker, a spacer linker, an heteroatom linker, a spacer linker, and a releasable linker taken together to form 3-thioalkylsulfonylalkyl(disubstituted silyl)oxy, where the disubstituted silyl is substituted with alkyl or optionally substituted aryl.
41. The drug delivery conjugate of 1 wherein the bivalent linker comprises a plurality of spacer linkers selected from the group consisting of the naturally occurring amino acids and stereoisomers thereof.
42. The drug delivery conjugate of 1, 2, or 3 wherein the bivalent linker comprises a releasable linker, a spacer linker, and a releasable linker taken together to form 3-dithioalkyloxycarbonyl, where the carbonyl forms a carbonate with the drug, or analog or derivative thereof.
43. The drug delivery conjugate of 1, 2, or 3 wherein the bivalent linker comprises a releasable linker, a spacer linker, and a releasable linker taken together to form 3-dithioarylalkyloxycarbonyl, where the carbonyl forms a carbonate with the drug, or analog or derivative thereof, and the aryl is optionally substituted.
44. The drug delivery conjugate of 1, 2, or 3 wherein the bivalent linker comprises a releasable linker, a spacer linker, and a releasable linker taken together to form 3-dithioalkylamino, where the amino forms a vinylogous amide with the vinca alkaloid, or analog or derivative thereof.
45. The drug delivery conjugate of 44 wherein the alkyl is ethyl.
46. The drug delivery conjugate of 1 wherein the bivalent linker comprises a releasable linker, a spacer linker, and a releasable linker taken together to form 3-dithioalkylaminocarbonyl, where the carbonyl forms a carbamate with the vinca alkaloid, or analog or derivative thereof.
47. The drug delivery conjugate of 46 wherein the alkyl is ethyl.
48. The drug delivery conjugate of 1 wherein the bivalent linker comprises a releasable linker, a spacer linker, and a releasable linker taken together to form 3-dithioarylalkyloxycarbonyl, where the carbonyl forms a carbamate or a carbamoylaziridine with the vinca alkaloid, or analog or derivative thereof.
49. A pharmaceutical composition comprising a drug delivery conjugate of 1, 2, or 3, and a pharmaceutically acceptable carrier, diluent, or excipient therefor.
50. A method of eliminating a population of pathogenic cells in a host animal harboring the population of pathogenic cells wherein the members of the pathogenic cell population have an accessible binding site for a vitamin, or an analog or a derivative thereof, and wherein the binding site is uniquely expressed, overexpressed, or preferentially expressed by the pathogenic cells, said method comprising the step of administering to said host a drug delivery conjugate of 1, 2, or 3, or a pharmaceutical composition thereof.

The invention described herein is further illustrated by the following examples; however, it is to be understood that those examples are solely intended to be illustrative of the invention, and should not be construed to limit the invention in any way. For example, in each compound presented herein, the stereochemistry of amino acids used in forming the linker may be optionally selected from the natural 1 configuration, or the unnatural d configuration. In addition, many variations are contemplated herein, including but not limited to various other analogs and derivatives of vinblastine, various other spacer, heteroatom, and linker combinations, and others. Each Example compound described herein was characterized by NMR, MS, and/or UV spectroscopy, and/or HPLC as indicated, and selected analytical data, including characteristic ¹H NMR signals, MS signals, etc. are noted as appropriate.

### METHOD EXAMPLES

METHOD EXAMPLE 1. Inhibition of Tumor Growth in Mice. The anti-tumor activity of the compounds described herein, when administered intravenously (i.v.) to tumor-bearing animals, was evaluated in Balb/c mice bearing subcutaneous M109 tumors. Approximately 11 days post tumor inoculation in the subcutis of the right axilla with 1 x 10⁶ M109 cells (tumor volume range at to = between 60 and 80 mm³), mice (5/group) were injected i.v. three times a week (TIW), for a defined length of time (e.g., 2-3 weeks) with (a) a defined dose level on a per kilogram body weight basis of a drug delivery conjugate described herein, or (b) an equivalent dose volume of PBS (control). Tumor growth was measured using calipers at 2-day or 3-day intervals in each treatment group. Tumor volumes were calculated using the equation V = a x b²/2, where "a" is the length of the tumor and "b" is the width expressed in millimeters.

METHOD EXAMPLE 2. Inhibition of Tumor Growth in Mice. The anti-tumor activity of the compounds described herein, when administered intravenously (i.v.) to tumor-bearing animals, was evaluated in nu/nu mice bearing subcutaneous KB tumors. Approximately 8 to 11 days post tumor inoculation in the subcutis of the right axilla with 1 x 10⁶ KB cells (tumor volume range at to = between 60 and 80 mm³), mice (5/group) were injected i.v. three times a week (TIW), for a defined length of time (e.g., 2-3 weeks) with (a) a defined dose level on a per kilogram body weight basis of a drug delivery conjugate described herein, or (b) an equivalent dose volume of PBS (control). Tumor growth was measured using calipers at 2-day or 3-day intervals in each treatment group. Tumor volumes were calculated using the equation V = a x b²/2, where "a" is the length of the tumor and "b" is the width expressed in millimeters.

METHOD EXAMPLE 3. Inhibition of Cellular DNA Synthesis. The compounds described herein were evaluated using an *in vitro* cytotoxicity assay that predicts the ability of the drug to inhibit the growth of folate receptor-positive KB cells. The compounds were comprised of folate linked to a respective chemotherapeutic drug, as prepared according to the protocols described herein. The KB cells were exposed for predetermined periods of time at 37°C to the indicated concentrations of folate-drug conjugate in the absence or presence of at least a 100-fold excess of folic acid. The cells were then rinsed with fresh culture medium and incubated in fresh culture medium for 72 hours at 37°C. Cell viability was assessed using a ³H-thymidine incorporation assay.

As shown in the figures herein, dose-dependent cytotoxicity was measurable, and in most cases, the IC₅₀ values (concentration of drug conjugate required to reduce ³H-thymidine incorporation into newly synthesized DNA by 50%) were in the low nanomolar range. Furthermore, the cytotoxicities of these conjugates were reduced in the presence of excess free folic acid, indicating that the observed cell killing was mediated by binding to the folate receptor.

METHOD EXAMPLE 4. Relative Affinity Assay. The affinity of the compounds described herein for folate receptors (FRs) relative to folate was determined according to a previously described method (Westerhof, G. R., J. H. Schornagel, et al. (1995) Mol. Pharm. 48: 459-471) with slight modification. Briefly, FR-positive KB cells were heavily seeded into 24-well cell culture plates and allowed to adhere to the plastic for 18 h. Spent incubation media was replaced in designated wells with folate-free RPMI (FFRPMI) supplemented with 100 nM ³H-folic acid in the absence and presence of increasing concentrations of test article or folic acid. Cells were incubated for 60 min at 37°C and then rinsed 3 times with PBS, pH 7.4, followed by the addition of 500 µL of 1% SDS in PBS, pH 7.4. Cell lysates were then collected and added to individual vials containing 5 mL of scintillation cocktail, and then counted for radioactivity. Negative control tubes contained only the ³H-folic acid in FFRPMI (no competitor). Positive control tubes contained a final concentration of 1 mM folic acid, and CPMs measured in these samples (representing non-specific binding of label) were subtracted from all samples. Notably, relative affinities were defined as the inverse molar ratio of compound required to displace 50% of ³H-folic acid bound to the FR on KB cells, and the relative affinity of folic acid for the FR was set to 1.

METHOD EXAMPLE 5. 4T-1 Tumor Volume Assay. Six to seven week-old mice (female Balb/c strain) were obtained from Harlan, Inc., Indianapolis, IN. The mice were maintained on Harlan's folate-free chow for a total of three weeks prior to the onset of and during this experiment. Folate receptor-negative 4T-1 tumor cells (1 x 10⁶ cells per animal) were inoculated in the subcutis of the right axilla. Approximately 5 days post tumor inoculation when the 4T-1 tumor average volume was ~100 mm³, mice (5/group) were injected i.v. three times a week (TIW), for 3 weeks with 3 µmol/kg of drug delivery conjugate or with an equivalent dose volume of PBS (control). Tumor growth was measured using calipers at 2-day or 3-day intervals in each treatment group. Tumor volumes were calculated using the equation V = a x b²/2, where "a" is the length of the tumor and "b" is the width expressed in millimeters.

METHOD EXAMPLE 6. Animal Weight Determination. The percentage weight change of the mice was determined in mice (5 mice/group) on the indicated days post-tumor inoculation (PTI) as shown in the graph for the samples described in the related tumor volume assay.

METHOD EXAMPLE 7. General Preparation of Folate-Peptides. Linkers described herein that include a peptide are prepared by polymer-supported sequential approach using standard methods, such as the Fmoc-strategy on an acid-sensitive Fmoc-AA-Wang resin. Illustratively, the folate-containing peptidyl fragment Pte-Glu-(AA)ₙ-NH(CHR₂)CO₂H (3) is prepared by the method shown in Scheme 1 from Wang resin supported amino acids and Fmoc protected amino acid synthesis. 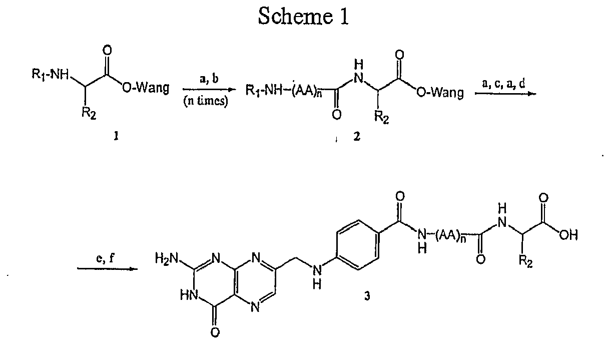 (a) 20% piperidine/DMF; (b) Fmoc-AA-OH, PyBop, DIPEA., DMF; (c) Fmoc-Glu-O-t-Bu or Fmoc-Glu(γ-O-t-Bu)-OH, PyBop, DIPEA, DMF; (d) N¹⁰(TFA)-Pte-OH; PyBop, DIPEA, DMSO; (e) TFAA, (CH₂SH)₂, *i*-Pr₃SiH; (f) NH₄OH, pH 9-10.

In this illustrative embodiment of the processes described herein, R₁ is Fmoc, R₂ is the desired appropriately protected amino acid side chain, Wang is a 2-chlorotrityl-Resin, and DIPEA is diisopropylethylamine. Standard coupling procedures, such as PyBOP and others described herein or known in the art are used, where the coupling agent is illustratively applied as the activating reagent to ensure efficient coupling. Fmoc protecting groups are removed after each coupling step under standard conditions, such as upon treatment with piperidine, tetrabutylammonium fluoride (TBAF), and the like. Appropriately protected amino acid building blocks, such as Fmoc-Glu-OtBu, *N*¹⁰-TFA-Pte-OH, and the like, are used, as described in Scheme 1, and represented in step (b) by Fmoc-AA-OH. Thus, AA refers to any amino acid starting material, that is appropriatedly protected. It is to be understood that the term amino acid as used herein is intended to refer to any reagent having both an amine and a carboxylic acid functional group separated by one or more carbons, and includes the naturally occurring alpha and beta amino acids, as well as amino acid derivatives and analogs of these amino acids. In particular, amino acids having side chains that are protected, such as protected serine, threonine, cysteine, aspartate, and the like may also be used in the folate-peptide synthesis described herein. Further, gamma, delta, or longer homologous amino acids may also be included as starting materials in the folate-peptide synthesis described herein. Further, amino acid analogs having homologous side chains, or alternate branching structures, such as norleucine, isovaline, β-methyl threonine, β-methyl cysteine, β,β-dimethyl cysteine, and the like, may also be included as starting materials in the folate-peptide synthesis described herein.

The coupling sequence (steps (a) & (b)) involving Fmoc-protected amino acids (AA) of the formula Fmoc-AA-OH is performed "n" times to prepare solid-support peptide (2), where n is an integer and may equal 0 to about 100. Following the last coupling step, the remaining Fmoc group is removed (step (a)), and the peptide is sequentially coupled to a glutamate derivative (step (c)), deprotected, and coupled to TFA-protected pteroic acid (step (d)). Subsequently, the peptide is cleaved from the polymeric support upon treatment with trifluoroacetic acid, ethanedithiol, and triisopropylsilane (step (e)). These reaction conditions result in the simultaneous removal of the *t*-Bu, *t*-Boc, and Trt protecting groups that may form part of the appropriately-protected amino acid side chain. The TFA protecting group is removed upon treatment with base (step (f)) to provide the folate-containing peptidyl fragment (3).

### COMPOUND EXAMPLES

### EXAMPLE 1

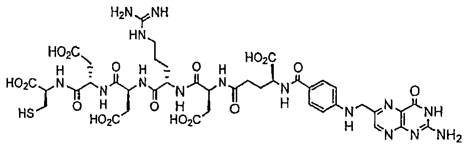

According to the general procedure of Method Example 7 (Scheme 1), Wang resin bound 4-methoxytrityl (MTT)-protected Cys-NH₂ was reacted according to the following sequence: 1) a. Fmoc-Asp(OtBu)-OH, PyBOP, DIPEA; b. 20% Piperidine/DMF; 2) a. Fmoc-Asp(OtBu)-OH, PyBOP, DIPEA; b. 20% Piperidine/DMF; 3) a. Fmoc-Arg(Pbf)-OH, PyBOP, DIPEA; b. 20% Piperidine/DMF; 4) a. Fmoc-Asp(OtBu)-OH, PyBOP, DIPEA; b. 20% Piperidine/DMF; 5) a. Fmoc-Glu-OtBu, PyBOP, DIPEA,; b. 20% Piperidine/DMF; 6) N¹⁰-TFA-pteroic acid, PyBOP, DIPEA. The MTT, tBu, and Pbf protecting groups were removed with TFA/H₂O/TIPS/EDT (92.5:2.5:2.5:2.5), and the TFA protecting group was removed with aqueous NH₄OH at pH =9.3. Selected ¹H NMR (D₂O) δ (ppm) 8.68 (s, 1H, FA H-7), 7.57 (d, 2H, J = 8.4 Hz, FA H-12 &16), 6.67 (d, 2H, J = 9 Hz, FA H-13 &15), 4.40-4.75 (m, 5H), 4.35 (m, 2H), 4.16 (m, 1H), 3.02 (m, 2H), 2.55-2.95 (m, 8H), 2.42 (m, 2H), 2.00-2.30 (m, 2H), 1.55-1.90 (m, 2H), 1.48 (m, 2H); MS (ESI, m+H⁺) 1046.

### EXAMPLE 2

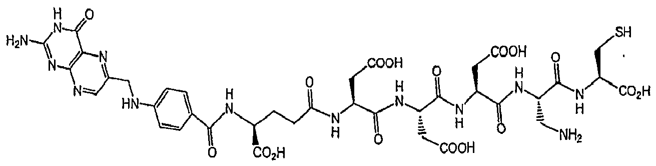

According to the general procedure of Method Example 7 (Scheme 1), Wang resin bound 4-methoxytrityl (MTT)-protected Cys-NH₂ was reacted according to the following sequence: 1) a. Fmoc-β-aminoalanine(NH-MTT)-OH, PyBOP, DIPEA; b. 20% Piperidine/DMF; 2) a. Fmoc-Asp(OtBu)-OH, PyBOP, DIPEA; b. 20% Piperidine/DMF; 3) a. Fmoc-Asp(OtBu)-OH, PyBOP, DIPEA; b. 20% Piperidine/DMF; 4) a. Fmoc-Asp(OtBu)-OH, PyBOP, DIPEA; b. 20% Piperidine/DMF; 5) a. Fmoc-Glu-OtBu, PyBOP, DIPEA; b. 20% Piperidine/DMF; 6) N¹⁰-TFA-pteroic acid, PyBOP, DIPEA. The MTT, tBu, and TFA protecting groups were removed with a. 2% hydrazine/DMF; b. TFA/H₂O/TIPS/EDT (92.5:2.5:2.5:2.5). The reagents shown in the following table were used in the preparation:

| Reagent | (mmol) | equivalents | amount |
|---|---|---|---|
| H-Cys(4-methoxytrityl)-2-chlorotrityl-Resin (loading 0.56mmol/g) | 0.56 | 1 | 1.0 g |
| Fmoc-β-aminoalanine(NH-MTT)-OH | 1.12 | 2 | 0.653 g |
| Fmoc-Asp(OtBu)-OH | 1.12 | 2 | 0.461 g |
| Fmoc-Asp(OtBu)-OH | 1.12 | 2 | 0.461 g |
| Fmoc-Asp(OtBu)-OH | 1.12 | 2 | 0.461 g |
| Fmoc-Glu-OtBu | 1.12 | 2 | 0.477 g |
| N¹⁰TFA-Pteroic Acid (dissolve in 10ml DMSO) | 0.70 | 1.25 | 0.286 g |
| DIPEA | 2.24 | 4 | 0.390 mL |
| PyBOP | 1.12 | 2 | 0.583 g |

The coupling step was performed as follows: In a peptide synthesis vessel add the resin, add the amino acid solution, DIPEA, and PyBOP. Bubble argon for 1 hr. and wash 3X with DMF and IPA. Use 20% piperidine in DMF for Fmoc deprotection, 3X (10 min), before each amino acid coupling. Continue to complete all 6 coupling steps. At the end wash the resin with 2% hydrazine in DMF 3X (5 min) to cleave TFA protecting group on Pteroic acid.

Cleave the peptide analog from the resin using the following reagent, 92.5% (50 ml) TFA, 2.5% (1.34 ml) H₂O, 2.5% (1.34 ml) Triisopropylsilane, 2.5% (1.34 ml) ethanedithiol, the cleavage step was performed as follows: Add 25 ml cleavage reagent and bubble for 1.5 hr, drain, and wash 3X with remaining reagent. Evaporate to about 5 mL and precipitate in ethyl ether. Centrifuge and dry. Purification was performed as follows: Column- Waters NovaPak C₁₈ 300x19mm; Buffer A= 10 mM Ammonium Acetate, pH 5; B= CAN; 1%B to 20%B in 40 minutes at 15 ml/min, to 350 mg (64%); HPLC-RT 10.307 min., 100% pure, ¹H HMR spectrum consistent with the assigned structure, and MS (ES-): 1624.8, 1463.2, 1462.3, 977.1, 976.2, 975.1, 974.1, 486.8, 477.8.

### EXAMPLE 3

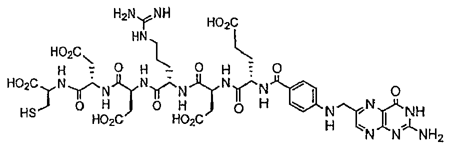

According to the general procedure of Method Example 7 (Scheme 1), Wang resin bound MTT-protected Cys-NH₂ was reacted according to the following sequence: 1) a. Fmoc-Asp(OtBu)-OH, PyBOP, DIPEA; b. 20% Piperidine/DMF; 2) a. Fmoc-Asp(OtBu)-OH, PyBOP, DIPEA; b. 20% Piperidine/DMF; 3) a. Fmoc-Arg(Pbf)-OH, PyBOP, DIPEA; b. 20% Piperidine/DMF; 4) a. Fmoc-Asp(OtBu)-OH, PyBOP, DIPEA; b. 20% Piperidine/DMF; 5) a. Fmoc-Glu(γ-OtBu)-OH, PyBOP, DIPEA; b. 20% Piperidine/DMF; 6) N¹⁰-TFA-pteroic acid, PyBOP, DIPEA. The MTT, tBu, and Pbf protecting groups were removed with TFA/H₂O/TIPS/EDT (92.5:2.5:2.5:2.5), and the TFA protecting group was removed with aqueous NH₄OH at pH =9.3. The ¹H NMR spectrum was consistent with the assigned structure.

### EXAMPLE 4

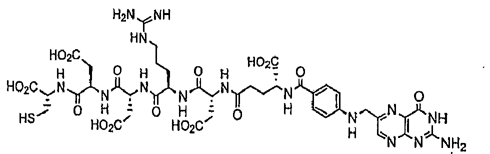

According to the general procedure of Method Example 7 (Scheme 1), Wang resin bound MTT-protected D-Cys-NH₂ was reacted according to the following sequence: 1) a. Fmoc-D-Asp(OtBu)-OH, PyBOP, DIPEA; b. 20% Piperidine/DMF; 2) a. Fmoc-D-Asp(OtBu)-OH, PyBOP, DIPEA; b. 20% Piperidine/DMF; 3) a. Fmoc-D-Arg(Pbf)-OH, PyBOP, DIPEA; b. 20% Piperidine/DMF; 4) a. Fmoc-D-Asp(OtBu)-OH, PyBOP, DIPEA,; b. 20% Piperidine/DMF; 5) a. Fmoc-D-Glu-OtBu, PyBOP, DIPEA; b. 20% Piperidine/DMF; 6) N¹⁰-TFA-pteroic acid, PyBOP, DIPEA. The MTT, tBu, and Pbf protecting groups were removed with TFA/H₂O/TIPS/EDT (92.5:2.5:2.5:2.5), and the TFA protecting group was removed with aqueous NH₄OH at pH =9.3. The ¹H NMR spectrum was consistent with the assigned structure.

### EXAMPLE 5

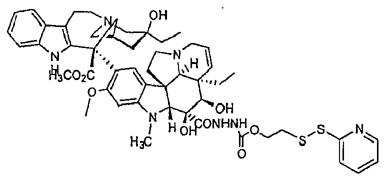

2-[(Benzotriazole-1-yl-(oxycarbonyloxy)-ethyldisulfanyl]-pyridine HCl (601 mg) and 378 µL of DIPEA were sequentially added to a solution of desacetyl vinblastine hydrazide (668 mg) in 5 ml of DCM at 0°C. The reaction was allowed to warm to room temperature and stirred for 3 hours. TLC (15% MeOH in DCM) showed complete conversion. The mixture was purified by silica gel chromatography (1:9 MeOH/DCM). The combined fractions were evaporated, redissolved in DCM and washed with 10% Na2C03, brine, dried (MgSO4), and evaporated to 550 mg (80%); HPLC-RT 12.651 min., 91% pure, 1H HMR spectrum consistent with the assigned structure, and MS (ESI+); 984.3, 983.3, 982.4, 492.4, 491.9, 141.8. Additional details of this procedure are described in U.S. patent application publication no. US 2005/0002942 A1, incorporated herein in its entirety by reference.

### EXAMPLE 6

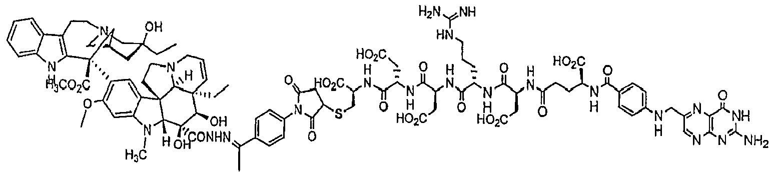

Desacetylvinblastine monohydrazide (1 eq.) was prepared according to Barnett et al., J. Med. Chem. 21:88-96 (1978), the disclosure of which is incorporated herein by reference, and treated in fresh distilled THF with 1 eq. of trifluoroacetic acid. After stirring for 10 min the solution was treated with 1.05 eq. of N-(4-acetylphenyl)maleimide. Acyl hydrazone formation was completed in 45 min and the solvent was evaporated.

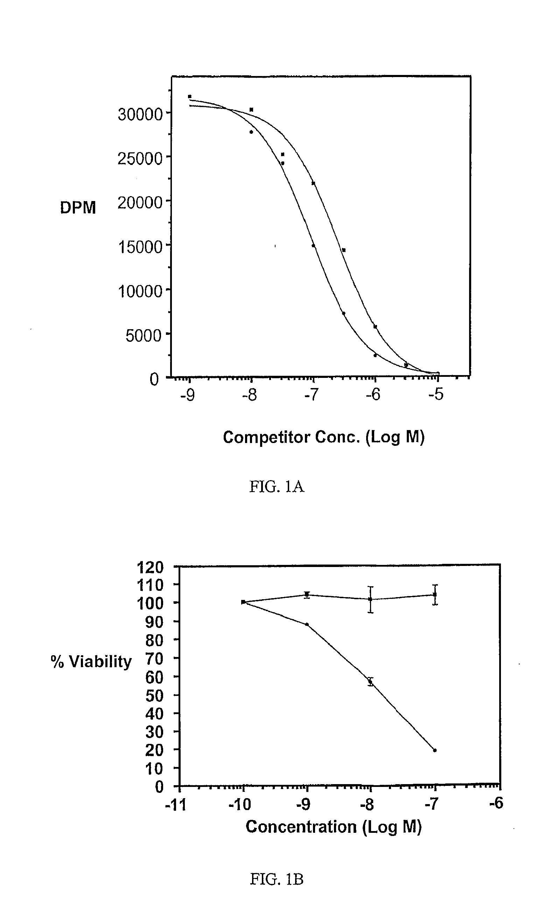

The peptidyl fragment Pte-Glu-Asp-Arg-Asp-Asp-Cys-OH (Example 1) (0.85 eq.) was dissolved in water, and the pH was adjusted to 2.5 with 0.1 N HCl, causing the peptide to precipitate. The peptidyl fragment was collected by centrifugation, dried, and dissolved in DMSO. To the resulting clear yellow solution was added Hünig's base (15 eq.) and the acyl hydrazone Micahel adduct. After 1 h, the final conjugate was purified by HPLC. FIGS. 1A and 1B show the relative binding affinity for folate versus the folate-deacetylvinblastine conjugate, the effects of the conjugate on ³H-thymidine incorporation, respectively. FIGS. 1A and 1B show the IC₅₀ of the conjugate (14 nM), and that folate competes with the folate-deacetylvinblastine conjugate for binding to the folate receptor demonstrating the specificity of binding of the conjugate. The assays were conducted according to Method Examples 3 and 4.

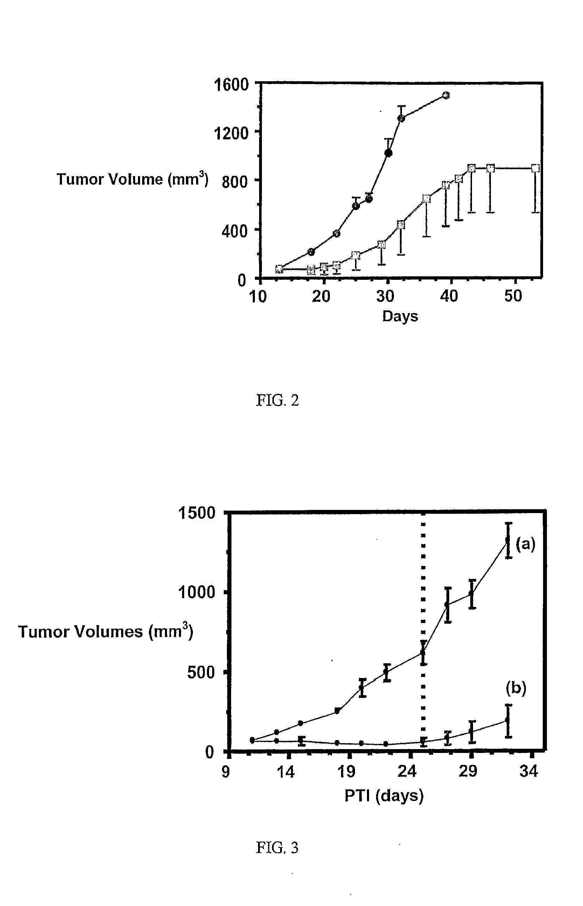

FIG. 2 shows the activity of Example 6 (1.5 µmol/kg) against M109 tumors in Balb/c mice. The assay was performed according to Method Example 1. Example 6 inhibits the growth of solid tumors. FIG.3 shows the activity of Example 6 at 10 µmol/kg given TIW for 3 weeks on FR-positive M109 tumors, where the dosing of the Example 6 compound ended on Day 25 as indicated by the dashed line. The assay was performed according to Method Example 1. Example 6 inhibits the growth of solid tumors.

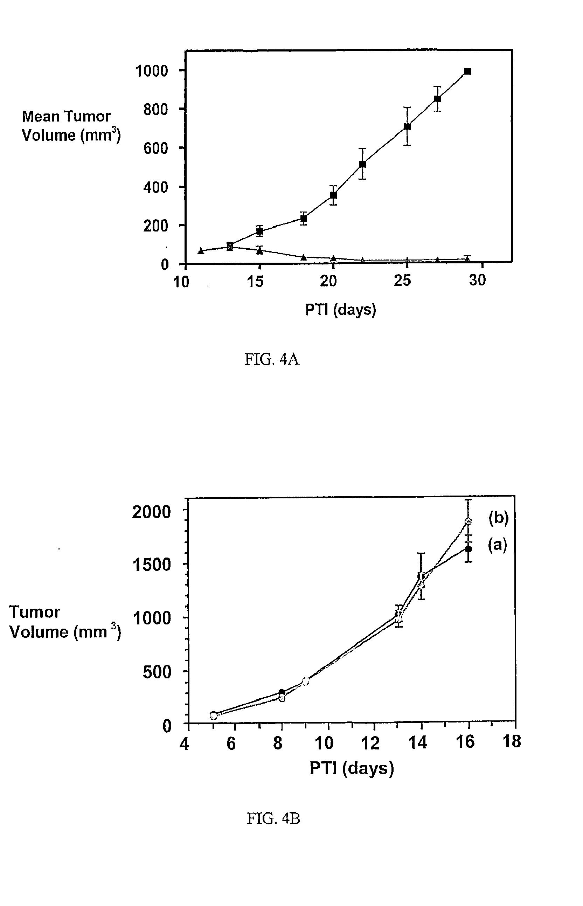

FIGS. 4A and 4B show the activity of Example 6 at 3 µmol/kg TIW for 3 weeks on FR-positive M109 tumors and FR negative 4T-1 tumor cells, respectively. The assays were performed as described in Method Examples 1 and 5, respectively. Example 6 inhibits the growth of solid M109 tumors, but not folate receptor (FR)-negative tumors.

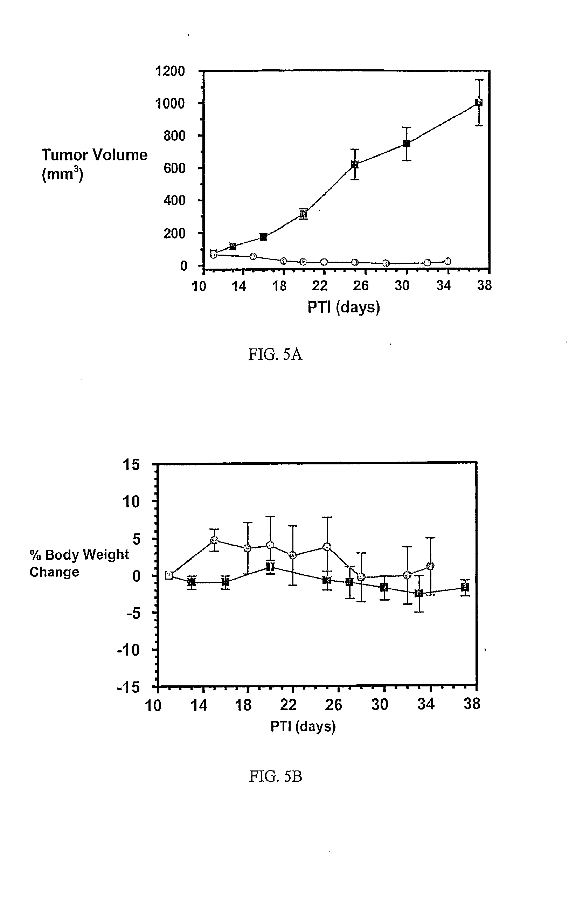

FIGS. 5A and 5B show the activity of Example 6 at 10 µmol/kg TIW for 3 weeks on FR-positive KB tumors and on the weight of nu/nu mice (nu/nu mice were used for the KB tumor volume assay), respectively. The assays were performed according the Method Examples 2 and 6, respectively. Example 6 inhibits the growth of solid tumors, but does not affect the weight of the mice.

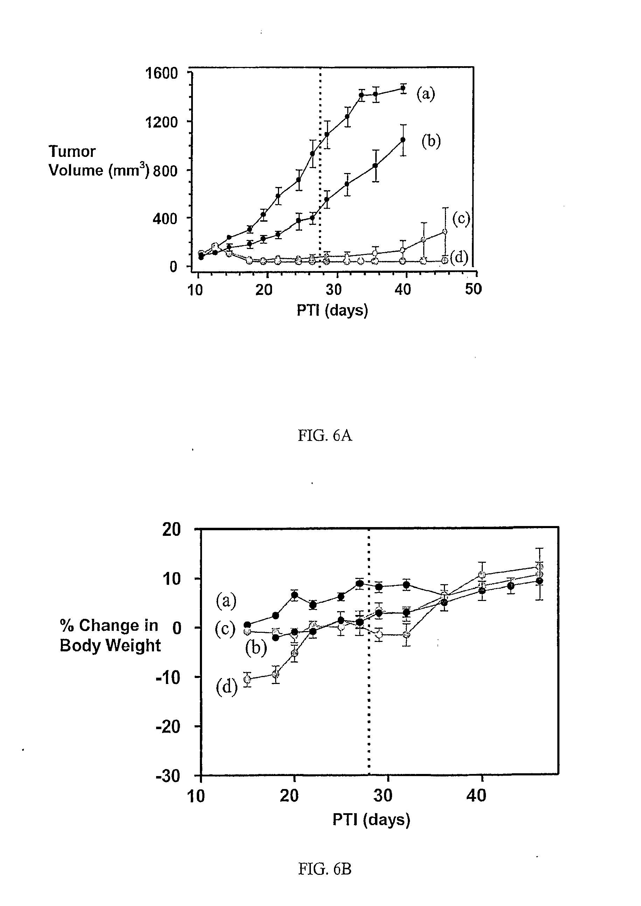

FIGS. 6A and 6B show the activity of Example 6 at 1, 5, and 10 µmol/kg TIW for 3 weeks on FR-positive KB tumors (average tumor volume at to = 50-100 mm³) and, at the same concentrations of Example 6, on the weight of nu/nu mice (nu/nu mice were used for the KB tumor volume assay), respectively. The assays were performed according the Method Examples 2 and 6, respectively. Example 6 inhibits the growth of solid tumors, but has little effect on the weight of the mice.

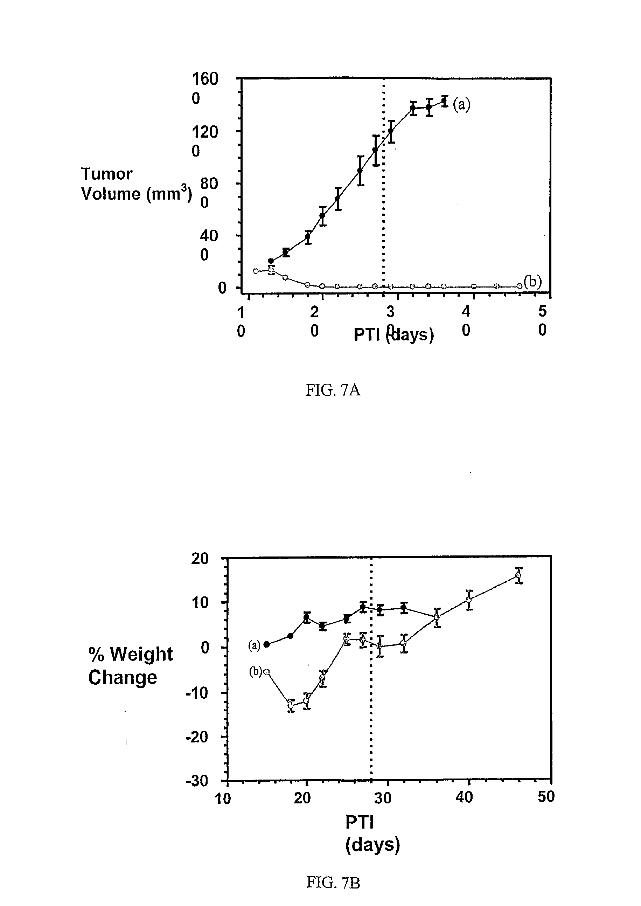

FIGS. 7A and 7B show the activity of Example 6 at 10 µmol/kg TIW for 3 weeks on FR-positive KB tumors (average tumor volume at to =100-150 mm³). The effect of Example 6 versus unconjugated vinblastine on the weight of Balb/c mice is also shown. The assays were performed according to the Method Examples 2 and 6, respectively. Example 6 inhibits the growth of solid tumors. Unconjugated vinblastine reduces the weight of the mice initially, but the weight of the mice eventually increases, probably due to tumor growth.

### EXAMPLE 7

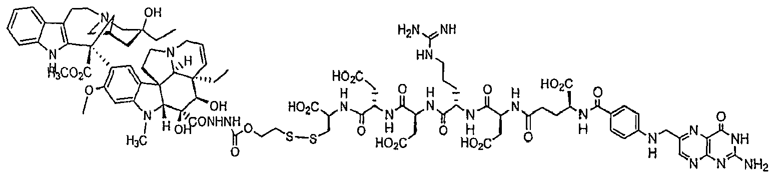

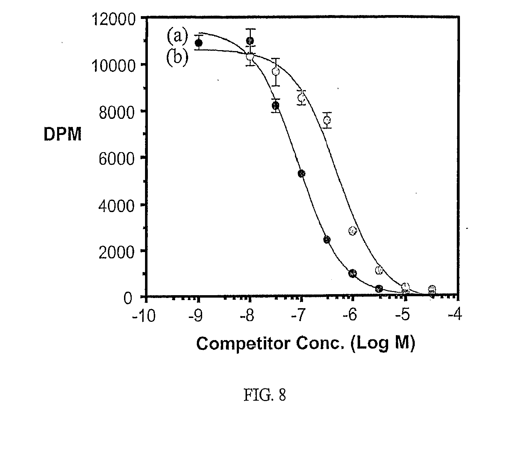

Peptidyl fragment Pte-Glu-Asp-Arg-Asp-Asp-Cys-OH (Example 1) in THF was treated with either the thiosulfonate or pyridyldithio-activated vinblastine (Example 5) as a yellow solution resulting dissolution in 0.1 M NaHCO₃ at pH > 6.5 under argon. Lyophilization and HPLC gave a 70% yield; selected ¹H NMR (D₂O) δ 8.67 (s, 1H, FA H-7), 7.50 (br s, 1H, VLB H-11'), 7.30-7.40 (br s, 1H, VLB H-14'), 7.35 (d, 2H, J = 7.8 Hz, FA H-12 &16), 7.25 (m, 1H, VLB H-13'), 7.05 (br s, 1H, VLB H-12'), 6.51 (d, 2H, J = 8.7 Hz, FA H-13 &15), 6.4 (s, 2H, VLB H-14 & 17), 5.7 (m, 1H, VLB olefin), 5.65 (m, 1H, VLB H-7), 5.5 (d, 1H, VLB olefin), 5.5 (m, 1H, VLB H-6), 4.15(m, 1H, VLB H-8'), 3.82 (s, 3H, VLB C₁₈,-CO₂CH₃), 3.69 (s, 3H, VLB C₁₆-OCH₃), 2.8 (s, 3H, VLB N-CH₃), 1.35 (br s, 1H, VLB H-3'), 1.15 (m, 1H, VLB H-2'), 0.9 (t, 3H, J = 7 Hz, VLB H-21'), 0.55 (t, 3H, J = 6.9 Hz, VLB H-21); LCMS (ESI, m+H⁺) 1918. FIG. 8 shows the relative binding affinity for folate versus the Example 7 conjugate. The assay was performed as described in Method Example 4.

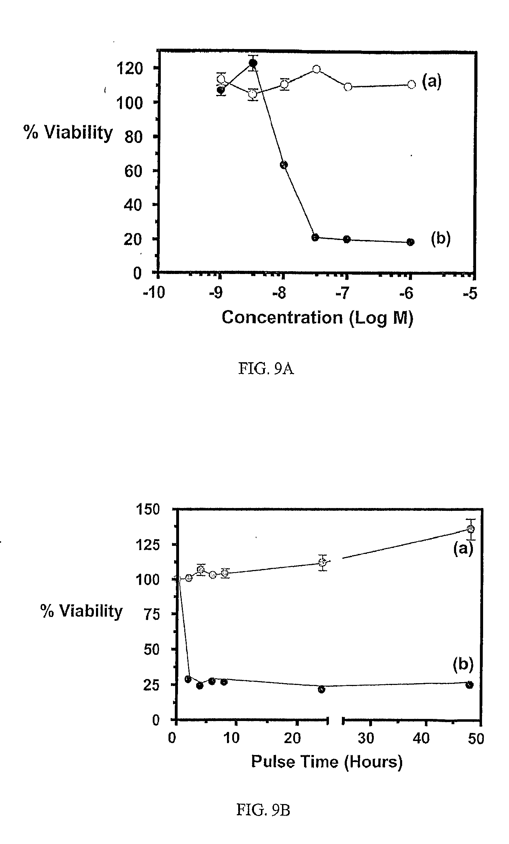

FIG. 9A shows the effects of Example 7 on ³H-thymidine incorporation, the IC₅₀ of the conjugate (9 nM), and that folate competes with the Example 7 conjugate for binding to the folate receptor demonstrating the specificity of binding of the conjugate. FIG. 9B shows the effect of Example 7 on ³H-thymidine incorporation versus the pulse time for treatment with the Example 7 conjugate (100 nM Example 7), and that folate competes with the Example 7 conjugate for binding to the folate receptor demonstrating the specificity of binding of the conjugate (100 nM Example 7 + 100 µM folic acid). The assays were performed according to Method Example 3.

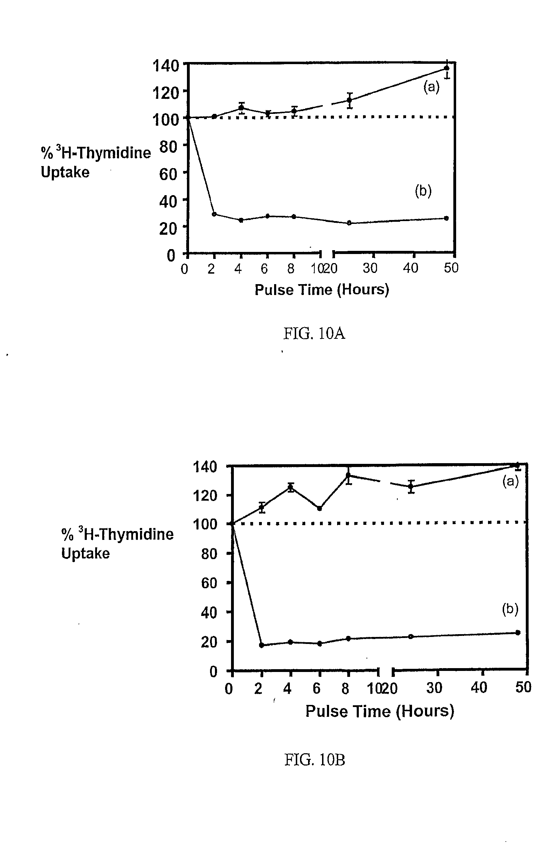

FIGS. 10A and 10B show the effect of 10 and 100 nM Example 7 on ³H-thymidine incorporation versus the pulse time for treatment with the Example 7 conjugate, and that folate competes with the Example 7 conjugate for binding to the folate receptor demonstrating the specificity of binding of the conjugate. The assays were performed according to Method Example 3.

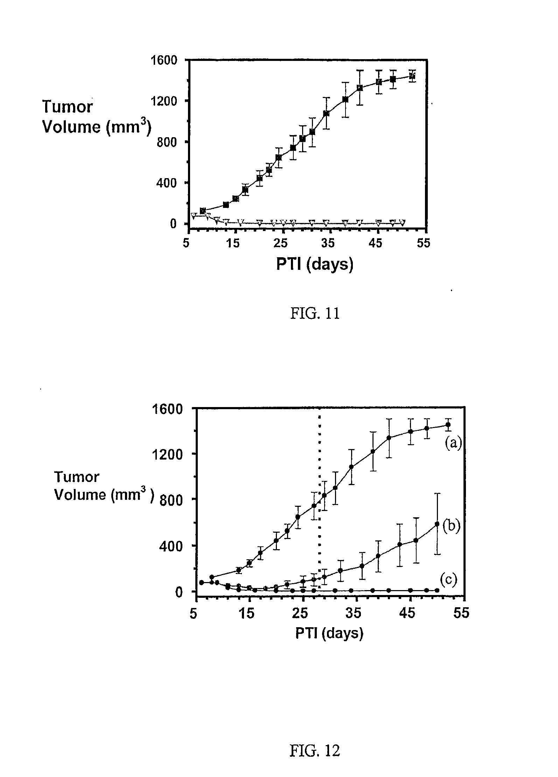

FIG. 11 shows the activity of Example 7 at 5 µmol/kg TIW for 3 weeks on FR-positive KB tumors (average tumor volume at to = 50-100 mm³). The assay was performed according the Method Example 2. Example 7 inhibits the growth of solid tumors.

FIG. 12 shows the activity of Example 7 at 5 µmol/kg TIW for 3 weeks on FR-positive KB tumors (nu/nu mice were used for the KB tumor volume assay). The assay was performed according the Method Example 2. Examples 7 and 8 inhibit the growth of solid tumors.

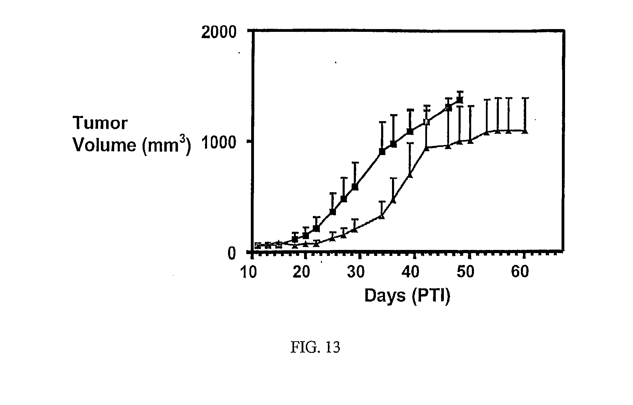

FIG. 13 shows the activity of Example 7 (1.5 µmol/kg) against M109 tumors in Balb/c mice. The assay was performed according to Method Example 1. Example 7 inhibits the growth of solid tumors.

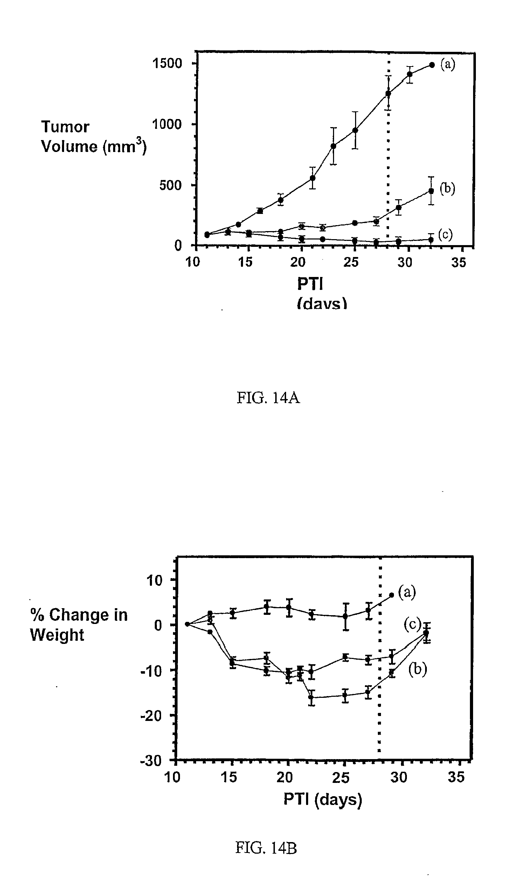

FIGS. 14A and 14B show the activity of Examples 7 and 8 (each at 10 µmol/kg) against M109 tumors in Balb/c mice and on the weight of Balb/c mice (Balb/c mice were used for the M109 tumor volume assay). The assays were performed according to Method Examples 1 and 6, respectively. Examples 7 and 8 inhibit the growth of solid tumors and have little effect on the weight of Balb/c mice.

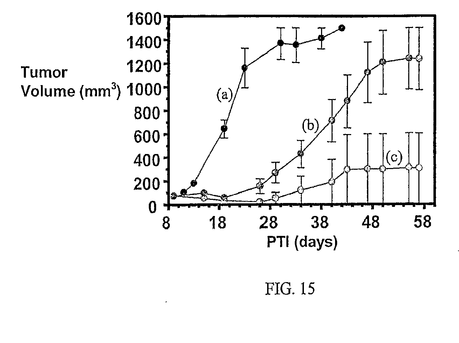

FIG. 15 shows the activity of Example 7 at 2 µmol/kg TIW for 2 weeks on FR-positive KB tumors +/- 40 µmol/kg EC20 (rhenium complex). Example 7 inhibits the growth of solid tumors, and that inhibitory effect is prevented (competed) by the EC20 rhenium complex. EC20 (rhenium complex) is the compound of the following formula: 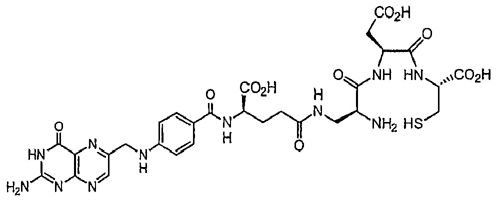 chelated to Rhenium. The preparation of EC20 is described in U.S. patent application publication no. US 2004/0033195 A1, the synthetic procedure description of which is incorporated herein by reference. The assay was performed according the Method Example 2. EC20 acts as a competitor of Example 7 at folate receptors, and the results show the specificity of the effects of Example 7.

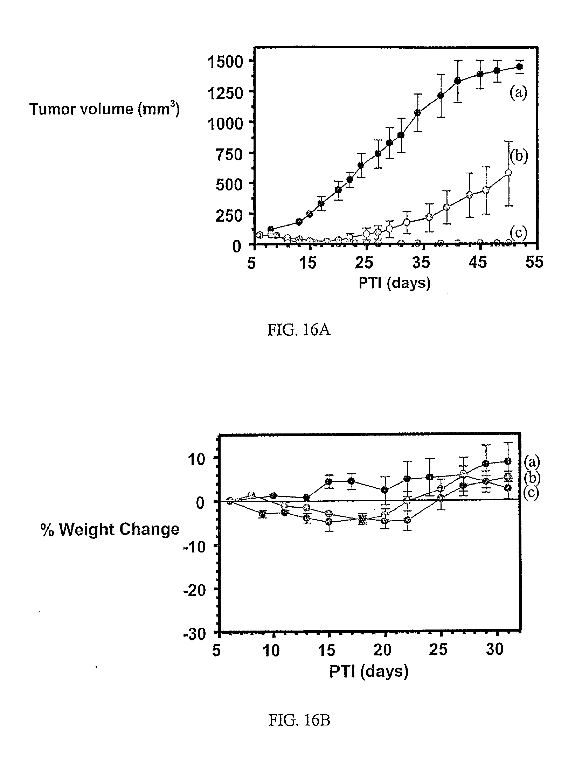

FIGS. 16A and 16B show the activity of Examples 7 and 8 at 5 µmol/kg TIW for 3 weeks on FR-positive KB tumors, and the effects of Examples 7 and 8 and on the weight of nu/nu mice (nu/nu mice were used for the KB tumor volume assay). The assays were performed according the Method Examples 2 and 6, respectively. The results show that Example 7 has a higher growth inhibitory activity than Example 6 against subcutaneous FR-positive human nasopharyngeal KB tumor xenografts in *nu*/*nu* mice. Examples 7 and 8 have little effect on the weights of nu/nu mice.

Example 7 showed a better therapeutic index than the unconjugated desacetylvinblastine hydrazide (DAVLBH) in *nu*/*nu* mice bearing s.c. KB tumor xenografts as shown in the following table:

| Compound | Dose (µmol/kg) | Dose Protocol | CR^{(a)} | %T/C^{(b)} | LCK^{(c)} | Weight Loss |
|---|---|---|---|---|---|---|
| Example 7 | 1 | TIW 2 wk | 0/5 | 227 | 1.6 | 0 |
| | 2 | TIW 2wk | 4/5 | 169 | 0.9 | 0 |
| | 5 | TIW 2wk | 5/5 | - | - | <6% |
| | 10 | BIW 3 wk | 5/5 | - | - | < 10% |
| DAVLBH | 0.5 | TIW 2 wk | 0/5 | 146 | 0.6 | 0 |
| | 0.75 | TIW 2wk | 0/5 | 265 | 1.8 | 0 |
| | 1 | TIW 2wk | 0/5 | 246 | 1.8 | > 10% |
| | 2 | TIW 2wk | 0/5 ^{(d)} | - | - | > 20% |

| | | | | | | |
|---|---|---|---|---|---|---|
| (a) CR corresponds to the number of animals (total of 5 tested) showing a complete response to treatment with the test compound compared to controls; (b) %T/C is percent tumor over controls for animals not showing complete response; (c) LCK is log of cell kill for animals not showing complete response; (d) 5 deaths. | | | | | | |

### EXAMPLE 9

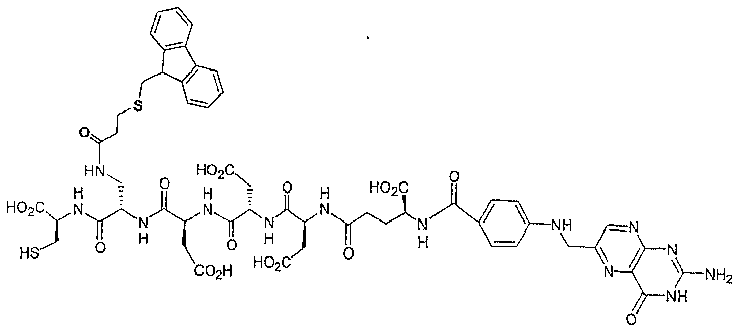

According to the general procedure of Method Example 7 (Scheme 1), Wang resin bound 4-methoxytrityl (MTT)-protected Cys-NH₂ was reacted according to the following sequence: 1) a. Fmoc-β-aminoalanine(NH-IvDde)-OH, PyBOP, DIPEA; b. 20% Piperidine/DMF; 2) a. Fmoc-Asp(OtBu)-OH, PyBOP, DIPEA; b. 20% Piperidine/DMF; 3) a. Fmoc-Asp(OtBu)-OH, PyBOP, DIPEA; b. 20% Piperidine/DMF; 4) a. Fmoc-Asp(OtBu)-OH, PyBOP, DIPEA; b. 20% Piperidine/DMF; 5) a. Fmoc-Glu-OtBu, PyBOP, DIPEA; b. 20% Piperidine/DMF; 6) N¹⁰-TFA-pteroic acid, PyBOP, DIPEA. The MTT, tBu, and TFA protecting groups were removed with a. 2% hydrazine/DMF; b. TFA/H₂O/TIPS/EDT (92.5:2.5:2.5:2.5). The reagents shown in the following table were used in the preparation:

| Reagent | (mmol) | Equivalents | Amount |
|---|---|---|---|
| H-Cys(4-methoxytrityl)-2-chlorotrityl-Resin (loading 0.56 mmol/g) | 0.56 | 1 | 1.0g |
| Fmoc-β-aminoalanine(NH-IvDde)-OH | 1.12 | 2 | 0.596g |
| Fmoc-Asp(OtBu)-OH | 1.12 | 2 | 0.461g |
| Fmoc-Asp(OtBu)-OH | 1.12 | 2 | 0.461g |
| Fmoc-Asp(OtBu)-OH | 1.12 | 2 | 0.461g |
| Fmoc-Glu-OtBu | 1.12 | 2 | 0.477g |
| N¹⁰TFA-Pteroic Acid (dissolve in 10 ml DMSO) | 0.70 | 1.25 | 0.286g |
| Fm-Thiopropionic acid | 0.70 | 1.25 | 199.08 |
| DIPEA | 2.24 | 4 | 0.390mL |
| PyBOP | 1.12 | 2 | 0.583g |

The coupling step was performed as follows: In a peptide synthesis vessel add the resin, add the amino acid solution in DMF, DIPEA, and PyBOP. Bubble argon for 1 hr. and wash 3X10 mL with DMF and IPA. Use 20% piperdine in DMF for Fmoc deprotection, 3X10 mL (10 min), before each amino acid coupling. Continue to complete 6 coupling steps. At the end wash the resin with 2% hydrazine in DMF 3X10 mL (5 min) to cleave TFA protecting group on Pteroic acid and IvDde protecting group on β-aminoalanine. Finally, couple the free amine of the β-aminoalanine with the Fmoc-thiopropionic acid in DMF using DIPEA and PyBop. Bubble argon for 1 hr. and wash 3X10 mL with DMF and IPA. Dry the resin under argon for 30 min.

Cleave the peptide analog from the resin using the following reagent, 92.5% (50 ml) TFA, 2.5% (1.34 ml) H₂O, 2.5% (1.34 ml) Triisopropylsilane, 2.5% (1.34 ml) ethanedithiol, the cleavage step was performed as follows: Add 25 ml cleavage reagent and bubble for 1.5 hr, drain, and wash 3X with remaining reagent. Evaporate to about 5 mL and precipitate in ethyl ether. Centrifuge and dry. Purification was performed as follows: Column-Waters NovaPak C₁₈ 300x19mm; Buffer A= 10 mM Ammonium Acetate, pH 5; B= CAN; 1%B to 20%B in 40 minutes at 15 ml/min, to 450 mg (65%); ¹H HMR spectrum consistent with the assigned structure.

### EXAMPLE 10

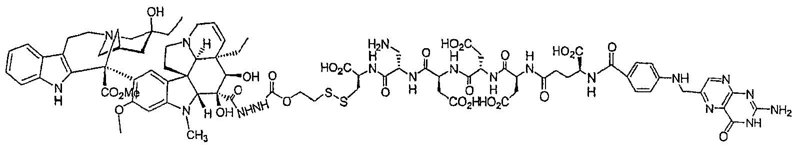

In a polypropylene centrifuge bottle, Example 2 (82 mg, 0.084 mmol) was dissolved in 5 mL of water and bubbled with argon for 10 min. In another flask, a 0.1N NaHCO₃ solution was argon bubbled for 10 min. pH of the linker solution was adjusted to about 6.9 using the 0.1N NaHCO₃ solution. The vinblastine hydrazide derivative (Example 5,91mg, 0.092 mM) in 5 mL of tetrahydrofuran (THF) was added slowly to the above solution. The resulting clear solution was stirred under argon for 15 min to 1h. Progress of the reaction was monitored by analytical HPLC (10mM ammonium acetate, pH = 7.0 and acetonitrile). THF was evaporated, and the aqueous solution was filtered and injected on a prep-HPLC column (XTerra Column, 19 X 300 mM). Elution with 1mM sodium phosphate pH = 7.0 and acetonitrile resulted in pure fractions containing the product, which was isolated after freeze-drying for 48h (78mg, 50%); C₈₃H₁₀₃N₁₉O₂₆S₂; exact mass: 1845.68; MW: 1846.95; HPLC-RT 15.113 min., 100% pure, ¹H HMR spectrum consistent with the assigned structure, and MS (ES-): 1846.6, 1845.5, 933.3, 924.2, 923.3, 922.5, 615.6, 614.7, 525.0.

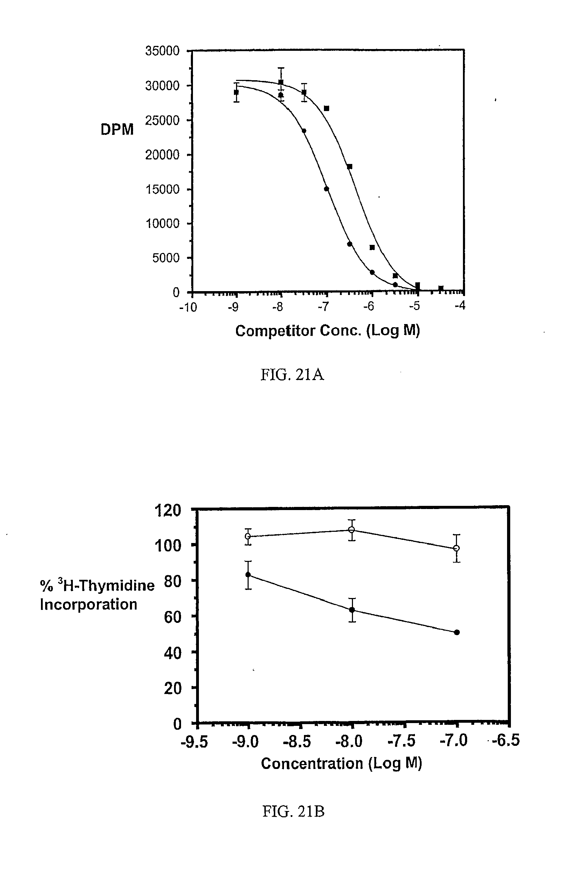

FIGS. 21A and 21B show the relative binding affinity for folate versus Example 10, and the effects of Example 10 on ³H-thymidine incorporation, the IC₅₀ of the conjugate (58 nM), and that folate competes with the conjugate for binding to the folate receptor demonstrating the specificity of binding of the conjugate. The assays were conducted according to Method Examples 4 and 3, respectively.

### EXAMPLE 11

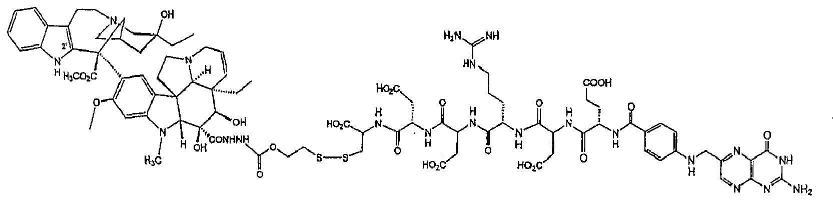

Prepared according to Example 7, except that Example 3 was substituted for Example 5. Ar was bubbled into a solution of Example 3 (302 mg) in 5 ml of water for 10 min. The pH of this solution was adjusted to 6.8-7.0 using saturated NaHCO3 solution. Example 5 (258 mg) in 5 ml of THF was added to the solution of and stirred for 30 min. The solvents were evaporated, and the resulting mixture was filtered. The filtrate was purified by preparative HPLC (Solvent A - 1 mM phosphate buffer; Solvent B - acetonitrile; Waters XTterra C18, 19 mm X 300 mm; Gradient - 5% B to 50% B in 30 minutes) to 240 mg; ¹H NMR spectrum consistent with the assigned structure; MS (ESI, m+H⁺) 1917.9, 960.9, 960.2, 959.3, 813.1, 812.3, 803.0, 295.0.

### EXAMPLE 12

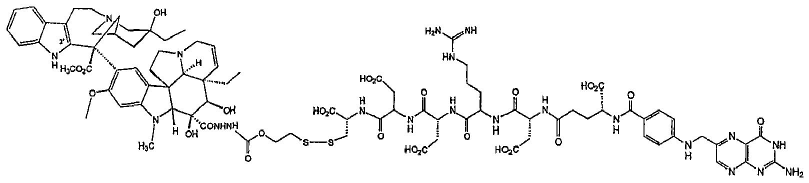

Prepared according to Example 7, except that Example 4 was substituted for Example 5. Ar was bubbled into a solution of Example 4 (40 mg) in 5 ml of water for 10 min. The pH of this solution was adjusted to 6.8-7.0 using saturated NaHCO3 solution. Example 5 (30 mg) in 5 ml ofTHF was added to the solution of and stirred for 30 min. The solvents were evaporated, and the resulting mixture was filtered. The filtrate was purified by preparative HPLC (Solvent A - 1 mM phosphate buffer; Solvent B - acetonitrile; Waters XTterra C18, 19 mm X 300 mm; Gradient - 5% B to 50% B in 30 minutes) to 43 mg. HPLC-RT 4.058 min., 98% pure,¹H HMR spectrum consistent with the assigned structure, and MS (ES-): 1917.5, 1916.5, 1915.6, 959.2, 958.4.

FIGS. 26A and 26B show the activities of Examples 11 and 12 at 2 µmol/kg TIW for 3 weeks on FR-positive KB tumors and on the weight of nu/nu mice (nu/nu mice were used for the KB tumor volume assay). The assays were performed according the Method Examples 2 and 6, respectively. Examples 11 and 12 inhibit the growth of solid tumors, but have little effect on the weight of the mice.

### EXAMPLE 13

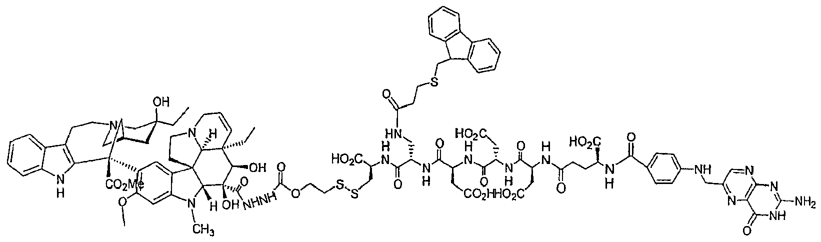

In a polypropylene centrifuge bottle, Example 9 (56 mg) was dissolved in 7.5 mL of water and bubbled with argon for 10 min. In another flask, a 0.1 N NaHCO₃ solution was bubbled with argon for 10 min. The pH of the Example 9 solution was adjusted to 6.9 using the 0.1 N NaHCO₃ solution. Example 5 (44mg) in 7.5 mL of tetrahydrofuran (THF) was added slowly to the Example 9 solution. The resulting clear solution was stirred under argon for 15 min to 1 h. Progress of the reaction was monitored by analytical HPLC (10mM ammonium acetate, pH = 7.0 and acetonitrile). THF was evaporated and the aqueous solution was filtered and purified by prep-HPLC. Elution with 1 mM sodium phosphate pH = 7.0 and acetonitrile resulted in pure fractions, which were pooled, evaporated at ambient temperature, and the resulting aqueous solution was adjusted to pH 4.0 using 0.1 N HCl. Example 13 was isolated after freeze-drying for 48 h (61mg, 64%). ¹H HMR spectrum and LCMS data consistent with the assigned structure.

### EXAMPLES 14 to 32

Prepared according to the processes and conditions described herein. Additional details for the preparation of the required thiosulfonate or pyridyldithio-activated vinblastine, and maleimide-activated vinblastine derivatives are described in U.S. patent application publication no. US 2005/0002942 A1.

### EXAMPLE 14

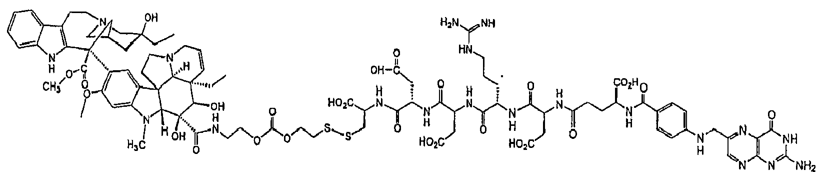

### EXAMPLE 15

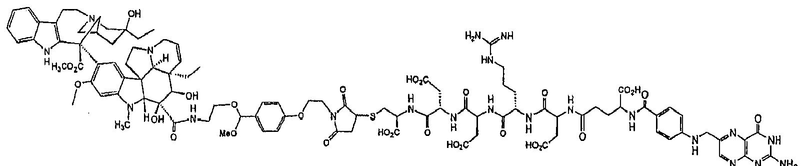

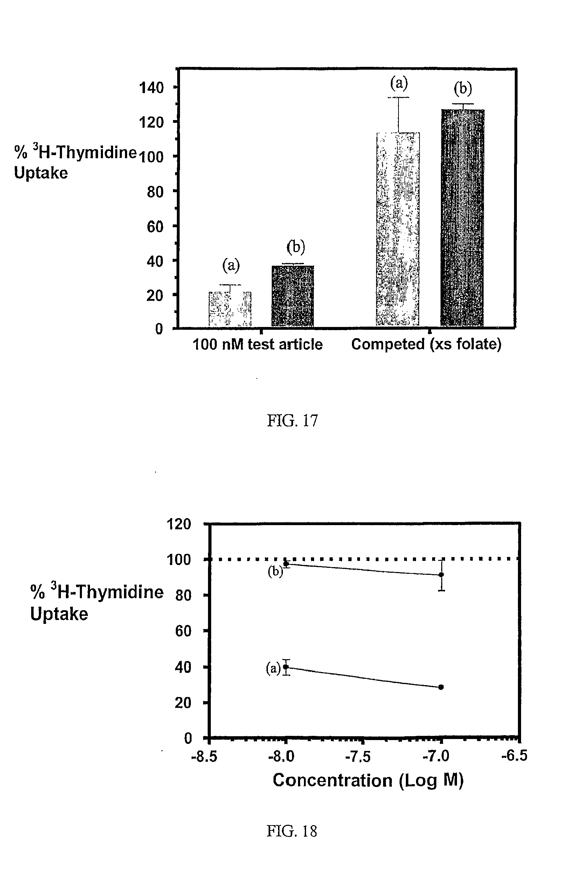

FIG. 17 shows the effects on ³H-thymidine incorporation of Examples 14 (a) and 15 (b) (first two bars; each at 100 nM for 1h with a 72 h chase, n = 2), and that folate competes with Examples 14 and 15 for binding to the folate receptor demonstrating the specificity of binding of the conjugates (second two bars). The assay was conducted according to Method Example 3.

### EXAMPLE 16

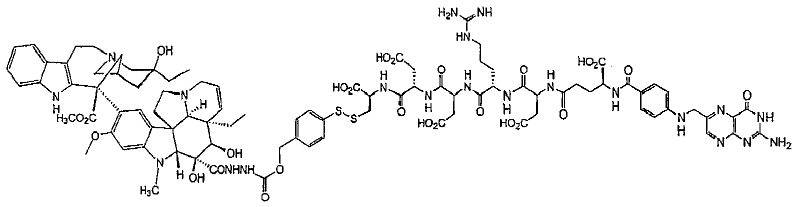

FIG. 18 shows the effects on ³H-thymidine incorporation of Example 16 (for a 2 h pulse with a 48 h chase, n = 2), and that folate competes with Example 16 for binding to the folate receptor demonstrating the specificity of binding of the conjugate. The assay was conducted according to Method Example 3.

### EXAMPLE 17

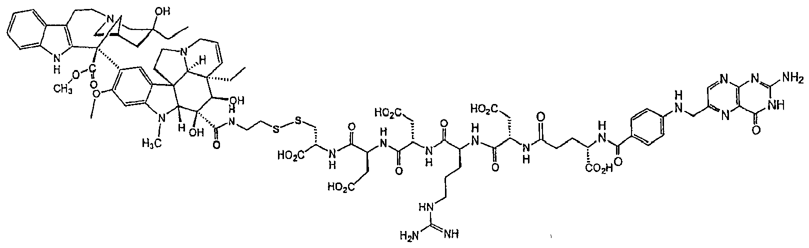

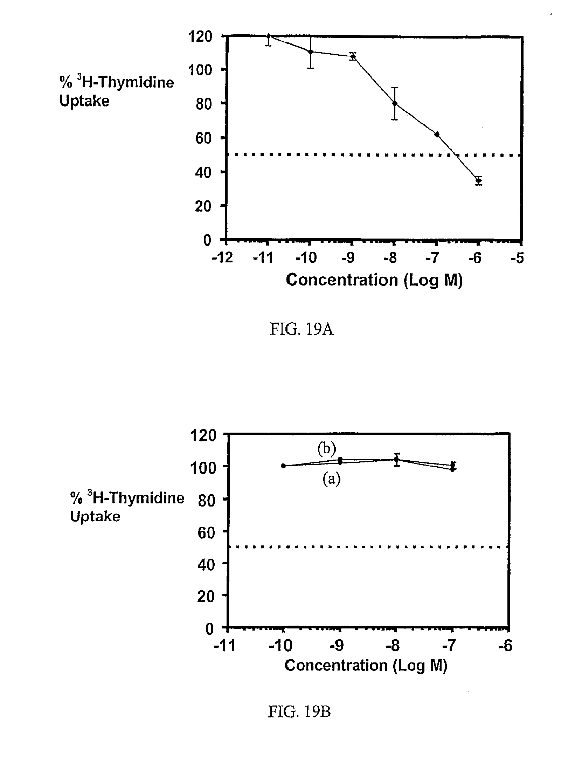

FIG. 19A shows the effects on ³H-thymidine incorporation of the unconjugated vinca. FIG. 19B shows the effects on ³H-thymidine incorporation of Example 17. The assays were conducted according to Method Example 3.

### EXAMPLE 18

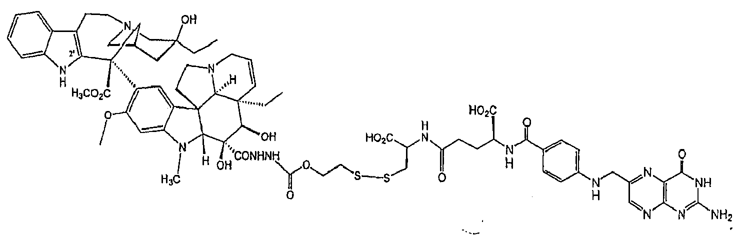

### EXAMPLE 19

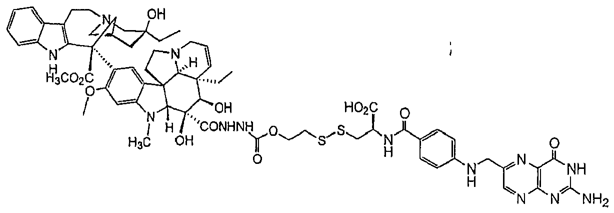

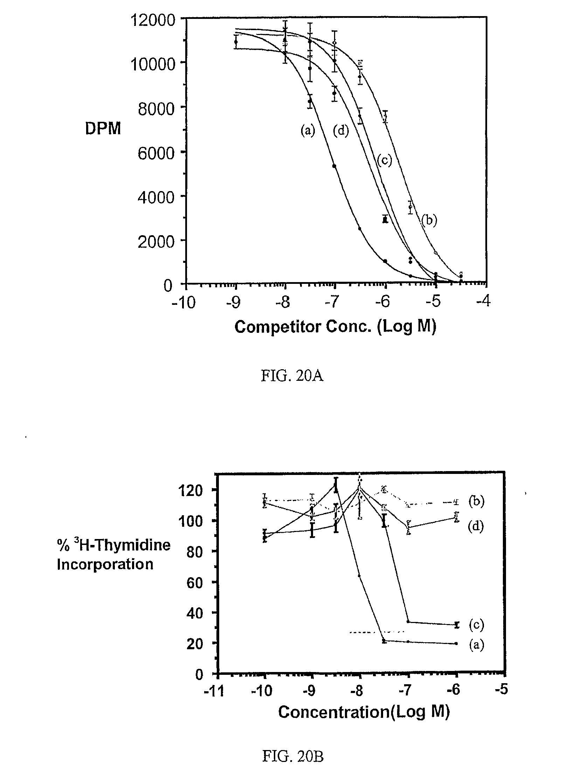

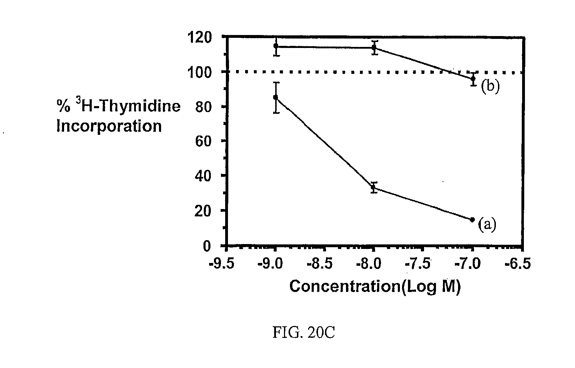

FIGS. 20A, 20B, and 20C show the relative binding affinity for folate versus Examples 18 and 19 compared to Example 7 (FIG. 20A), and their effects on ³H-thymidine incorporation (FIGS. 20B and 20C), and that folate competes with the conjugates for binding to the folate receptor demonstrating the specificity of binding of the conjugates. The assays were conducted according to Method Example 3 (FIGS. 20B and 20C) and Method Example 4 (FIG. 20A).

### EXAMPLE 20

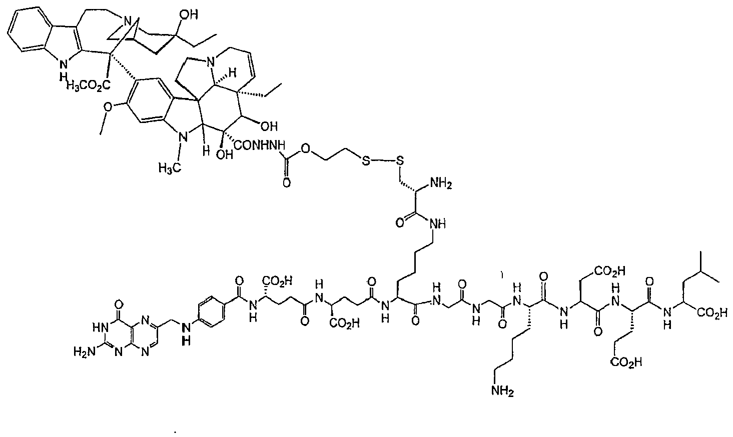

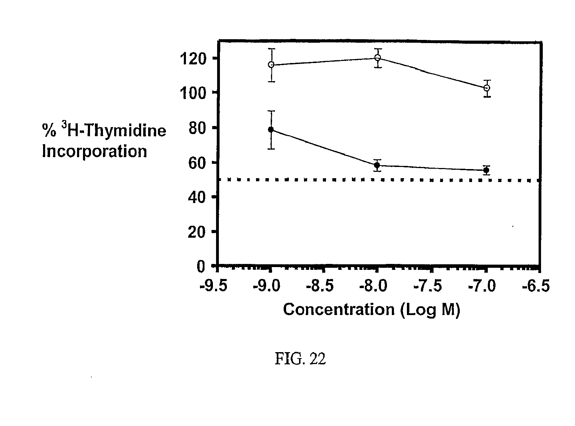

FIG. 22 shows the effects of Example 20 on ³H-thymidine incorporation, and that folate competes with the conjugate for binding to the folate receptor demonstrating the specificity of binding of the conjugate. The assay was conducted according to Method Example 3.

### EXAMPLE 21

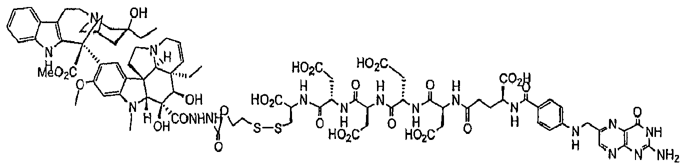

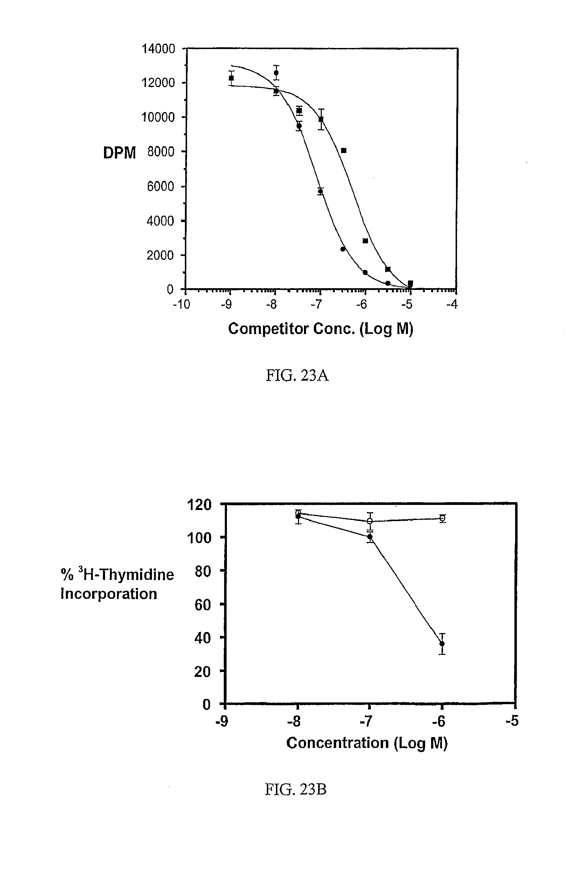

FIGS. 23A and 23B show the relative binding affinity for folate versus Example 21, and the effects of Example 21 on ³H-thymidine incorporation, and that folate competes with the conjugate for binding to the folate receptor demonstrating the specificity of binding of the conjugate. The assays were conducted according to Method Examples 4 and 3, respectively.

### EXAMPLE 22

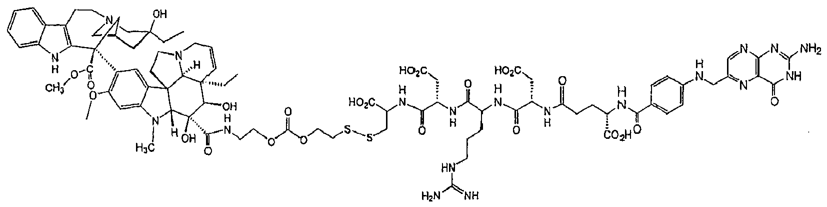

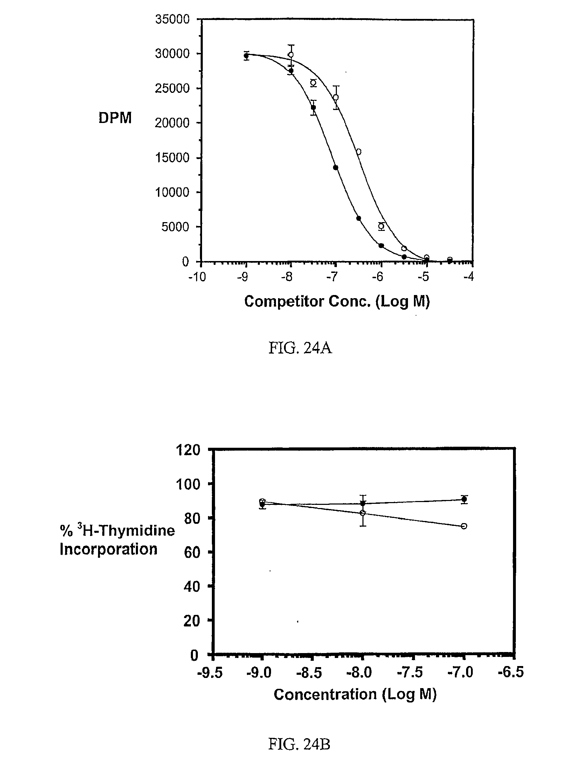

FIGS. 24A and 24B show the relative binding affinity for folate versus Example 22, and the effects of Example 22 on ³H-thymidine incorporation. The assays were conducted according to Method Examples 4 and 3, respectively.

Activity Comparison of Examples 21 & 22 to Example 7. FIGS. 25A and 25B show the activity of Examples 21 and 22 in comparison to 14B (each at 3 µmol/kg) against M109 tumors in Balb/c mice and on the weight of Balb/c mice (Balb/c mice were used for the M109 tumor volume assay). The assays were performed according to Method Examples 1 and 6, respectively. Examples 21, 22, and 7 inhibit the growth of solid tumors, but have little effect on the weight of the mice.

### EXAMPLE 23

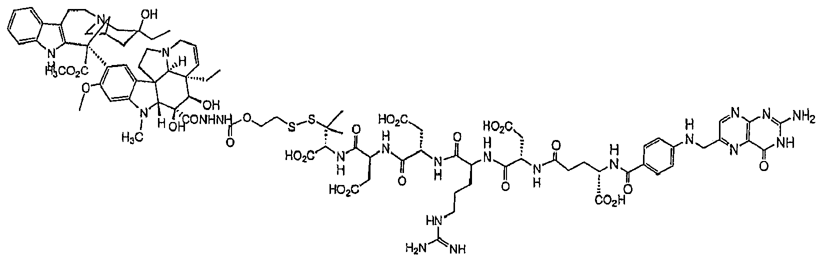

FIGS. 27A and 27B show the relative binding affinity for folate versus Example 23, and the effects of Example 23 on ³H-thymidine incorporation, the IC₅₀ of the conjugate (15 nM), and that folate competes with the conjugate for binding to the folate receptor demonstrating the specificity of binding of the conjugate. The assays were conducted according to Method Examples 4 and 3, respectively.

### EXAMPLE 24

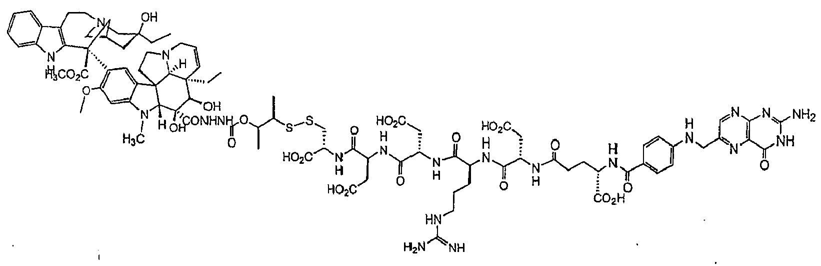

FIGS. 28A and 28B show the relative binding affinity for folate versus Example 24, and the effects of Example 24 on ³H-thymidine incorporation, the IC₅₀ of the conjugate (9 nM), and that folate competes with the conjugate for binding to the folate receptor demonstrating the specificity of binding of the conjugate. The assays were conducted according to Method Examples 4 and 3, respectively.

### EXAMPLE 25

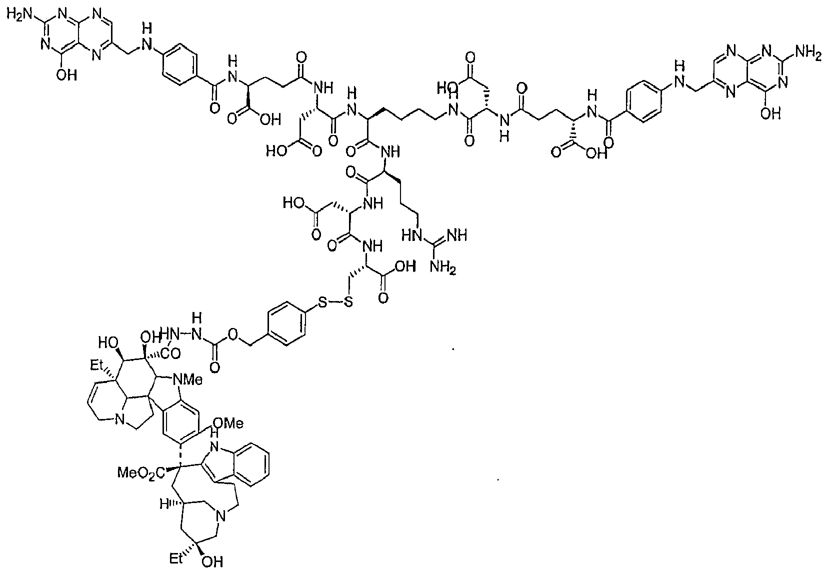

C₁₁₆H₁₄₀N₃₀O₃₂S₂; mol. wt.: 2530.67; exact mass: 2528.97; C, 55.05; H, 5.58; N, 16.60; O, 20.23; S, 2.53.

### EXAMPLE 26

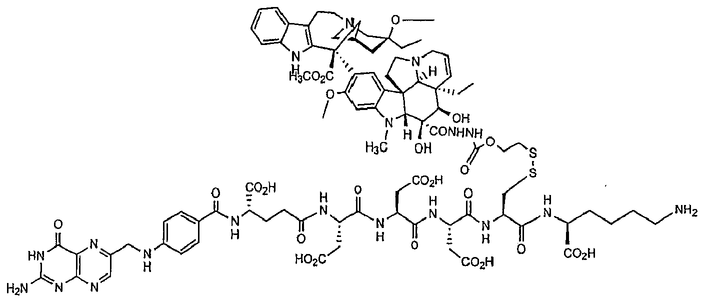

### EXAMPLE 27

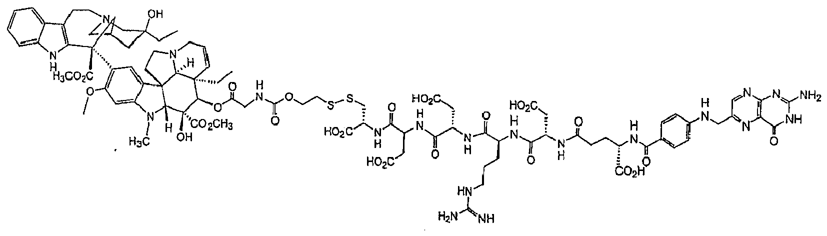

### EXAMPLE 28

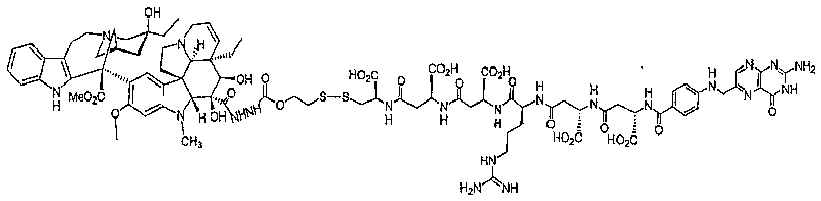

### EXAMPLE 29

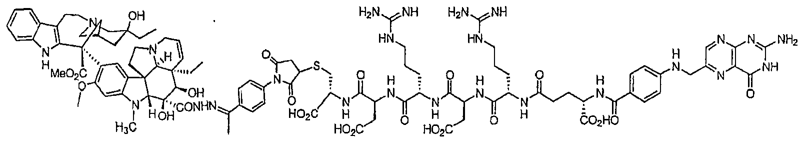

### EXAMPLE 30

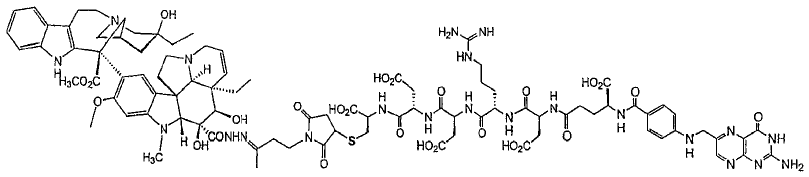

### EXAMPLE 31

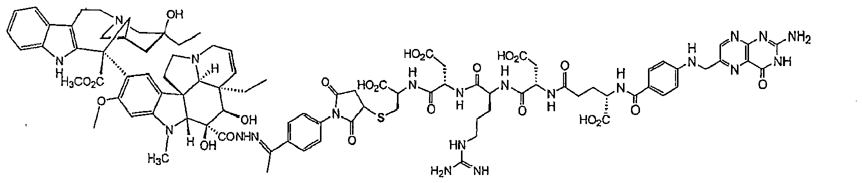

### EXAMPLE 32

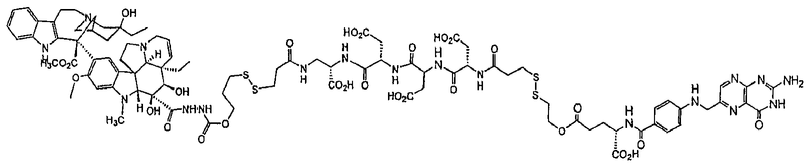

The following table summarizes the activity on KB cells, folate receptor competition, and the relative folate receptor affinity for selected drug deliver conjugates described herein:

| Example | IC₅₀ KB Cells (nM) | Folate Receptor Competition | Relative Folate Receptor Affinity |
|---|---|---|---|
| 6 | < 10 nM | Yes | 0.31 |
| 7 | < 100 nM | Yes | 0.17 |
| 14 | < 100 | Yes | 0.23 |
| 15 | < 100 | Yes | - |
| 16 | < 10 nM | Yes | - |
| 17 | > 100 | n/a | 0.10 |
| 18 | 6nM | Yes | 0.13 |
| 19 | < 10 nM | Yes | 0.05 |
| 29 | 36 | Yes | 0.05 |
| 30 | - | - | 0.19 |
| 31 | - | - | 0.14 |

## Claims

1. A receptor binding drug delivery conjugate comprising:
(a) a receptor binding moiety;
(b) a releasable bivalent linker; and
(c) a vinca alkaloid, or an analog or derivative thereof;
wherein the receptor binding moiety is covalently linked to the bivalent linker; the vinca alkaloid, or the analog or the derivative thereof, is covalently linked to the bivalent linker; and the bivalent linker comprises one or more divalent radicals selected from the group consisting of a disulfide group, a carbonate group, a silyl-containing group, an aziridine-containing group, a hydrazone-containing group, an alkyleneaminocarbonyl(dicarboxylarylene)carboxylate group, or a group of the formula 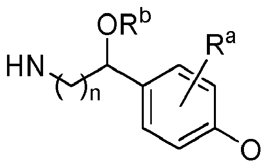 where n is selected from 1, 2, 3, and 4; R^{b} is an alkyl or optionally substituted arylalkyl, and R^{a} is hydrogen or an optional substitution;
wherein the conjugate is not of the formulae 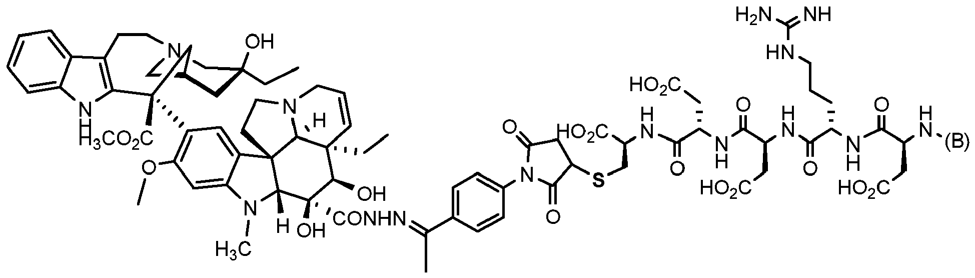 or 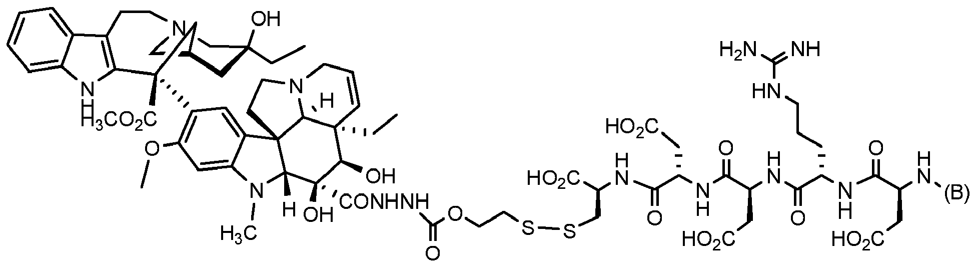 where B is a receptor binding moiety.

2. The drug delivery conjugate of claim 1 wherein the vinca alkaloid is vinblastine, desacetylvinblastine, vindesine, or thiovindesine.

3. The drug delivery conjugate of any one of claims 1 to 2 wherein the vinca alkaloid, or an analog or derivative thereof includes a carboxamide attached to the bivalent linker through the nitrogen.

4. The drug delivery conjugate of any one of claims 1 to 2 wherein the vinca alkaloid, or an analog or derivative thereof includes a carboxylate attached to the bivalent linker through the oxygen.

5. The drug delivery conjugate of any one of claims 1 to 4 wherein the receptor binding moiety is a folate, a folate analog, or a folate derivative.

6. The drug delivery conjugate of any one of claims 1 to 4 wherein the receptor binding moiety is selected from the list consisting of folic acid, folinic acid, pteroylpolyglutamic acid, pteroic acid, and amino acid derivatives thereof; tetrahydropterins, dihydrofolates, tetrahydrofolates, and deaza, dideaza, 1-deaza, 3-deaza, 5-deaza, 8-deaza, and 10-deaza, 1,5 dideaza, 5,10-dideaza, 8,10-dideaza, and 5,8-dideaza analogs thereof; aminopterin, amethopterin, N¹⁰-methylfolate, 2-deamino-hydroxyfolate, 1-deazamethopterin, 3-deazamethopterin, and 3',5'-dichloro-4-amino-4-deoxy-N¹⁰-methylpteroylglutamic acid.

7. The drug delivery conjugate of any one of claims 1 to 6 wherein the bivalent linker includes a disulfide.

8. The drug delivery conjugate of any one of claims 1 to 7 wherein the bivalent linker includes a carbonate.

9. The drug delivery conjugate of any one of claims 1 to 8 wherein the bivalent linker includes a silyl-containing group.

10. The drug delivery conjugate of any one of claims 1 to 9 wherein the bivalent linker further comprises at least one peptide.

11. The drug delivery conjugate of any one of claims 1 to 9 wherein the bivalent linker further comprises a plurality of naturally occurring amino acids and stereoisomers thereof.

12. The drug delivery conjugate of any one of claims 1 to 9 wherein the bivalent linker comprises one or more amino acids selected from the group consisting of asparagine, aspartic acid, glutamic acid, glutamine, beta-amino alanine, ornithine, lysine, arginine, serine, threonine, cysteine, and combinations thereof.

13. The drug delivery conjugate of any one of claims 1 to 12 wherein the bivalent linker further comprises one or more divalent heteroatoms selected from the group consisting of nitrogen, oxygen, sulfur, NHR¹NHR², SO, S(O)₂, and NR³O, wherein R¹, R², and R³ are each independently selected from the group consisting of hydrogen, alkyl, aryl, arylalkyl, substituted aryl, substituted arylalkyl, heteroaryl, substituted heteroaryl, and alkoxyalkyl.

14. The drug delivery conjugate of any one of claims 1 to 13 wherein the bivalent linker further comprises carbonyl, thionocarbonyl, alkylene, cycloalkylene, alkylenecycloalkyl, alkylenecarbonyl, cycloalkylenecarbonyl, carbonylalkylcarbonyl, 1-alkylenesuccinimid-3-yl, 1-(carbonylalkyl)succinimid-3-yl, alkylenesulfoxyl, sulfonylalkyl, alkylenesulfoxylalkyl, alkylenesulfonylalkyl, carbonyltetrahydro-2H-pyranyl, carbonyltetrahydrofuranyl, 1-(carbonyltetrahydro-2H-pyranyl)succinimid-3-yl, 1-(carbonyltetrahydrofuranyl)succinimid-3-yl, or a combination thereof, each of which is optionally substituted with one or more substituents X¹;
wherein each substituent X¹ is independently selected from the group consisting of alkyl, alkoxy, alkoxyalkyl, hydroxy, hydroxyalkyl, amino, aminoalkyl, alkylaminoalkyl, dialkylaminoalkyl, halo, haloalkyl, sulfhydrylalkyl, alkylthioalkyl, aryl, substituted aryl, arylalkyl, substituted arylalkyl, heteroaryl, substituted heteroaryl, carboxy, carboxyalkyl, alkyl carboxylate, alkyl alkanoate, guanidinoalkyl, R⁴-carbonyl, R⁵-carbonylalkyl, R⁶-acylamino, and R⁷-acylaminoalkyl, wherein R⁴ and R⁵ are each independently selected from the group consisting of an amino acid, an amino acid derivative, and a peptide, and wherein R⁶ and k⁷ are each independently selected from the group consisting of an amino acid, an amino acid derivative, and a peptide.

15. A pharmaceutical composition for eliminating a population of pathogenic cells in a host animal harboring the population of pathogenic cells wherein the members of the pathogenic cell population have an accessible binding site for a vitamin, or an analog or a derivative thereof, and wherein the binding site is uniquely expressed, overexpressed, or preferentially expressed by the pathogenic cells, the composition comprising a drug delivery conjugate of any one of claims 1 to 14, and a pharmaceutically acceptable carrier, diluent, or excipient therefor.
